# EUROPEAN PATENT SPECIFICATION

(11) **EP 1 041 982 B1**
(45) Date of publication and mention of the grant of the patent: **19.10.2011**
(21) Application number: 98964709.4
(22) Date of filing: 22.12.1998
(51) Int. Cl.: A61K 31/415, A61K 31/4439, A61P 29/00, A61P 37/02

(54) **INHIBITION OF p38 KINASE ACTIVITY USING SUBSTITUTED HETEROCYCLIC UREAS**
HEMMUNG DER p38 KINASE AKTIVITÄT DURCH SUBSTITUIERTE HETEROCYCLISCHE HARNSTOFFE
INHIBITION DE L'ACTIVITE DE P38 KINASE AU MOYEN D'UREES HETEROCYCLIQUES SUBSTITUEES

(30) Priority: 22.12.1997 US 995750
(43) Date of publication of application: 11.10.2000
(73) Proprietor: Bayer HealthCare LLC, Tarrytown, NY 10591 (US)
(72) Inventor: DUMAS, Jacques, Orange, CT 06477 (US); KHIRE, Uday, Hamden, CT 06518 (US); LOWINGER, Timothy, B., Nishinomiya, Hyogo 662-0046 (JP); PAULSEN, Holger, D-42115 Wuppertal (DE); RIEDL, Bernd, 42329 Wuppertal (DE); SCOTT, William, J., Guilford, CT 06437 (US); SMITH, Roger, A., Madison, CT 06443 (US); WOOD, Jill, E., Hamden, CT 06517 (US); HATOUM-MOKDAD, Holia, Hamden, CT 06514 (US); JOHNSON, Jeffrey, Branford, CT 06405 (US); LEE, Wendy, Hamden, CT 06518 (US); REDMAN, Aniko, Derby, CT 06418 (US)
(74) Representative: Weiss, Wolfgang
(86) International application number: PCT/US1998/026080
(87) International publication number: WO 1999/032111

(56) References cited:
- WO-A-95/07922
- WO-A-98/52558
- WO-A-98/52937
- WO-A-98/52941
- WO-A-98/56377
- WO-A-99/23091
- WO-A-99/32106
- WO-A-99/32110
- WO-A-99/32455
- US-A- 3 646 059
- US-A- 5 162 360
- US-A- 5 319 099
- US-A- 5 559 137
- HANSON G J: "inhibitors of p38 kinase" EXPERT OPINION ON THERAPEUTIC PATENTS, ASHLEY PUBLICATIONS, GB, vol. 7, no. 7, July 1997 (1997-07), pages 729-733, XP002086152 ISSN: 1354-3776
- BADGER ET AL.: J. PHARMACOL. EXP. THER., vol. 279, no. 3, 1996, pages 1453-1461,
- SHAPIRO ET AL.: PROC. NATL. ACAD. SCI., vol. 92, 1995, pages 12230-12234,
- G. HANSON: EXP. OPIN. THER. PATENTS, vol. 7, no. 7, 1997, pages 729-733,

## Description

### Field of the Invention

This invention relates to the use of a group of aryl ureas in treating cytokine mediated diseases and proteolytic enzyme mediated diseases, and pharmaceutical compositions for use in such therapy.

### Background of the Invention

Two classes of effector molecules which are critical for the progression of rheumatoid arthritis are pro-inflammatory cytokines and tissue degrading proteases. Recently, a family of kinases was described which is instrumental in controlling the transcription and translation of the structural genes coding for these effector molecules.

The mitogen-activated protein (MAP) kinase family is made up of a series of structurally related proline-directed serine/threonine kinases which are activated either by growth factors (such as EGF) and phorbol esters (ERK), or by IL-1, TNFα or stress (p38, JNK). The MAP kinases are responsible for the activation of a wide variety of transcription factors and proteins involved in transcriptional control of cytokine production. A pair of novel protein kinases involved in the regulation of cytokine synthesis was recently described by a group from SmithKline Beecham (Lee et al. Nature 1994, 372, 739). These enzymes were isolated based on their affinity to bond to a class of compounds, named CSAIDSs (cytokine suppressive anti-inflammatory drugs) by SKB. The CSAIDs, bicyclic pyridinyl imidazoles, have been shown to have cytokine inhibitory activity both *in vitro* and *in vivo.* The isolated enzymes, CSBP-1 and -2 (CSAID binding protein 1 and 2) have been cloned and expressed. A murine homologue for CSBP-2, p38, has also been reported (Han et al. Science 1994, 265, 808):

Early studies suggested that CSAIDs function by interfering with m-RNA translational events during cytokine biosynthesis. Inhibition of p38 has been shown to inhibit both cytokine production (eg., TNFα, IL-1, IL-6, IL-8) and proteolytic enzyme production (eg., MMP-1, MMP-3) *in vitro* and/or *in vivo.*

Clinical studies have linked TNFα production and/or signaling to a number of diseases including rheumatoid arthritis (Maini. J. Royal Coll. Physicians London 1996, 30, 344). In addition, excessive levels of TNFα have been implicated in a wide variety of inflammatory and/or immunomodulatory diseases, including acute rheumatic fever (Yegin et al. Lancet 1997, 349, 170), bone resorption (Pacifici et al. J. Clin. Endocrinol. Metabol. 1997, 82, 29), postmenopausal osteoperosis (Pacifici et al. J. Bone Mineral Res. 1996, 11, 1043), sepsis (Blackwell et al. Br. J. Anaesth. 1996, 77, 110), gram negative sepsis (Debets et al. Prog. Clin. Biol. Res. 1989, 308, 463), septic shock (Tracey et al. Nature 1987, 330, 662; Girardin et al. New England J. Med. 1988, 319, 397), endotoxic shock (Beutler et al. Science 1985, 229, 869; Ashkenasi et al. Proc. Nat'l. Acad. Sci. USA 1991, 88, 10535), toxic shock syndrome, (Saha et al. J. Immunol. 1996, 157, 3869; Lina et al. FEMS Immunol. Med. Microbiol. 1996, 13, 81), systemic inflammatory response syndrome (Anon. Crit. Care Med. 1992, 20, 864), inflammatory bowel diseases (Stokkers et al. J. Inflamm. 1995-6, 47, 97) including Crohn's disease (van Deventer et al. Aliment. Pharmacol. Therapeu. 1996, 10 (Suppl. 2), 107; van Dullemen et al. Gastroenterology 1995, 109, 129) and ulcerative colitis (Masuda et al. J. Clin. Lab. Immunol. 1995, 46, 111), Jarisch-Herxheimer reactions (Fekade et al. New England J. Med. 1996, 335, 311), asthma (Amrani et al. Rev. Malad. Respir. 1996, 13, 539), adult respiratory distress syndrome (Roten et al. Am. Rev. Respir. Dis. 1991, 143, 590; Suter et al. Am. Rev. Respir. Dis. 1992, 145, 1016), acute pulmonary fibrotic diseases (Pan et al. Pathol. Int. 1996, 46, 91), pulmonary sarcoidosis (Ishioka et al. Sarcoidosis Vasculitis Diffuse Lung Dis. 1996, 13, 139), allergic respiratory diseases (Casale et al. Am. J. Respir. Cell Mol. Biol. 1996, 15, 35), silicosis (Gossart et al. J. Immunol. 1996, 156, 1540; Vanhee et al. Eur. Respir. J. 1995, 8, 834), coal worker's pneumoconiosis (Borm et al. Am. Rev. Respir. Dis. 1988, 138, 1589), alveolar injury (Horinouchi et al. Am. J. Respir. Cell Mol. Biol. 1996, 14, 1044), hepatic failure (Gantner et al. J. Pharmacol. Exp. Therap. 1997, 280, 53), liver disease during acute inflammation (Kim et al. J. Biol. Chem. 1997, 272, 1402), severe alcoholic hepatitis (Bird et al. Ann. Intern. Med. 1990, 112, 917), malaria (Grau et al. Immunol. Rev. 1989, 112, 49; Taverne et al. Parasitol. Today 1996, 12, 290) including Plasmodium falciparum malaria (Perlmann et al. Infect. Immunit. 1997, 65, 116) and cerebral malaria (Rudin et al. Am. J. Pathol. 1997, 150, 257), non-insulin-dependent diabetes mellitus (NIDDM; Stephens et al. J. Biol. Chem. 1997, 272, 971; Ofei et al. Diabetes 1996, 45, 881), congestive heart failure (Doyama et al. Int. J. Cardiol. 1996, 54, 217; McMurray et al. Br. Heart J. 1991, 66, 356), damage following heart disease (Malkiel et al. Mol. Med. Today 1996, 2, 336), atherosclerosis (Parums et al. J. Pathol. 1996, 179, A46), Alzheimer's disease (Fagarasan et al. Brain Res. 1996, 723, 231; Aisen et al. Gerontology 1997, 43, 143), acute encephalitis (Ichiyama et al. J. Neurol. 1996, 243, 457), brain injury (Cannon et al. Crit. Care Med. 1992, 20, 1414; Hansbrough et al. Surg. Clin. N. Am. 1987, 67, 69; Marano et al. Surg. Gynecol. Obstetr. 1990, 170, 32), multiple sclerosis (M.S.; Coyle. Adv. Neuroimmunol. 1996, 6, 143; Matusevicius et al. J. Neuroimmunol. 1996, 66, 115) including demyelation and oligiodendrocyte loss in multiple sclerosis (Brosnan et al. Brain Pathol. 1996, 6, 243), advanced cancer (MucWierzgon et al. J. Biol. Regulators Homeostatic Agents 1996, 10, 25), lymphoid malignancies (Levy et al. Crit. Rev. Immunol. 1996, 16, 31), pancreatitis (Exley et al. Gut 1992, 33, 1126) including systemic complications in acute pancreatitis (McKay et al. Br. J. Surg. 1996, 83, 919), impaired wound healing in infection inflammation and cancer (Buck et al. Am. J. Pathol. 1996, 149, 195), myelodysplastic syndromes (Raza et al. Int. J. Hematol. 1996, 63, 265), systemic lupus erythematosus (Maury et al. Arthritis Rheum. 1989, 32, 146), biliary cirrhosis (Miller et al. Am. J. Gasteroenterolog. 1992, 87, 465), bowel necrosis (Sun et al. J. Clin. Invest. 1988, 81, 1328), psoriasis (Christophers. Austr. J. Dermatol. 1996, 37, S4), radiation injury (Redlich et al. J. Immunol. 1996, 157, 1705), and toxicity following administration of monoclonal antibodies such as OKT3 (Brod et al. Neurology 1996, 46, 1633). TNFα levels have also been related to host-versus-graft reactions (Piguet et al. Immunol. Ser. 1992, 56, 409) including ischemia reperfusion injury (Colletti et al. J. Clin. Invest. 1989, 85, 1333) and allograft rejections including those of the kidney (Maury et al. J. Exp. Med. 1987, 166, 1132), liver (Imagawa et al. Transplantation 1990, 50, 219), heart (Bolling et al. Transplantation 1992, 53, 283), and skin (Stevens et al. Transplant. Proc. 1990, 22, 1924), lung allograft rejection (Grossman et al. Immunol. Allergy Clin. N. Am. 1989, 9, 153) including chronic lung allograft rejection (obliterative bronchitis; LoCicero et al. J. Thorac. Cardiovasc. Surg. 1990, 99, 1059), as well as complications due to total hip replacement (Cirino et al. Life Sci. 1996, 59, 86). TNFα has also been linked to infectious diseases (review: Beutler et al. Crit. Care Med. 1993, 21, 5423; Degre. Biotherapy 1996, 8, 219) including tuberculosis (Rook et al. Med. Malad. Infect. 1996, 26, 904), Helicobacter pylori infection during peptic ulcer disease (Beales et al. Gastroenterology 1997, 112, 136), Chaga's disease resulting from Trypanosoma cruzi infection (Chandrasekar et al. Biochem. Biophys. Res. Commun. 1996, 223, 365), effects of Shiga-like toxin resulting from E. coli infection (Harel et al. J. Clin. Invest. 1992, 56, 40), the effects of enterotoxin A resulting from Staphylococcus infection (Fischer et al, J. Immunol. 1990, 144, 4663), meningococcal infection (Waage et al. Lancet 1987, 355; Ossege et al. J. Neurolog. Sci. 1996, 144, 1), and infections from Borrelia burgdorferi (Brandt et al. Infect. Immunol. 1990, 58, 983), Treponema pallidum (Chamberlin et al. Infect. Immunol. 1989, 57, 2872), cytomegalovirus (CMV; Geist et al. Am. J. Respir. Cell Mol. Biol. 1997, 16, 31), influenza virus (Beutler et al. Clin. Res. 1986, 34, 491a), Sendai virus (Goldfield et al. Proc. Nat'l. Acad. Sci. USA 1989, 87, 1490), Theiler's encephalomyelitis virus (Sierra et al. Immunology 1993, 78, 399), and the human immunodeficiency virus (HIV; Poli. Proc. Nat'l. Acad. Sci. USA 1990, 87, 782; Vyakaram et al. AIDS 1990, 4, 21; Badley et al. J. Exp. Med. 1997, 185, 55).

Because inhibition of p38 leads to inhibition of TNFα production, p38 inhibitors will be useful in treatment of the above listed diseases.

A number of diseases are thought to be mediated by excess or undesired matrix-destroying metalloprotease (MMP) activity or by an imbalance in the ratio of the MMPs to the tissue inhibitors of metalloproteinases (TIMPs). These include osteoarthritis (Woessner et al. J. Biol. Chem. 1984, 259, 3633), rheumatoid arthritis (Mullins et al. Biochim. Biophys. Acta 1983, 695, 117; Woolley et al. Arthritis Rheum. 1977, 20, 1231; Gravallese et al. Arthritis Rheum. 1991, 34, 1076), septic arthritis (Williams et al. Arthritis Rheum. 1990, 33, 533), tumor metastasis (Reich et al. Cancer Res. 1988, 48, 3307; Matrisian et al. Proc. Nat'l. Acad. Sci., USA 1986, 83, 9413), periodontal diseases (Overall et al. J. Periodontal Res. 1987, 22, 81), corneal ulceration (Bums et al. Invest. Opthalmol. Via. Sci. 1989, 30, 1569), proteinuria (Baricos et al. Biochem. J. 1988, 254, 609), coronary thrombosis from atherosclerotic plaque rupture (Henney et al. Proc. Nat'l. Acad. Sci., USA 1991, 88, 8154), aneurysmal aortic disease (Vine et al. Clin. Sci. 1991, 81, 233), birth control (Woessner et al. Steroids 1989, 54, 491), dystrophobic epidermolysis bullosa (Kronberger et al. J. Invest. Dermatol. 1982, 79, 208), degenerative cartilage loss following traumatic joint injury, osteopenias mediated by MMP activity, tempero mandibular joint disease, and demyelating diseases of the nervous system (Chantry et al. J. Neurochem. 1988, 50, 688).

Because inhibition of p38 leads to inhibition of MMP production, p38 inhibitors will be useful in treatment of the above listed diseases.

Inhibitors of p38 are active in animal models of TNFα production, including a muime lipopolysaccharide (LPS) model of TNFα production. Inhibitors of p38 are active in a number of standard animal models of inflammatory diseases, including carrageenan-induced edema in the rat paw, arachadonic acid-induced edema in the rat paw, arachadonic acid-induced peritonitis in the mouse, fetal rat long bone resorption, murine type II collagen-induced arthritis, and Fruend's adjuvant-induced arthritis in the rat. Thus, inhibitors of p38 will be useful in treating diseases mediated by one or more of the above-mentioned cytokines and/or proteolytic enzymes.

The need for new therapies is especially important in the case of arthritic diseases. The primary disabling effect of osteoarthritis, rheumatoid arthritis and septic arthritis is the progressive loss of articular cartilage and thereby normal joint function. No marketed pharmaceutical agent is able to prevent or slow this cartilage loss, although nonsteroidal antiinflammatory drugs (NSAIDs) have been given to control pain and swelling. The end result of these diseases is total loss of joint function which is only treatable by joint replacement surgery. P38 inhibitors will halt or reverse the progression of cartilage loss and obviate or delay surgical intervention.

Several patents have appeared claiming polyarylimidazoles and/or compounds containing polyarylimidazoles as inhibitors of p38 (for example, Lee et al. WO 95/07922; Adams et al. WO 95/02591; Adams et al. WO 95/13067; Adams et al. WO 95/31451). It has been reported that arylimidazoles complex to the ferric form of cytochrome P450_{cam} (Harris et al. Mol. Eng. 1995, 5, 143, and references therein), causing concern that these compounds may display structure-related toxicity (Howard-Martin et al. Toxicol. Pathol. 1987, 15, 369). US 5,162,360 discloses N-substituted aryl-N'-heterocyclic substituted ureas and thioureas, which are useful in treating hypercholesterolemia and artherosclerosis. Further, WO 99/23091 A1 discloses aromatic heterocyclic compounds as anti-inflammatory agents which inhibit cytokines production involved in immunoregulation and inflammation such as interleukin-1 and tumour necrosis factor production.

Therefore, there remains a need for improved p38 inhibitors.

### Summary of the Invention

This invention provides compounds, generally described as aryl ureas, including both aryl and heteroaryl analogues, which inhibit p38 mediated events and thus inhibit the production of cytokines (such as TNFα, IL-1 and IL-8) and proteolytic enzymes (such as MMP-1 and MMP-3). The invention also provides a medicament for treating a cytokine mediated disease state in humans or mammals, wherein the cytokine is one whose production is affected by p38. Examples of such cytokines include, but are not limited to TNFα, IL-1 and IL-8. The invention also provides a medicament for treating a protease mediated disease state in humans or mammals, wherein the protease is one whose production is affected by p38. Examples of such proteases include collagenase (MMP-1) and stromelysin (MMP-3).

Accordingly, these compounds are useful therapeutic agents for such acute and chronic inflammatory and/or immunomodulatory diseases as rheumatoid arthritis, osteoarthritis, septic arthritis, rheumatic fever, bone resorption, postmenopausal osteoperosis, sepsis, gram negative sepsis, septic shock, endotoxic shock, toxic shock syndrome, systemic inflammatory response syndrome, inflammatory bowel diseases including Crohn's disease and ulcerative colitis, Jarisch-Herxheimer reactions, asthma, adult respiratory distress syndrome, acute pulmonary fibrotic diseases, pulmonary sarcoidosis, allergic respiratory diseases, silicosis, coal worker's pneumoconiosis, alveolar injury, hepatic failure, liver disease during acute inflammation, severe alcoholic hepatitis, malaria including Plasmodium falciparum malaria and cerebral malaria, non-insulin-dependent diabetes mellitus (NIDDM), congestive heart failure, damage following heart disease, atherosclerosis, Alzheimer's disease, acute encephalitis, brain injury, multiple sclerosis including demyelation and oligiodendrocyte loss in multiple sclerosis, advanced cancer, lymphoid malignancies, tumor metastasis, pancreatitis, including systemic complications in acute pancreatitis, impaired wound healing in infection, inflammation and cancer, periodontal diseases, corneal ulceration, proteinuria, myelodysplastic syndromes, systemic lupus erythematosus, biliary cirrhosis, bowel necrosis, psoriasis, radiation injury, toxicity following administration of monoclonal antibodies such as OKT3, host-versus-graft reactions including ischemia reperfusion injury and allograft rejections including kidney, liver, heart, and skin allograft rejections, lung allograft rejection including chronic lung allograft rejection (obliterative bronchitis) as well as complications due to total hip replacement, and infectious diseases including tuberculosis, Helicobacter pylori infection during peptic ulcer disease, Chaga's disease resulting from Trypanosoma cruzi infection, effects of Shiga-like toxin resulting from E. coli infection, effects of enterotoxin A resulting from Staphylococcus infection, meningococcal infection, and infections from Borrelia burgdorferi, Treponema pallidum, cytomegalovirus, influenza virus, Theiler's encephalomyelitis virus, and the human immunodeficiency virus (HIV).

Accordingly, the present invention is directed to the use of a compound of formula I 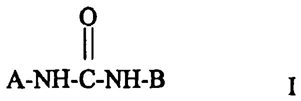 wherein B is selected from the group consisting of 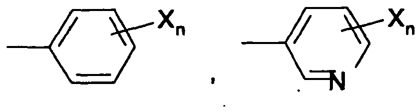 which is substituted or unsubstituted by halogen, up to per-halosubstitution, and
wherein n = 0-3 and each X is independently selected from the group consisting of -CN, -CO₂R⁵, -C(O)NR⁵R^{5'}, -C(O)R⁵, -NO₂, -OR⁵, - SR⁵, - NR⁵R^{5'}, -NR⁵C(O)OR^{5'}, -NR⁵C(O)R^{5'}, C₁-C₁₀ alkyl, C₂₋₁₀-alkenyl, C₁₋₁₀-alkoxy, C₃-C₁₀ cycloalkyl, C₆-C₁₄ aryl, C₇-C₂₄ alkaryl, C₃-C₁₃ heteroaryl, C₄-C₂₃ alkheteroaryl, and substituted C₁-C₁₀ alkyl, substituted C₂₋₁₀-alkenyl, substituted C₁-₁₀-alkoxy, substituted C₃-C₁₀ cycloalkyl, substituted C₄-C₂₃ alkheteroaryl and -Y-Ar;
wherein if X is a substituted group, it is substituted by one or more substituents independently selected from the group consisting of -CN, -CO₂R⁵, -C(O)R⁵, -C(O)NR⁵R^{5'}, -OR⁵, -SR⁵, -NR⁵R^{5'}, NO₂, -NR⁵C(O)R^{5'}, -NR⁵C(O)OR^{5'} and halogen up to per-halosubstitution;
wherein R⁵ and R^{5'} are independently selected from H, C₁-C₁₀ alkyl, C₂₋₁₀-alkenyl, C₃-C₁₀ cycloalkyl, C₆-C₁₄ aryl, C₃-C₁₃ heteroaryl, C₇-C₂₄ alkaryl, C₄-C₂₃, alkheteroaryl, up to per-halosubstituted C₁-C₁₀ alkyl, up to per-halosubstituted C₂₋₁₀-alkenyl, up to per-halosubstituted C₃-C₁₀ cycloalkyl, up to per-halosubstituted C₆-C₁₄ aryl and up to per-halosubstituted C₃-C₁₃ heteroaryl,
wherein Y is - O-, or -S-,
and
Ar is a 5-10 member aromatic structure containing 0-4 members of the group consisting of nitrogen, oxygen and sulfur which is unsubstituted or substituted by halogen up to per-halo and optionally substituted by Zₙ₁, wherein nl is 0 to 3 and each Z is independently selected from the group consisting of -CN, -CO₂R⁵, -C(O)R⁵, =O, -SO₂R⁵, -SO₂NR⁵R^{5'}, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀ alkyl, C₁-C₁₀ alkoxy, C₃-C₁₀ cycloalkyl, C₆-C₁₄ aryl, C₃-C₁₃ heteroaryl, C₇-C₂₄ alkaryl, C₄-C₂₃, alkheteroaryl, substituted C₁-C₁₀ alkyl, substituted C₃-C₁₀ cycloalkyl, substituted C₇-C₂₄ alkaryl and substituted C₄-C₂₃ alkheteroaryl; wherein if Z is a substituted group, it is substituted by one or more substituents independently selected from the group consisting of -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, C₁-C₁₀ alkyl, C₁-C₁₀ alkoxy, C₃-C₁₀ cycloalkyl, C₃-C₁₀ heteroaryl, C₆-C₁₄ aryl, C₄-C₂₄ alkheteroaryl and C₇-C₂₄ alkaryl, and
A is 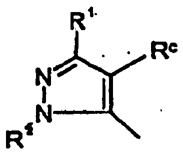 wherein
R¹ is selected from the group consisting of halogen, C₃-C₁₀ alkyl, C₃-C₁₀ cycloalkyl, C₁-C₁₃ heteroaryl, C₆-C₁₄ aryl, C₇-₂₄ alkaryl, up to per-halosubstituted C₁-C₁₀ alkyl, up to per-halosubstituted C₃-C₁₀ cycloalkyl, up to per-halosubstituted C₁-C₁₃ heteroaryl, up to per-halosubstituted C₆-₁₄ aryl, and up to per-halosubstituted C₇-₂₄ alkaryl;
R² is selected from the group consisting of H, -C(O)R⁴, -CO₂R₄, -C(O)NR³R^{3'}, C₁-C₁₀ alkyl, C₃-C₁₀ cycloalkyl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl,
where R² is a substituted group, it is substituted by one or more substituents independently selected from the group consisting of -CN, - CO₂R⁴, -C(O)-NR³R^{3'}, -NO₂, -OR⁴, -SR⁴, and halogen up to per-halosubstitution,
wherein R³ and R^{3'} are independently selected from the group consisting of H, -OR⁴, -SR⁴, -NR⁴R^{4'}, -C(O)R⁴, -CO₂R⁴, -C(O)NR⁴R^{4'}, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstituted C₃-C₁₀ cycloalkyl, up to per-halosubstituted C₆-C₁₄ aryl and up to per-halosubstituted C₃-C₁₃ heteroaryl; and
wherein R⁴ and R^{4'} are independently selected from the group consisting of H, C₁-C₁₀ alkyl, C₃-C₁₀ cycloalkyl, C₆-C₁₄ aryl, C₃-C₁₃ heteroaryl; C₇-C₂₄ alkaryl, C₄-C₂₃ alkheteroaryl, up to per-halosubstituted C₁-C₁₀ alkyl, up to per-halosubstituted C₃-C₁₀ cycloalkyl, up to per-halosubstituted C₆-C₁₄ aryl and up to per-halosubstituted C₃-C₁₃ heteroaryl,
and
R^{c} is hydrogen, halogen, C₁-C₁₀ alkyl, up to per-halosubstituted C₁-C₁₀ alkyl or combines with R¹ and the ring carbon atoms to which R¹ and R^{c} are bound to form a 5- or 6-membered cycloalkyl, aryl or heteroaryl ring with 0-2 members selected from O, N and S, and pharmaceutically acceptable salts thereof for the preparation of a medicament for the treatment of a disease mediated by p38 other than cancer, wherein the disease is an inflammatory or immunomodulatory disease.

The aryl and heteroaryl moiety of B contain separate cyclic structures. The substituents for these aryl and heteroaryl moieties can vary widely and include halogen, hydrogen, hydrosulfide, cyano, nitro, amines and various carbon-based moieties, including those which contain one or more of sulfur, nitrogen, oxygen and/or halogen and are discussed more particularly below.

Examples of suitable aromatic ring structures include phenyl, pyridinyl, naphthyl, pyrimidinyl, benzothiazolyl, quinoline, isoquinoline, phthalimidinyl and combinations thereof, such as diphenyl ether (phenyloxyphenyl), diphenyl thioether (phenylthiophenyl), diphenyl amine (phenylaminophenyl), phenylpyridinyl ether (pyridinyloxyphenyl), pyridinylmethylphenyl, phenylpyridinyl thioether (pyridinylthiophenyl), phenylbenzothiazolyl ether (benzothiazolyloxyphenyl), phenylbenzothiazolyl thioether (benzothiazolylthiophenyl), phenylpyrimidinyl ether, phenylquinoline thioether, phenylnaphthyl ether, pyridinylnapthyl ether, pyridinylnaphthyl thioether, and phenylphthalimidylmethyl.

Examples of suitable heteroaryl groups include, but are not limited to, 5-12 carbon-atom aromatic rings or ring systems containing 1-3 rings, at least one of which is aromatic, in which one or more, e.g., 1-4 carbon atoms in one or more of the rings can be replaced by oxygen, nitrogen or sulfur atoms. Each ring typically has 3-7 atoms. For example, B can be 2- or 3-furyl, 2- or 3-thienyl, 2- or 4-triazinyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-, -4- or -5-yl, 1,2,4-triazol-1-, -3- or-5-yl, 1- or 5-tetrazolyl, 1,2,3-oxadiazol-4- or -5-yl, 1,2,4-oxadiazol-3- or -5-yl, 1,3,4-thiadiazol-2-or -5-yl, 1,2,4-oxadiazol-3- or -5-yl, 1,3,4-thiadiazol-2- or -5-yl, 1,3,4-thiadiazol-3-or -5-yl, 1,2,3-thiadiazol-4- or -5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6- or 7-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5- 6- or 7-benzisoxazolyl, 1-, 3-, 4-, 5-, 6- or 7-benzothiazolyl, 2-, 4-, 5-, 6- or 7-benzisothiazolyl, 2-, 4-, 5-, 6- or 7-benz-1,3-oxadiazolyl, 2-, 3-, 4-, 5-, 6-, 7- or 8-quinolinyl, 1-, 3-, 4-, 5-, 6-, 7-, 8- isoquinolinyl, 1-, 2-, 3-, 4- or 9-carbazolyl, 1-, 2-, 3-, 4-, 5-, 6-, 7-, 8- or 9-acridinyl, or 2-, 4-, 5-, 6-, 7- or 8-quinazolinyl, or additionally optionally substituted phenyl, 2- or 3-thienyl, 1,3,4-thiadiazolyl, 3-pyrryl, 3-pyrazolyl, 2-thiazolyl or 5-thiazolyl, etc. For example, B can be 4-methyl-phenyl, 5-methyl-2-thienyl, 4-methyl-2-thienyl, 1-methyl-3-pyrryl, 1-methyl-3-pyrazolyl, 5-methyl-2-thiazolyl or 5-methyl-1,2,4-thiadiazol-2-yl.

Suitable alkyl groups and alkyl portions of groups, e.g., alkoxy throughout include methyl, ethyl, propyl, butyl, including all straight-chain and branched isomers such as isopropyl, isobutyl, *sec*-butyl, *tert*-butyl.

Suitable aryl groups include, for example, phenyl and 1- and 2-naphthyl.

Suitable cycloalkyl groups include cyclopropyl, cyclobutyl, cyclohexyl. The term "cycloalkyl", as used herein, refers to cyclic structures with or without alkyl substituents such that, for example, "C₄ cycloalkyl" includes methyl substituted cyclopropyl groups as well as cyclobutyl groups. The term "cycloalkyl" also includes saturated heterocyclic groups.

Suitable halogens include F, Cl, Br, and/or I, from one to persubstitution (i.e., all H atoms on the group are replaced by halogen atom), being possible, mixed substitution of halogen atom types also being possible on a given moiety.

As indicated above, these ring systems can be unsubstituted or substituted by substituents such as halogen up to per-halosubstitution. Other suitable substituents for the moieties of B include alkyl, alkoxy, carboxy, cycloalkyl, aryl, heteroaryl, cyano, hydroxy and amine. These other substituents, generally referred to as X herein, include -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀ alkyl, C₂-C₁₀ alkenyl, C₁-C₁₀ alkoxy, C₃-C₁₀ cycloalkyl, C₆-C₁₄ aryl, C₇-C₂₄ alkaryl, C₃-C₁₃ heteroaryl, C₄-C₂₃ alkheteroaryl, substituted C₁-C₁₀ alkyl, substituted C₂-C₁₀ alkenyl, substituted C₁-C₁₀ alkoxy, substituted C₃-C₁₀ cycloalkyl, substituted C₄-C₂₃ alkheteroaryl and -Y-Ar.

Where a substituent X is a substituted group, it is preferably substituted by one or more substituents independently selected from the group consisting of -CN, -CO₂R⁵,-C(O)R⁵, -C(O)NR⁵R^{5'}, -OR⁵, -SR⁵, -NR⁵R^{5'}, -NO₂, -NR^{5'}C(O)R^{5'}, -NR⁵C(O)OR^{5'} and halogen up to per-halo substitution.

The moieties R⁵ and R^{5'} are preferably independently selected from H, C₁-C₁₀ alkyl, C₂-C₁₀ alkenyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstituted C₂-C₁₀ alkenyl, up to per-halosubstituted C₃-C₁₀ cycloalkyl, up to per-halosubstituted C₆-C₁₄ aryl and up to per-halosubstituted C₁-C₁₃ heteroaryl.

The bridging group Y is preferably -O- or -S-.

The moiety Ar is preferably a 5-10 member aromatic structure containing 0-4 members of the group consisting of nitrogen, oxygen and sulfur which is unsubstituted or substituted by halogen up to per-halosubstitution and optionally substituted by Zₙ₁, wherein n1 is 0 to 3.

Each Z substituent is preferably independently selected from the group consisting of -CN, -CO₂R⁵, =O, -C(O)NR⁵R^{5'}, -C(O)-NR⁵, -NO₂, -OR⁵, -SR⁵, -NR⁵R^{5'}, -NR⁵C(O)OR^{5'}, -C(O)R⁵, -NR⁵C(O)R^{5'}, -SO₂R⁵, -SO₂NR⁵R^{5'}, C₁-C₁₀ alkyl, C₁-C₁₀ alkoxy, C₃-C₁₀ cycloalkyl, C₆-C₁₄ aryl, C₃-C₁₃ heteroaryl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl. If Z is a substituted group, it is substituted by the one or more substituents independently selected from the group consisting of -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, C₁-C₁₀ alkyl, C₁-C₁₀ alkoxy, C₃-C₁₀ cycloalkyl, C₁-C₁₀ heteroaryl, C₆-C₁₄ aryl, C₄-C₂₄ alkheteroaryl and C₇-C₂₄ alkaryl.

The aryl and heteroaryl moieties of B of Formula I are selected from the group consisting of 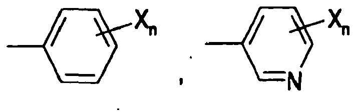 which are unsubstituted or substituted by halogen, up to per-halosubstitution. X is as defined above and n = 0.3.

The aryl and heteroaryl moieties of B are more preferably of the formula II: 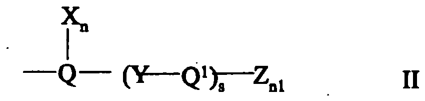 wherein Y is selected from the group consisting of -O- or -S-.

Q is phenyl or 3-pyridinyl, substituted or unsubstituted by halogen, up to perhalosubstitution and Q¹ is a mono- or bicyclic aromatic structure of 3 to 10 carbon atoms and 0-4 members of the group consisting of N, O and S, unsubstituted or unsubstituted by halogen up to per-halosubstitution. X, Z, n and nl are as defined above and s = 0 or 1.

In preferred embodiments, Q is phenyl or 3-pyridinyl, substituted or unsubstituted by halogen, up to per-halosubstitution and Q¹ is selected from the group consisting of phenyl, pyridinyl, naphthyl, pyrimidinyl, quinoline, isoquinoline, imidazole and benzothiazolyl, substituted or unsubstituted by halogen, up to per-halo substitution, or -Y-Q¹ is phthalimidinyl substituted or unsubstituted by halogen up to per-halo substitution. Z and X are preferably independently selected from the group consisting of -R⁶, -OR⁶ and -NHR⁷, wherein R⁶ is hydrogen, C₁-C₁₀-akyl or C₃-C₁₀-cycloalkyl and R⁷ is preferably selected from the group consisting of hydrogen, C₃-C₁₀-alkyl, C₃-C₆-cycloalkyl and C₆-C₁₀-aryl, wherein R⁶ and R⁷ can be substituted by halogen or up to per-halosubstitution.

The heteroaryl moiety A of formula I is preferably selected from the group consisting of:

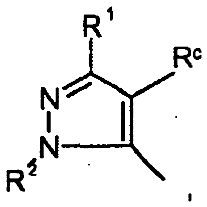

The substituent R¹ preferably is selected from the group consisting of halogen, C₃-C₁₀ alkyl, C₁-C₁₃ heteroaryl, C₆-C₁₄ aryl, C₇-C₂₄ alkylaryl, C₃-C₁₀ cycloalkyl, up to per-halosubstituted C₁-C₁₀ alkyl and up to per-halosubstituted C₃-C₁₀ cycloalkyl, up to per-halosubstituted C₁-C₁₃ hetero, up to per-halosubstituted C₆-C₁₃ aryl and up to per-halosubstituted C₇-C₂₄ alkaryl.

The substituent R² is preferably selected from the group consisting of H, -C(O)R⁴, -CO₂R⁴, -C(O)NR³R^{3'}, C₁-C₁₀ alkyl, C₃-C₁₀ cycloalkyl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl. Where R² is a substituted group, it is preferably substituted by one or more substituents independently selected from the group consisting of -CN, - CO₂R⁴, -C(O)-NR³R^{3'}, -NO₂, -OR⁴, -SR⁴, and halogen up to per-halosubstitution.

R³ and R^{3'} are preferably independently selected from the group consisting of H, -OR⁴, -SR⁴, -NR⁴R^{4'}, -C(O)R⁴, -CO₂R⁴, -C(O)NR⁴R^{4'}, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstituted C₃-C₁₀ cycloalkyl, up to per-halosubstituted C₆-C₁₄ aryl and up to per-halosubstituted C₃-C₁₃ heteroaryl.

R⁴ and R⁴ are preferably independently selected from the group consisting of H, C₁-C₁₀ alkyl, C₃-C₁₀ cycloalkyl, C₆-C₁₄ aryl, C₃-C₁₃ heteroaryl; C₇-C₂₄ alkaryl, C₄-C₂₃ alkheteroaryl, up to per-halosubstituted C₁-C₁₀ alkyle, up to per-halosubstituted C₃-C₁₀ cycloalkyl, up to per-halosubstituted C₆-C₁₄ aryl and up to per-halosubstituted C₃-C₁₃ heteroaryl.

R^{c} is hydrogen, halogen, C₁-C₁₀ alkyl, up to per-halosubstituted C₁-C₁₀ alkyl or combines with R¹ and the ring carbon atoms to which R¹ and R^{c} are bound to form a 5- or 6-membered cycloalkyl, aryl or heteroaryl ring with 0-2 members selected from O, N and S.

A preferred compound of formula I is a compound of the formula 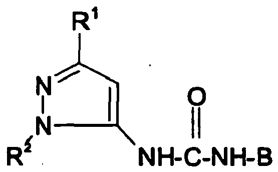 wherein R¹ and R² and B are defined as above.

R¹ is particularly preferred t-butyl.

Preferred pyrazolyl ureas include those wherein B is 2,3-dichlorophenyl or of the formula II above, wherein Q is phenyl, Q¹ is phenyl or pyridinyl, Y ix -0-, -S-, -CH₂ or -SCH₂, X is CF₃, Z is OH, Cl or -NHC(O)-CₚH₂ₚ₊₁, wherein p = 2-4, s = 0 or 1, n = 0 or 1 and n1 = 0 or 1.

Particular preferred pyrazolyl ureas include:
*N*-(3-*tert*-Butyl-5-pyrazolyl)-*N'*-(4-(2,3-dichlorophenyl)urea;
*N*-(3-*tert*- Butyl-5-pyrazolyl)-*N'*-(3-(4-pyridinyl)thiophenyl)urea;
*N*-(3-*tert*-Butyl-5-pyrazolyl)-*N'*-(4-(4-pyridinyl)methylphenyl)urea;
*N*-(3-*tert*-Butyl-5-pyrazolyl)-*N'*-(4-(4-pyridinyl)oxyphenyl)urea;
*N*-(3-*tert*-Butyl-5-pyrazolyl)-*N'*-(4-(4-pyridinyl)thiophenyl)urea;
*N*-(3-*tert*-Butyl-5-pyrazolyl)-*N'*-(4-(4-pyridinyl)methylphenyl)urea;
*N*-(1-Methyl-3-tert-butyl-5-pyrazolyl)-*N'*-(2,3-dichlorophenyl)urea;
*N*-(1-Methyl-3-*tert*-butyl-5-pyrazolyl)-*N'*-(4-(4-hydroxyphenyl)-thiophenyl)urea;
*N*-(1-Methyl-3-*tert*-butyl-5-pyrazolyl)-*N'*-(4-(4-ethylaminocarbonylphenyl)-oxyphenyl)urea;
*N*-(1-Methyl-3-*tert*-butyl-5-pyrazolyl)-*N'*-(4-(4-isobutylaminocarbonyl-phenyl)-thiophenyl)urea;
*N*-(1-Methyl-3-*tert*-butyl-5-pyrazolyl)-*N'*-(4-(4-pyridinyl)thiophenyl)urea;
*N*-(1-Methyl-3-*tert*-butyl-5-pyrazolyl)-*N'*-(3-(4-pyridinyl)thiophenyl)urea;
*N*-(1-Methyl-3-*tert*-butyl-5-pyrazolyl)-*N'*-(4-(4-pyridinyl)thio-3-(trifluoromethyl)-phenyl)urea;
*N*-(1-Methyl-3-*tert*-butyl-5-pyrazolyl)-*N'*-(4-(4-pyridinyl)oxyphenyl)urea;
*N*-(1-Methyl-3-*tert*-butyl-5-pyrazolyl)-*N'*-(4-((4-pyridinyl)methylthio)-phenyl)urea;
*N*-(1-(2,2,2-Trifluoroethyl)-3-*tert*-butyl-5-pyrazolyl)-*N'*-(2,3-dichlorophenyl)urea;
*N*-(1-(2-Hydroxyethyl)-3-*tert*-butyl-5-pyrazolyl)-N'-(2,3-dichlorophenyl)urea;
*N*-(1-Ethoxycarbonylmethyl-3-*tert*-butyl-5-pyrazolyl)-*N'*-(2,3-dichlorophenyl)urea;
*N*-(1-(2-Cyanoethyl)-3-*tert*-butyl-5-pyrazolyl)-*N'*-(2,3-dichlorophenyl)urea;
*N*-(1-(3-Hydroxyphenyl)methyl-3-*tert*-butyl-5-pyrazolyl)-*N'*-(2,3-dichlorophenyl)-urea;
*N*-(1-Cyclohexyl-3-*tert*-butyl-5-pyrazolyl)-n'-(4-(4-pyridinyl)methyl-phenyl)urea;
*N*-(1-methyl-3-phenyl-5-pyrazolyl)-*N'*-(3-(4-(2-methylcarbamoyl)pyridyl)-thiophenyl) urea;
*N*-(1-methyl-3-tert-butyl-5-pyrazolyl)-*N'*-(4-(4-pyridyl)thiophenyl) urea;
*N*-(1-methyl-3-tert-butyl-5-pyrazolyl)-*N'*-(3-(4-pyridyl)thiophenyl) urea;
*N*-(1-methyl-3-tert-butyl-5-pyrazolyl)-*N'*-(3-trifluoromethyl-4-(4-pyridylthio)phenyl) urea;
*N*-(3-tert-butyl-5-pyralyl)-N'-(3-(4-pyridyl)oxyphenyl) urea; and
*N*-(3-tert-butyl-5-pyrazolyl)-*N'*-(4-(4-pyridyl)oxyphenyl) urea.

The invention also relates to which are within the scope of general formula I described above and more specifically include compounds of the formulae:
5d) 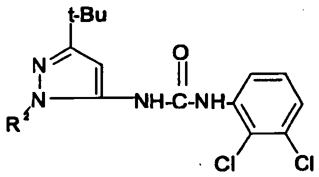 wherein R² is -CH₂-CF₃ , -C₂H₄-OH, -CH₂-(3-HOC₆H₄), -CH₂C(O)NH₃, -CH₂C(O)OC₂H₅, -C₂H₄CN, or 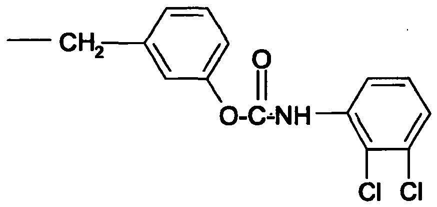 and
e) 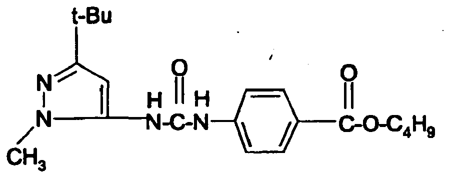 and pharmaceutically acceptable salts thereof.

The present invention is also directed to pharmaceutically acceptable salts of formula I. Suitable pharmaceutically acceptable salts are well known to those skilled in the art and include basic salts of inorganic and organic acids, such as hydrochloric acid, hydrobromic acid, sulphuric acid, phosphoric acid, methanesulphonic acid, sulphonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid. In addition, pharmaceutically acceptable salts include acid salts of inorganic bases, such as salts containing alkaline cations (e.g., Li⁺ Na⁺ or K⁺), alkaline earth cations (e.g., Mg⁺², Ca⁺² or Ba⁺²), the ammonium cation, as well as acid salts of organic bases, including aliphatic and aromatic substituted ammonium, and quaternary ammonium cations such as those arising from protonation or peralkylation of triethylamine, *N,N*-diethylamine, *N,N*-dicyclohexylamine, pyridine, *N,N*-dimethylaminopyridine (DMAP), 1,4-diazabiclo[2.2.2]octane (DABCO), 1,5-diazabicyclo[4.3.0]non-5-ene (DBN) and 1,8-diazabicyclo[5.4.0]undec-7-ene (DBU).

A number of the compounds of Formula I possess asymmetric carbons and can therefore exist in racemic and optically active forms. Methods of separation of enantiomeric and diastereomeric mixtures are well known to one skilled in the art.

The present invention encompasses any isolated racemic or optically active form of compounds described in Formula I which possess p38 kinase inhibitory activity .

According to an embodiment of the invention the compound for formula I displays p38 activity (IC₅₀) better than 10 µm as determined by an in-vitro kinase assay.

According to an embodiment of the invention the disease is mediated by a cytokine or protease regulated by p38.

According to an embodiment of the invention the amount of the compound of formula I is effective to inhibit p38.

According to an embodiment of the invention the amount of the compound of formula I is effective to inhibit production of a disease-mediating cytokine or protease. According to an embodiment of the invention the disease is mediated by TNFα, MMP-1, MMP-3, IL-1, IL-6 or IL-8.

According to an embodiment of the invention the disease is an inflammatory disease.

According to an embodiment of the invention the disease is an immunomodulatory disease.

According to an embodiment of the invention the disease is rheumatoid arthritis, osteoporosis, osteoarthritis, asthma, septic shock, inflammatory bowel disease, or the result of host-versus-graft reactions.

### General Preparative Methods

The compounds of Formula I may be prepared by use of known chemical reactions and procedures, some from starting materials which are commercially available. Nevertheless, the following general preparative methods are presented to aid one of skill in the art in synthesizing the inhibitors, with more detailed particular examples being presented in the experimental section describing the working examples.

Heterocyclic amines may be synthesized utilizing known methodology (Katritzky, et al. Comprehensive Heterocyclic Chemistry; Permagon Press: Oxford, UK (1984). March. Advanced Organic Chemistry, 3rd Ed.; John Wiley: New York (1985)). For example, 3-substituted-5-aminoisoxazoles **(3)** are available by the reaction of hydroxylamine with an α-cyanoketone **(2)**, as shown in Scheme I. Cyanoketone **2,** in turn, is available from the reaction of acetamidate ion with an appropriate acyl derivative, such as an ester, an acid halide, or an acid anhydride. Reaction of an α-cyanoketone with hydrazine (R²=H) or a monosubstituted hydrazine affords the 3-substituted- or 1,3-disubstituted-5-aminopyrazole **(5).** Pyrazoles unsubstituted at *N*-1 (R²=H) may be acylated at *N*-1, for example using di-*tert*-butyl dicarbonate, to give pyrazole **7**. Similarly, reaction of nitrile **8** with α-thioacetate ester gives the 5-substituted-3-amino-2-thiophenecarboxylate (**9,** Ishizaki et al. JP 6025221). Decarboxylation of ester **9** may be achieved by protection of the amine, for example as the *tert*-butoxy (BOC) carbamate **(10),** followed by saponification and treatment with acid. When BOC protection is used, decarboxylation may be accompanied by deprotection giving the substituted 3-thiopheneammonium salt **11**. Alternatively, ammonium salt **11** may be directly generated through saponification of ester **9** followed by treatment with acid.

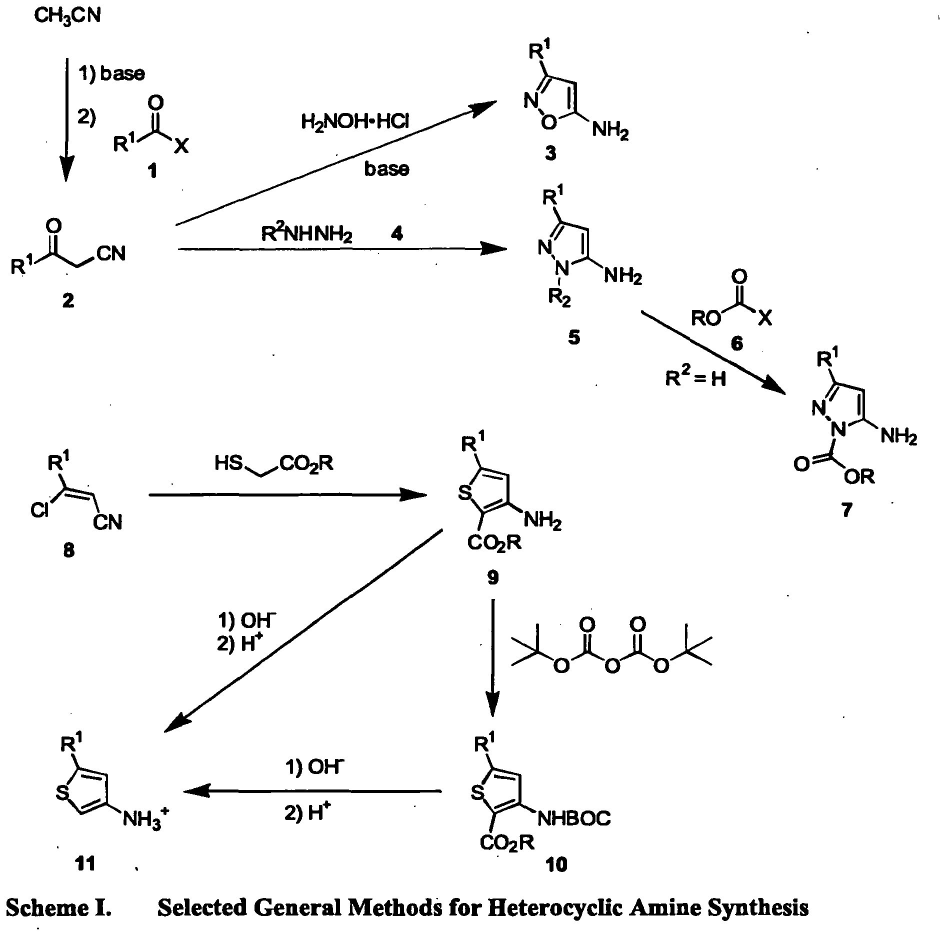

Substituted anilines may be generated using standard methods (March. Advanced Organic Chemistry, 3rd Ed.; John Wiley: New York (1985). Larock. Comprehensive Organic Transformations; VCH Publishers: New York (1989)). As shown in Scheme II, aryl amines are commonly synthesized by reduction of nitroaryls using a metal catalyst, such as Ni, Pd, or Pt, and H₂ or a hydride transfer agent, such as formate, cyclohexadiene, or a borohydride (Rylander. Hydrogenation Methods; Academic Press: London, UK (1985)). Nitroaryls may, also be directly reduced using a strong hydride source, such as LiAlH₄ (Seyden-Penne. Reductions by the Alumino- and Borohydrides in Organic Synthesis; VCH Publishers: New York (1991)), or using a zero valent metal, such as Fe, Sn or Ca, often in acidic media. Many methods exist for the synthesis of nitroaryls (March. Advanced Organic Chemistry, 3rd Ed.; John Wiley: New York (1985). Larock. Comprehensive Organic Transformations; VCH Publishers: New York (1989)).

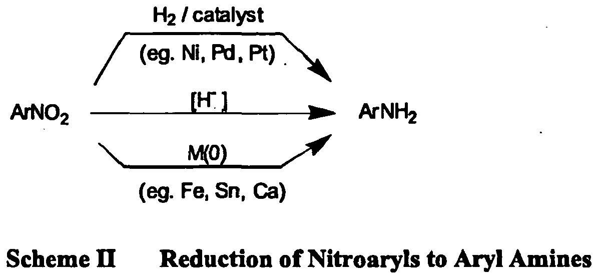

Nitroaryls are commonly formed by electrophilic aromatic nitration using HNO₃, or an alternative NO₂⁺ source. Nitroaryls may be further elaborated prior to reduction. Thus, nitroaryls substituted with

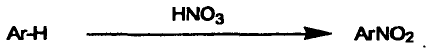

potential leaving groups (eg. F, Cl, Br, etc.) may undergo substitution reactions on treatment with nucleophiles, such as thiolate (exemplified in Scheme III) or phenoxide. Nitroaryls may also undergo Ullman-type coupling reactions (Scheme III).

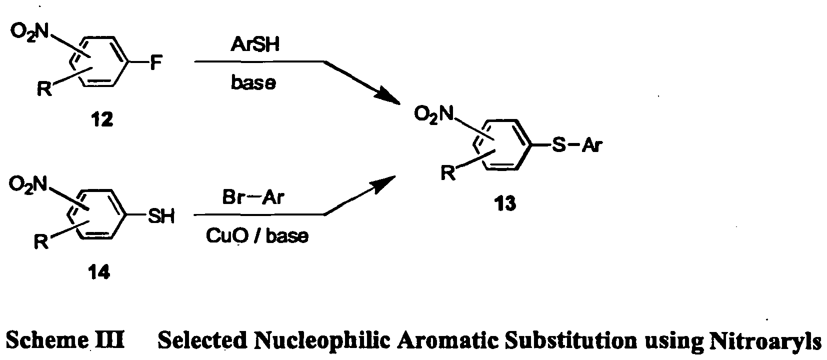

As shown in Scheme IV, urea formation may involve reaction of a heteroaryl isocyanate **(17)** with an aryl amine **(16).** The heteroaryl isocyanate may be synthesized from a heteroaryl amine by treatment with phosgene or a phosgene equivalent, such as trichloromethyl chloroformate (diphosgene), bis(trichloromethyl) carbonate (triphosgene), or N,N'-carbonyldiimidazole (CDI). The isocyanate may also be derived from a heterocyclic carboxylic acid derivative, such as an ester, an acid halide or an anhydride by a Curtius-type rearrangement. Thus, reaction of acid derivative **21** with an azide source, followed by rearrangement affords the isocyanate. The corresponding carboxylic acid **(22)** may also be subjected to Curtius-type rearrangements using diphenylphosphoryl azide (DPPA) or a similar reagent. A urea may also be generated from the reaction of an aryl isocyanate **(20)** with a heterocyclic amine.

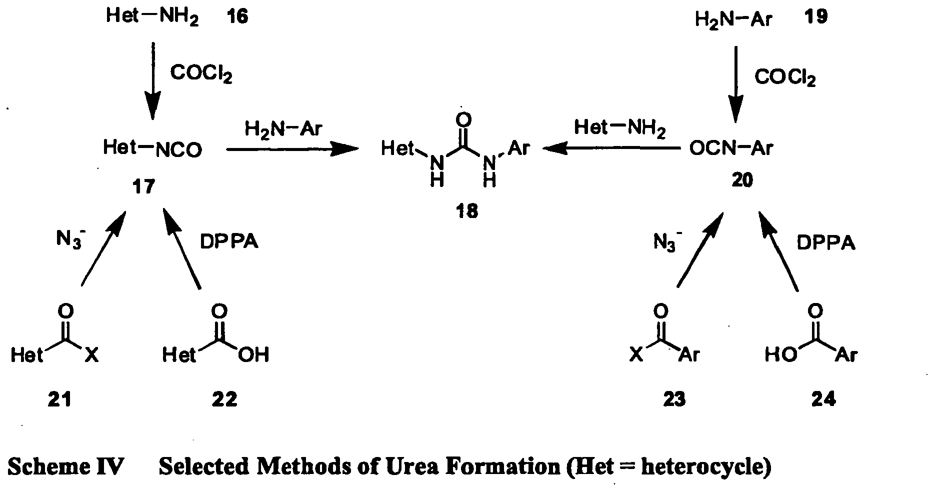

1-Amino-2-heterocyclic carboxylic esters (exemplified with thiophene **9**, Scheme V) may be converted into an isatoic-like anhydride **(25)** through saponification, followed by treatment with phosgene or a phosgene equivalent. Reaction of anhydride **25** with an aryl amine can generate acid **26** which may spontaneously decarboxylate, or may be isolated. If isolated, decarboxylation of acid **26** may be induced upon heating.

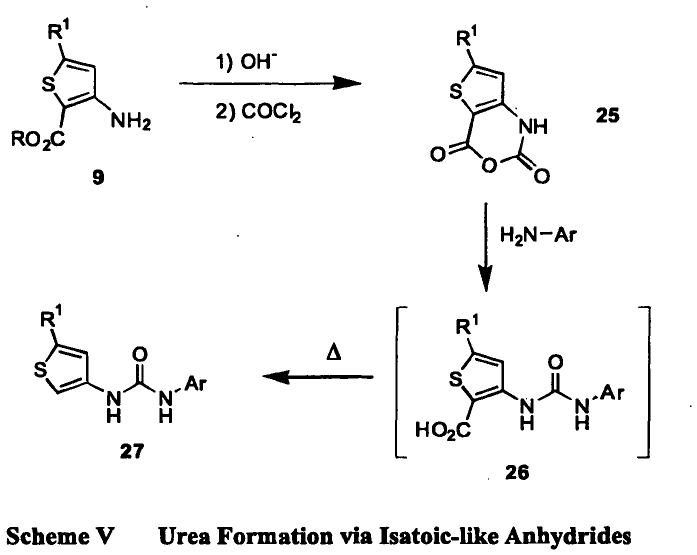

Finally, ureas may be further manipulated using methods familiar to those skilled in the art.

The invention also includes pharmaceutical compositions including a compound of this invention as described above, or a pharmaceutically acceptable salt thereof, and a physiologically acceptable carrier.

The compounds may be administered orally, topically, parenterally, by inhalation or spray, sublingually, or rectally or vaginally in dosage unit formulations. The term 'administration by injection' includes intravenous, intramuscular, subcutaneous and parenteral injections, as well as use of infusion techniques. Dermal administration may include topical application or transdermal administration. One or more compounds may be present in association with one or more non-toxic pharmaceutically acceptable carriers and if desired other active ingredients.

Compositions intended for oral use may be prepared according to any suitable method known to the art for the manufacture of pharmaceutical compositions. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions containing the active materials in admixture with excipients suitable for the manufacture of aqueous suspensions may also be used. Such excipients are suspending agents, for example sodium carboxymethylcellulose, methylcellulose, hydroxypropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The compounds may also be in the form of non-aqueous liquid formulations, e.g., oily suspensions which may be formulated by suspending the active ingredients in a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Pharmaceutical compositions of the invention may also be in the form of oil-in-water emulsions. The oil phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The compounds may also be administered in the form of suppositories for rectal administration of the drug. These compositions can be prepared by mixing the drug with a suitable non-irritating excipient which is solid at ordinary temperatures but liquid at the rectal or vaginal temperature and will therefore melt in the rectum or vagina to release the drug. Such materials include cocoa butter and polyethylene glycols.

Compounds of the invention may also be administrated transdermally using methods known to those skilled in the art (see, for example: Chien; "Transdermal Controlled Systemic Medications"; Marcel Dekker, Inc.; 1987. Lipp et al. WO94/04157 3Mar94). For example, a solution or suspension of a compound of Formula I in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of a compound of Formula I may be formulated into a lotion or salve.

Suitable solvents for processing transdermal delivery systems are known to those skilled in the art, and include lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents may also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery system are known to those skilled in the art, and include, for example, monohydroxy or polyhydroxy alcohols such as ethanol, propylene glycol or benzyl alcohol, saturated or unsaturated C₈-C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, n-butyl, sec-butyl isobutyl tertbutyl or monoglycerin esters of acetic acid, capronic acid, lauric acid, myristinic acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures of one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene coploymers, and natural and synthetic rubbers. Cellulose ethers, derivatized polyethylenes, and silicates may also be used as matrix components. Additional additives, such as viscous resins or oils may be added to increase the viscosity of the matrix.

For all regimens of use disclosed herein for compounds of Formula I, the daily oral dosage regimen will preferably be from 0.01 to 200 mg/Kg of total body weight. The daily dosage for administration by injection, including intravenous, intramuscular, subcutaneous and parenteral injections, and use of infusion techniques will preferably be from 0.01 to 200 mg/Kg of total body weight. The daily rectal dosage regimen will preferably be from 0.01 to 200 mg/Kg of total body weight. The daily vaginal dosage regimen will preferably be from 0.01 to 200 mg/Kg of total body weight. The daily topical dosage regimen will preferably be from 0.1 to 200 mg administered between one to four times daily. The transdermal concentration will preferably be that required to maintain a daily dose of from 0.01 to 200 mg/Kg. The daily inhalation dosage regimen will preferably be from 0.01 to 10 mg/Kg of total body weight.

It will be appreciated by those skilled in the art that the particular method of administration will depend on a variety of factors, all of which are considered routinely when administering therapeutics.

It will also be understood, however, that the specific dose level for any given patient will depend upon a variety of factors, including, the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy.

It will be further appreciated by one skilled in the art that the optimal course of treatment, ie, the mode of treatment and the daily number of doses of a compound of Formulae I or a pharmaceutically acceptable salt thereof given for a defined number of days, can be ascertained by those skilled in the art using conventional course of treatmment tests.

The following examples are for illustrative purposes only.

### EXAMPLES

All reactions were performed in flame-dried or oven-dried glassware under a positive pressure of dry argon or dry nitrogen, and were stirred magnetically unless otherwise indicated. Sensitive liquids and solutions were transferred via syringe or cannula, and introduced into reaction vessels through rubber septa. Unless otherwise stated, the term 'concentration under reduced pressure' refers to use of a Buchi rotary evaporator at approximately 15 mmHg.

All temperatures are reported uncorrected in degrees Celsius (°C). Unless otherwise indicated, all parts and percentages are by weight.

Commercial grade reagents and solvents were used without further purification. Thin-layer chromatography (TLC) was performed on Whatman^{®} pre-coated glass-backed silica gel 60A F-254 250 µm plates. Visualization of plates was effected by one or more of the following techniques: (a) ultraviolet illumination, (b) exposure to iodine vapor, (c) immersion of the plate in a 10% solution of phosphomolybdic acid in ethanol followed by heating, (d) immersion of the plate in a cerium sulfate solution followed by heating, and/or (e) immersion of the plate in an acidic ethanol solution of 2,4-dinitrophenylhydrazine followed by heating. Column chromatography (flash chromatography) was performed using 230-400 mesh EM Science^{®} silica gel.

Melting points (mp) were determined using a Thomas-Hoover melting point apparatus or a Mettler FP66 automated melting point apparatus and are uncorrected. Fourier transform intrared spectra were obtained using a Mattson 4020 Galaxy Series spectrophotometer. Proton (¹H) nuclear magnetic resonance (NMR) spectra were measured with a General Electric GN-Omega 300 (300 MHz) spectrometer with either Me₄Si (δ 0.00) or residual protonated solvent (CHCl₃ δ 7.26; MeOH δ 3.30; DMSO δ 2.49) as standard Carbon (¹³C) NMR spectra were measured with a General Electric GN-Omega 300 (75 MHz) spectrometer with solvent (CDCl₃ δ 77.0; MeOD-d₃; δ 49.0; DMSO-d₆ δ 39.5) as standard. Low resolution mass spectra (MS) and high resolution mass spectra (HRMS) were either obtained as electron impact (EI) mass spectra or as fast atom bombardment (FAB) mass spectra. Electron impact mass spectra (EI-MS) were obtained with a Hewlett Packard 5989A mass spectrometer equipped with a Vacumetrics Desorption Chemical Ionization Probe for sample introduction. The ion source was maintained at 250 °C. Electron impact ionization was performed with electron energy of 70 eV and a trap current of 300 µA. Liquid-cesium secondary ion mass spectra (FAB-MS), an updated version of fast atom bombardment were obtained using a Kratos Concept 1-H spectrometer. Chemical ionization mass spectra (CI-MS) were obtained using a Hewlett Packard MS-Engine (5989A) with methane as the reagent gas (1x10⁻⁴ torr to 2.5x10⁻⁴ torr). The direct insertion desorption chemical ionization (DCI) probe (Vaccumetrics, Inc.) was ramped from 0-1.5 amps in 10 sec and held at 10 amps until all traces of the sample disappeared (∼1-2 min). Spectra were scanned from 50-800 amu at 2 sec per scan. HPLC - electrospray mass spectra (HPLC ES-MS) were obtained using a Hewlett-Packard 1100 HPLC equipped with a quaternary pump, a variable wavelength detector, a C-18 column, and a Finnigan LCQ ion trap mass spectrometer with electrospray ionization. Spectra were scanned from 120-800 amu using a variable ion time according to the number of ions in the source. Gas chromatography - ion selective mass spectra (GC-MS) were obtained with a Hewlett Packard 5890 gas chromatograph equipped with an HP-1 methyl silicone column (0.33 mM coating; 25 m x 0.2 mm) and a Hewlett Packard 5971 Mass Selective Detector (ionization energy 70 eV).

Elemental analyses were conducted by Robertson Microlit Labs, Madison NJ. All ureas displayed NMR spectra, LRMS and either elemental analysis or HRMS consistant with assigned structures.

### List of Abbreviations and Acronyms:

| | |
|---|---|
| AcOH | acetic acid |
| anh | anhydrous |
| BOC | *tert*-butoxycarbonyl |
| conc | concentrated |
| dec | decomposition |
| DMPU. | 1,3-dimethyl-3,4,5,6-tetrahydro-2(1H)-pyrimidinone |
| DMF | *N,N-*dimethylformamide |
| DMSO | dimethylsulfoxide |
| DPPA | diphenylphosphoryl azide |
| EtOAc | ethyl acetate |
| EtOH | ethanol (100%) |
| Et₂O | diethyl ether |
| Et₃N | triethylamine |
| *m*-CPBA | 3-chloroperoxybenzoic acid |
| MeOH | methanol |
| pet. ether | petroleum ether (boiling range 30-60 °C) |
| THF | tetrahydrofuran |
| TFA | trifluoroacetic acid |
| Tf | trifluoromethanesulfonyl |

### A. General Methods for Synthesis of Heterocyclic Amines

### A2. General Synthesis of 5-Amino-3-alkylisoxazoles (not part of claimed invention)

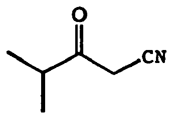

**Step 1. 3-Oxo-4-methylpentanenitrile:** A slurry of sodium hydride (60% in mineral oil; 10.3 g, 258 mmol) in benzene (52 mL) was warmed to 80 °C for 15 min., then a solution of acetonitrile (13.5 mL, 258 mmol) in benzene (52 mL) was added dropwise via addition funnel followed by a solution of ethyl isobutyrate (15 g, 129 mmol) in benzene (52 mL). The reaction mixture was heated overnight, then cooled with an ice water bath and quenched by addition of 2-propanol (50 mL) followed by water (50 mL) via addition funnel. The organic layer was separated and set aside. EtOAc (100 mL) was added to the aqueous layer and the resulting mixture was acidified to approximately pH 1 (conc. HCl) with stirring. The resulting aqueous layer was extracted with EtOAc (2 x 100 mL). The organic layers were combined with the original organic layer, dried (MgSO₄), and concentrated *in vacuo* to give the a-cyanoketone as a yellow oil which was used in the next step without further purification. 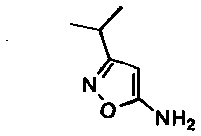 **Step 2. 5-Amino-3-isopropylisoxazole:** Hydroxylamine hydrochloride (10.3 g, 148 mmol) was slowly added to an ice cold solution of NaOH (25.9 g, 645 mmol) in water (73 mL) and the resulting solution was poured into a solution of crude 3-oxo-4-methylpentanenitrile while stirring. The resulting yellow solution was heated at 50°C for 2.5 hours to produce a less dense yellow oil. The warm reaction mixture was immediately extracted with CHCl₃ (3 x 100 mL) without cooling. The combined organic layers were dried (MgSO₄), and concentrated *in vacuo.* The resulting oily yellow solid was filtered through a pad of silica (10% acetone/90% CH₂Cl₂) to afford the desired isoxazole as a yellow solid (11.3 g, 70%): mp 63-65 °C; TLC R*_{f}* (5% acetone/95% CH₂Cl₂) 0.19; ¹H-NMR (DMSO-d₆) d 1.12 (d, *J*=7.0 Hz, 6H), 2.72 (sept, *J*=7.0 Hz, 1H), 4.80 (s, 2H), 6.44 (s, 1H); FAB-MS *m*/*z* (rel abundance) 127 ((M+H) ⁺; 67%).

### A3. General Method for the Preparation of 5-Amino-1-alkyl-3-alkylpyrazoles

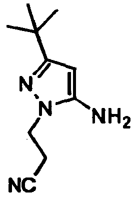

**5-Amino-3-tert-butyl-1-(2-cyanoethyl)pyrazole:** A solution of 4,4-dimethyl-3-oxopentanenitrile (5.6 g, 44.3 mmol) and 2-cyanoethyl hydrazine (4.61 g, 48.9 mmol) in EtOH (100 mL) was heated at the reflux temperature overnight after which TLC analysis showed incomplete reaction. The mixture was concentrated under reduced pressure and the residue was filtered through a pad of silica (gradient from 40% EtOAc/60% hexane to 70% EtOAc/30% hexane) and the resulting material was triturated (Et₂O/hexane) to afford the desired product (2.5 g, 30%): TLC (30% EtOAc/70% hexane) R*_{f}* 0.31; ¹H-NMR (DMSO-d₆) δ 1.13 (s, 9H), 2.82 (t, *J*=6.9 Hz, 2H), 4.04 (t, *J*=6.9 Hz, 2H), 5.12 (br s, 2H), 5.13 (s, 1H).

### A 4. Synthesis of 3-Amino-5-alkylthiophenes- (not part of claimed invention)

### A4a. Synthesis of 3-Amino-5-alkylthiophenes by Thermal Decarboxylation of Thiophenecarboxylic Acids

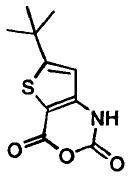

**Step 1. 7-*tert*-Butyl-2H-thieno[3,2-d]oxazine-2,4(1H)-dione:** A mixture of methyl 3-amino-5-*tert*-butylthiophenecarboxylate (7.5 g, 35.2 mmol) and KOH (5.92 g) in MeOH (24 mL) and water (24 mL) was stirred at 90 °C for 6 h. The reaction mixture was concentrated under reduced pressure and the residue was dissolved in water (600 mL). Phosgene (20% in toluene, 70 mL) was added dropwise over a 2 h period. The resulting mixture was stirred at room temperature overnight and the resulting precipitate was triturated (acetone) to afford the desired anhydride (5.78 g, 73%): ¹H-NMR (CDCl₃) δ 1.38 (s, 9H), 2.48 (s, 1H), 6.75 (s, 1H); FAB-MS *m*/*z* (rel abundance) 226 ((M+H)⁺, 100%). 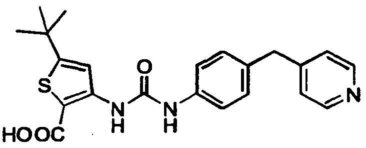 **Step 2. *N*-(5-*tert-*Butyl-2-carboxy-3-thienyl)-*N'*-(4-(4-pyridinylmethyl)phenyl)-urea:** A solution of 7-*tert*-butyl-2H-thieno[3,2-d]oxazine-2,4(1H)-dione (0.176 g, 0.78 mmol) and 4-(4-pyridinylinethyl)aniline (0.144 g, 0.78 mmol) in THF (5 mL) was heated at the reflux temp. for 25 h. After cooling to room temp., the resulting solid was triturated with Et₂O to afford the desired urea (0.25 g, 78%): mp 187-189 °C; TLC (50% EtOAc/50% pet. ether) R_{f} 0.04; ¹H-NMR (DMSO-d₆) δ 1.34 (s, 9H), 3.90 (s, 2H), 7.15 (d, *J*=7Hz, 2H), 7.20 (d, *J*=3 Hz, 2H), 7.40 (d, *J*=7 Hz, 2H), 7.80 (s 1H), 8.45 (d, *J*=3 Hz, 2H) 9.55 (s, 1H), 9.85 (s, 1H), 12.50 (br s, 1H); FAB-MS *m*/*z* (rel abundance) 410 ((M+H)⁺; 20%). 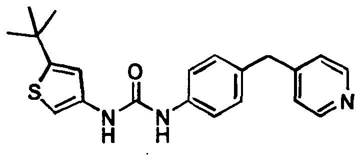 **Step 3. *N*-(5-*tert-*Butyl-3-thienyl)-*N'*-(4-(4-pyridlnylmethyl)phenyl)urea:** A vial containing N-(5-*tert*-butyl-2-carboxy-3-thienyl)-*N'*-(4-(4-pyridinylmethyl)phenyl)-urea (0.068 g, 0.15 mmol) was heated to 199 °C in an oil bath. After gas evolution ceased, the material was cooled and purified by preparative HPLC (C-18 column; gradient from 20% CH₃CN/79.9% H₂O/0.1% TFA to 99.9% H₂O/0.1% TFA) to give the desired product (0.024 g, 43%): TLC (50% EtOAc/50% pet. ether) R*_{f}* 0.18; ¹H-NMR (DMSO-d₆) δ 1.33 (s, 9H), 4.12 (s, 2H), 6.77 (s, 1H), 6.95 (s, 1H), 7.17 (d, *J*=9 Hz, 2H), 7.48 (d, *J*=9 Hz, 2H), 7.69 (d, *J*=7 Hz, 1H), 8.58 (s, 1H), 8.68 (d, *J=7* Hz, 2H), 8.75 (s, 1H); EI-MS *m*/*z* 365 (M⁺).

### A4b. Synthesis 3-Amino-5-alkylthiophenes from 3-Amino-5-alkyl-2-thiophenecarboxylate esters (not part of claimed invention)

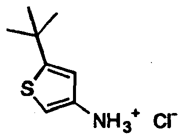

**5-*tert*-Butyl-3-thiopheneammonium Chloride:** To a solution of methyl 3-amino-5-*tert*-butyl-2-thiophene-carboxylate (5.07 g, 23.8 mmol, 1.0 equiv) in EtOH (150 mL) was added NaOH (2.0 g, 50 mmol, 2.1 equiv). The resulting solution was heated at the reflux temp. for 2.25 h. A conc. HCl solution (approximately 10 mL) was added dropwise with stirring and the evolution of gas was observed. Stirring was continued for 1 h, then the solution was concentrated under reduced pressure. The white residue was suspended in EtOAc (150 mL) and a saturated NaHCO₃ solution (150 mL) was added to dissolve. The organic layer was washed with water (150 mL) and a saturated NaCl solution (150 mL), dried (Na₂SO₄), and concentrated under reduced pressure to give the desired ammonium salt as a yellow oil (3.69 g, 100%). This material was used directly in urea formation without further purification.

### A4c. Synthesis 3-Amino-5-alkylthiophenes from N-BOC 3-Amino-5-alkyl-2-thiophenecarboxylate esters (not part of claimed invention)

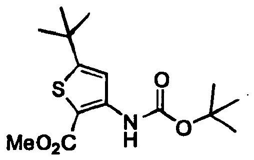

**Step 1. Methyl 3-(*tert*-Butoxycarbonylamino)-5-*tert*-butyl-2-thiophenecarboxy-late:** To a solution of methyl 3-amino-5-*tert*-butyl-2-thiophenecarboxylate (150 g, 0.70 mol) in pyridine (2.8 L) at 5 °C was added di-*tert*-butyl dicarbonate (171.08 g, 0.78 mol, 1.1 equiv) and *N,N*-dimethylaminopyridine (86 g, 0.70 mol, 1.00 equiv) and the resulting mixture was stirred at room temp for 7 d. The resulting dark solution was concentrated under reduced pressure (approximately 0.4 mmHg) at approximately 20°C. The resulting red solids were dissolved in CH₂Cl₂ (3 L) and sequentially washed with a 1 M H₃PO₄ solution (2 x 750 mL), a saturated NaHCO₃ solution (800 mL) and a saturated NaCl solution (2 x 800 mL), dried (Na₂SO₄) and concentrated under reduced pressure. The resulting orange solids were dissolved in abs. EtOH (2 L) by warming to 49 °C, then treated with water (500 mL) to afford the desired product as an off-white solid (163 g, 74%): ¹H-NMR. (CDCl₃) δ 1.38 (s, 9H), 1.51 (s, 9H), 3.84 (s, 3H), 7.68 (s, 1H), 9.35 (br s, 1H); FAB-MS *m*/*z* (rel abundance) 314 ((M+H)⁺, 45%). 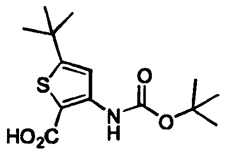 **Step 2. 3-(*tert*-Butoxycarbonylamino)-S-*tert*-butyl-2-thiophenecarboxylic Acid:** To a solution of methyl 3-(*tert*-butoxycarbonylamino)-5-*tert*-butyl-2-thiophenecarboxylate (90.0 g, 0.287 mol) in THF (630 mL) and MeOH (630 mL) was added a solution of NaOH (42.5 g, 1.06 mL) in water (630 mL). The resulting mixture was heated at 60 °C for 2 h, concentrated to approximately 700 mL under reduced pressure, and cooled to 0 °C. The pH was adjusted to approximately 7 with a 1.0 N HCl solution (approximately 1 L) while maintaining the internal temperature at approximately 0 °C. The resulting mixture was treated with EtOAc (4 L). The pH was adjusted to approximately 2 with a 1.0 N HCl solution (500 mL). The organic phase was washed with a saturated NaCl solution (4 x 1.5 L), dried (Na₂SO₄), and concentrated to approximately 200 mL under reduced pressure. The residue was treated with hexane (1 L) to form a light pink (41.6 g). Resubmission of the mother liquor to the concentration-precipitation protocol afforded additional product (38.4 g, 93% total yield): ¹H-NMR (CDCl₃) δ 1.94 (s, 9H), 1.54 (s, 9H), 7.73 (s, 1H), 9.19 (br s, 1H); FAB-MS *m*/*z* (rel abundance) 300 ((M+H)⁺, 50%). 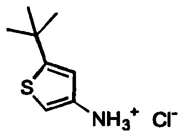 **Step 3. 5-tert-Butyl-3-thiopheneammonium Chloride:** A solution of 3-(*tert-*butoxycarbonylamino)-5-*tert*-butyl-2-thiophenecarboxylic acid (3.0 g, 0.010 mol) in dioxane (20 mL) was treated with an HCl solution (4.0 M in dioxane, 12.5 mL, 0.050 mol, 5.0 equiv), and the resulting mixture was heated at 80 °C for 2 h. The resulting cloudy solution was allowed to cool to room temp forming some precipitate. The slurry was diluted with EtOAc (50 mL) and cooled to -20 °C. The resulting solids were collected and dried overnight under reduced pressure to give the desired salt as an off-white solid (1.72 g, 90%): ¹H-NMR (DMSO-d₆) δ 1.31 (s, 9H), 6.84 (d, *J*=1.48 Hz, 1H), 7.31 (d, *J*=1.47 Hz, 1H), 10.27 (br s, 3H).

### A5. General Method for the Synthesis of BOC-Protected Pyrazoles

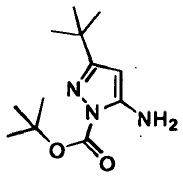

**5-Amino-3-*tert*-butyl-*N'*-(*tert*-butoxycarbonyt)pyrazote:** To a solution of 5-amino-3-*tert*-butylpyrazole (3.93 g, 28.2 mmol) in CH₂Cl₂ (140 mL) was added di-*tert*-butyl dicarbonate (6.22 g, 28.5 mmol) in one portion. The resulting solution was stirred at room temp. for 13 h, then diluted with EtOAc (500 mL). The organic layer was washed with water (2 x 300 mL), dried (MgSO₄) and concentrated under reduced pressure. The solid residue was triturated (100 mL) hexane) to give the desired carbamate (6.26 g, 92%): mp 63-64 °C; TLC R_{f} (5% acetone/95% CH₂Cl₂); ¹H-NMR (DMSO-d₆) δ 1.15 (s, 9H), 1.54 (s, 9H), 5.22 (s, 1H), 6.11 (s, 2H); FAB-MS *m*/*z* ((M +H)⁺).

### A6. General Method for the Synthesis of 2-Aminothiadiazoles (not part of claimed invention)

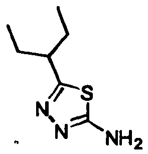

**2-Amino-5-(1-(1-ethyl)propyl)thiadiazine:** To concentrated sulfuric acid (9.1 mL) was slowly added 2-ethylbutyric acid (10.0 g, 86 mmol, 1.2 equiv). To this mixture was slowly added thiosemicarbazide (6.56 g, 72 mmol, 1 equiv). The reaction mixture was heated at 85 °C for 7 h, then cooled to room temperature, and treated with a concentrated NH₄OHsolution until basic. The resulting solids were filtered to afford 2-amino-5-(1-(1-ethyl)propyl)thiadiazine product was isolated via vacuum filtration as a beige solid (6.3 g, 51%): mp 155-158 °C; TLC (5% MeOH/ 95% CHCl₃) R*_{f}* 0.14; ¹H-NMR (DMSO-d₆) δ 0.80 (t, J=7.35 Hz, 6H), 1.42-1.60 (m, 2H), 1.59-1.71 (m, 2H), 2.65-2.74 (m, 1H), 7.00 (br s, 2H); HPLC ES-MS *m*/*z* 172 ((M+H) ⁺).

### A7. GeneralMethod for the Synthesis of 2-Aminooxadiazoles (not part of claimed invention)

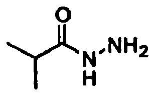

**Step 1. Isobutyric Hydrazide:** A solution of methyl isobutyrate (10.0 g) and hydrazine (2.76 g) in MeOH (500 mL) was heated at the reflux temperature over night then stirred at 60 °C for 2 weeks. The resulting mixture was cooled to room temperature and concentrated under reduced pressure to afford isobutyric hydrazide as a yellow oil (1.0 g, 10%), which was used inb the next step withour further purification. 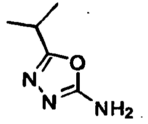 **Step 2. 2-Amino-5-isopropyl oxadiazole:** To a mixture of isobutyric hydrazide (0.093 g), KHCO₃ (0.102 g), and water (1 mL) in dioxane (1 mL) at room temperature was added cyanogen bromide (0.10 g). The resulting mixture was heated at the refulx temperature for 5 h, and stirred at room temperature for 2 d, then treated with CH₂Cl₂ (5 mL). The organic layer was washed with water (2 x 10 mL), dried (MgSO₄) and concentrated under reduced pressure to afford 2-amino-5-isopropyl oxadiazole as a white solid: HPLC ES-MS *m*/*z* 128 ((M+H)⁺).

### A8. General Method for the Synthesis of 2-Aminoozazoles (not part of claimed invention)

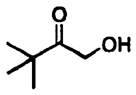

**Step 1. 3,3-Dimethyl-1-hydrogy-2-butanone:** A neat sample of 1-bromo-3,3-dimethyl-2-butanone (33.3 g) at 0 °C was treated with a 1N NaOH solution, then was stirred for 1 h. The resulting mixture was extracted with EtOAc (5 x 100 mL). The combined organics were dried (Na₂SO₄) and concentrated under reduced pressure to give 3,3-dimethyl-1-hydroxy-2-butanone (19 g, 100%), which was used inb the next step withour further purification. 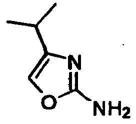 **Step 2. 2-Amino-4-isopropyl-1,3-oxazole:** To a solution of 3,3-dimethyl-1-hydroxy-2-butanone (4.0 g) and cyanimide (50% w/w, 2.86 g) in THF (10 mL) was added a 1N NaOAc solution (8 mL), followed by tetra-n-butylammonium hydroxide (0.4 M, 3.6 mL), then a 1N NaOH solution (1.45 mL). The resulting mixtuire was stirred at room temperature for 2 d. The resulting organic layer was separated, washed with water (3 x 25 mL), and the aqueous layer was extraced with Et₂O (3 x 25 mL). The combined organic, layers were treated with a 1N NaOH solution tuntil basic, then extracted with CH₂Cl₂ (3 x 25 mL). The combined organic layers were dried (Na₂SO₄) and concentrated under reduced pressure to afford 2-Amino-4-isopropyl-1,3-oxazole (1.94 g, 41%): HPLC ES-MS *m*/*z* 141 ((M+M⁺).

### A9. Method for the Synthesis of Substituted-5-aminotetrazoles (not part of claimed invention)

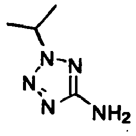

To a solution of 5-aminotetrazole (5 g), NaOH (2.04 g) and water (25 mL) in EtOH (115 mL) at the reflux temperature was added 2-bromopropane (5.9g). The resulting mixture was heated at the reflux temperature for 6 d, then cooled to room temperature, and concentrated under reduced pressure. The resulting aqueous mixture was washed with CH₂Cl₂ (3 x 25 mL), then concentrated under reduced pressure with the aid of a lyophlizer to afford a mixture of 1- and 2-isopropyl-5-aminotetrazole (50%), which was used without further purification: HPLC ES-MS *m*/*z* 128 ((M+H)⁺).

### B. General Methods for Synthesis of Substituted Anilines

### B1. General Method for Substituted Aniline Formation via Hydrogenation of a Nitroarene

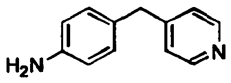

**4-(4-Pyridinylmethyl)aniline:** To a solution of 4-(4-nitrobenzyl)pyridine (7.0 g, 32.68 mmol) in EtOH (200 mL) was added 10% Pd/C (0.7 g) and the resulting slurry was shaken under a H₂ atmosphere (50 psi) using a Parr shaker. After 1 h, TLC and ¹H-NMR of an aliquot indicated complete reaction. The mixture was filtered through a short pad of Celite^{®}. The filtrate was concentrated *in vacuo* to afford a white solid (5.4 g, 90%): ¹H-NMR (DMSO-d₆) δ 3.74 (s, 2H), 4.91 (br s, 2H), 6.48 (d, *J*=8.46 Hz, 2H), 6.86 (d, *J*=8.09 Hz, 2H), 7.16 (d, *J*=5.88 Hz, 2H), 8.40 (d, *J*=5.88 Hz, 2H); EI-MS *m*/*z* 184 (M⁺), This material was used in urea formation reactions without further purification.

### B2. General Method for Substituted Aniline Formation via Dissolving Metal Reduction of a Nitroarene

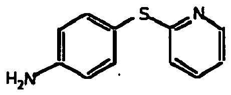

4-(2-Pyridinylthio)aniline: To a solution of 4-(2-pyridinylthio)-1-nitrobenzene (Menai ST 3355A; 0.220 g, 0.95 mmol) and H2O (0.5 mL) in AcOH ( 5 mL) was added iron powder (0.317 g, 5.68 mmol) and the resulting slurry stirred for 16 h at room temp. The reaction mixture was diluted with EtOAc (75 mL) and H₂O (50 mL), basified to pH 10 by adding solid K₂CO₃ in portions (*Caution:* foaming). The organic layer was washed with a saturated NaCl solution, dried (MgSO₄), concentrated *in vacuo.* The residual solid was purified by MPLC (30% EtOAc/70% hexane) to give the desired product as a thick oil (0.135 g, 70%): TLC (30% EtOAc/70% hexanes) R_{f} 0.20.

### B3a. General Method for Substituted Aniline Formation via Nitroarene Formation Through Nucleophilic Aromatic Substitution, Followed by Reduction

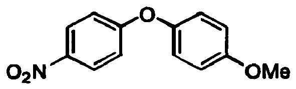

**Step 1. 1-Methoxy-4-(4-nitrophenoxy)benzene:** To a suspension of NaH (95%, 1.50 g, 59 mmol) in DMF (100 mL) at room temp. was added dropwise a solution of 4-methoxyphenol (7.39 g, 59 mmol) in DMF (50 mL). The reaction was stirred 1 h, then a solution of 1-fluoro-4-nitrobenzene (7.0 g, 49 mmol) in DMF (50 mL) was added dropwise to form a dark green solution. The reaction was heated at 95°C overnight, then cooled to room temp., quenched with H₂O, and concentrated *in vacuo.* The residue was partitioned between EtOAc (200 mL) and H₂O (200 mL) . The organic layer was sequentially washed with H₂O (2 x 200 mL), a saturated NaHCO₃ solution (200 mL), and a saturated NaCl solution (200 mL), dried (Na₂SO₄), and concentrated *in vacuo.* The residue was triturated (Et₂O/hexane) to afford 1-methoxy-4-(4-nitrophenoxy)benzene (12.2 g, 100%): ¹H-NMR (CDCl₃) δ 3.83 (s, 3H), 6.93-7.04 (m, 6H), 8.18 (d, *J*=9.2 Hz, 2H); EI-MS *m*/*z* 245 (M⁺). 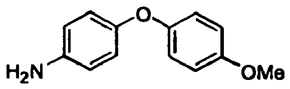 Step 2. 4-(4-Methozyphenoxy)aniline: To a solution of 1-methoxy-4-(4-nitrophenoxy)benzene (12.0 g, 49 mmol) in EtOAc (250 mL) was added 5% Pt/C (1.5 g) and the resulting slurry was shaken under a H₂ atmosphere (50 psi) for 18 h. The reaction mixture was filtered through a pad of **Celite^{®}** with the aid of EtOAc and concentrated *in vacuo* to give an oil which slowly solidified (10.6 g, 100%): ¹H-NMR (CDCl₃) δ 3.54 (br s, 2H), 3.78 (s, 3H), 6.65 (d, *J*=8.8 Hz, 2H), 6.79-6.92 (m, 6H); EI-MS m/z 215 (M⁺).

### B3b. General Method for Substituted Aniline Formation via Nitroarene Formation Through Nucleophilic Aromatic Substitution, Followed by Reduction

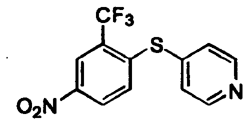

Step 1. 3-(Trifluoromethyl)-4-(4-pyridinylthio)nitrobenzene: A solution of 4-mercaptopyridine (2.8 g, 24 mmoles), 2-fluoro-5-nitrobenzotrifluoride (5 g, 23.5 mmoles), and potassium carbonate (6.1 g, 44.3 mmoles) in anhydrous DMF (80 mL) was stirred at room temperature and under argon overnight. TLC showed complete reaction. The mixture was diluted with Et₂O (100 mL) and water (100 mL) and the aqueous layer was back-extracted with Et₂O (2 x 100 mL). The organic layers were washed with a saturated NaCl solution (100 mL), dried (MgSO₄), and concentrated under reduced pressure. The solid residue was triturated with Et₂O to afford the desired product as a tan solid (3.8 g, 54%): TLC (30% EtOAc/70% hexane) R*_{f}* 0.06; ¹H-NMR (DMSO-d₆) δ 7.33 (dd, *J*=1.2,4.2 Hz, 2H), 7.78 (d, *J*=8.7 Hz, 1H), 8.46 (dd, *J*=2.4, 8.7Hz, 1H), 8.54-8.56 (m, 3H). 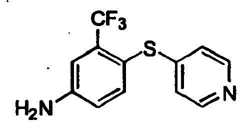 **Step 2. 3-(Trifluoromethyl)-4-(4-pyridinylthio)aniline:** A slurry of 3-trifluoromethyl-4-(4-pyridinylthio)nitrobenzene (3.8 g, 12.7 mmol), iron powder (4.0 g, 71.6 mmol), acetic acid (100 mL), and water (1 mL) were stirred at room temp. for 4 h. The mixture was diluted with Et₂O (100 mL) and water (100 mL). The aqueous phase was adjusted to pH 4 with a 4 N NaOH solution. The combined organic layers were washed with a saturated NaCl solution (100 mL), dried (MgSO₄), and concentrated under reduced pressure. The residue was filtered through a pad of silica (gradient from 50% EtOAc/50% hexane to 60% EtOAc/40% hexane) to afford the desired product (3.3 g): TLC (50% EtOAc/50% hexane) R_{f} 0.10; ¹H-NMR (DMSO-d₆) δ 6.21 (s, 2H), 6.84-6.87 (m, 3H), 7.10 (d, *J*=2.4 Hz, 1H), 7.39 (d, *J*=8.4 Hz, 1H), 8.29 (d, *J*=6.3 Hz, 2H).

### B3c. General Method for Substituted Aniline Formation via Nitroarene Formation Through Nucleophilic Aromatic Substitution, Followed by Reduction

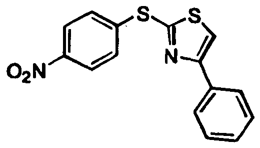

**Step 1. 4-(2-(4-Phenyl)thiazolyl)thio-1-nitrobenzene:** A solution of 2-mercapto-4-phenylthiazole (4.0 g, 20.7 mmoles) in DMF (40 mL) was treated with 1-fluoro-4-nitrobenzene (2.3 mL, 21.7 mmoles) followed by K₂CO₃ (3.18 g, 23 mmol), and the mixture was heated at approximately 65 °C overnight. The reaction mixture was then diluted with EtOAc (100 mL), sequentially washed with water (100 mL) and a saturated NaCl solution (100 mL), dried (MgSO₄ and concentrated under reduced pressure. The solid residue was triturated with a Et₂O/hexane solution to afford the desired product (6.1 g): TLC (25% EtOAc/75% hexane) R*_{f}* 0.49; ¹H-NMR (CDCl₃) δ 7.35-7.47 (m, 3H), 7.58-7.63 (m, 3H), 7.90 (d, *J*=6.9 Hz, 2H), 8.19 (d, *J*=9.0 Hz, 2H). 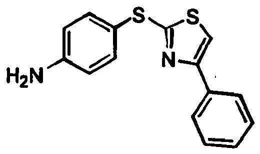 **Step 2. 4-(2-(4-Phenyl)thiazolyl)thioaniline:** 4-(2-(4-Phenyl)thiazolyl)thio-1-nitrobenzene was reduced in a manner analagous to that used in the preparation of 3-(trifluoromethyl)-4-(4-pyridinylthio)aniline:TLC (25% EtOAc/75% hexane) R_{f} 0.18; ¹H-NMR (CDCl₃) δ 3.89 (br s, 2H), 6.72-6.77 (m, 2H), 7.26-7.53 (m, 6H), 7.85-7.89 (m, 2H).

### B3d. General Method for Substituted Aniline Formation via Nitroarene Formation Through Nucleophilic Aromatic Substitution, Followed by Reduction

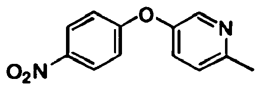

**Step 1. 4-(6-Methyl-3-pyridinyloxy)-1-nitrobenzene:** To a solution of 5-hydroxy-2-methylpyridine (5.0 g, 45.8 mmol) and 1-fluoro-4-nitrobenzene (6.5 g, 45.8 mmol) in anh DMF (50 mL) was added K₂CO₃ (13.0 g, 91.6 mmol) in one portion. The mixture was heated at the reflux temp. with stirring for 18 h and then allowed to cool to room temp. The resulting mixture was poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo* to afford the desired product (8.7 g, 83%). The this material was carried to the next step without further purification. 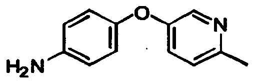 **Step 2. 4-(6-Methyl-3-pyridinyloxy)aniline:** A solution of 4-(6-methyl-3-pyridinyloxy)-1-nitrobenzene (4.0 g, 17.3 mmol) in EtOAc (150 mL) was added to 10% Pd/C (0.500 g, 0.47 mmol) and the resulting mixture was placed under a H₂ atmosphere (balloon) and was allowed to stir for 18 h at room temp. The mixture was then filtered through a pad of Celite^{®} and concentrated *in vacuo* to afford the desired product as a tan solid (3.2 g, 92%): EI-MS *m*/*z* 200 (M⁺).

### B3e. General Method for Substituted Aniline Formation via Nitroarene Formation Through Nucleophilic Aromatic Substitution, Followed by Reduction

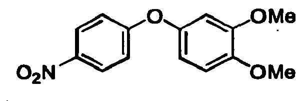

**Step 1. 4-(3,4-Dimetmoxyphenoxy)-1-nitrobenzene:** To a solution of 3,4-dimethoxyphenol (1.0 g, 6.4 mmol) and 1-fluoro-4-nitrobenzene (700 µL, 6.4 mmol) in anh DMF (20 mL) was added K₂CO₃ (1.8 g, 12.9 mmol) in one portion. The mixture was heated at the reflux temp with stirring for 18 h and then allowed to cool to room temp. The mixture was then poured into water (100 mL) and extracted with EtOAc (3 x 100 mL). The combined organics were sequentially washed with water (3 x 50 mL) and a saturated NaCl solution (2 x 50 mL), dried (Na₂SO₄), and concentrated *in vacuo* to afford the desired product (0.8 g, 54%). The crude product was carried to the next step without further purification. 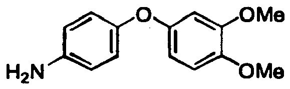 **Step 2. 4-(3,4-Dimethoxyphenoxy)aniline:** A solution of 4-(3,4-dimethoxy-phenoxy)-1-nitrobenzene (0.8 g, 3.2 mmol) in EtOAc (50 mL) was added to 10% Pd/ C (0.100 g) and the resulting mixture was placed under a H₂ atmosphere (balloon) and was allowed to stir for 18 h at room temp. The mixture was then filtered through a pad of Celite^{®} and concentrated *in vacuo* to afford the desired product as a white solid (0.6 g, 75%): EI-MS *m*/*z* 245 (M⁺).

### B3f. General Method for Substituted Aniline Formation via Nitroarene Formation Through Nucleophilic Aromatic Substitution, Followed by Reduction

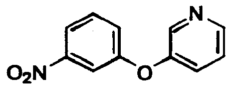

**Step 1. 3-(3-Pyridinylogy)-1-nitrobenzene:** To a solution of 3-hydroxypyridine (2.8 g, 29.0 mmol), 1-bromo-3-nitrobenzene (5.9 g, 29.0 mmol) and copper(I) bromide (5.0 g, 34.8 mmol) in anh DMF (50 mL) was added K₂CO₃ (8.0 g, 58.1 mmol) in one portion. The resulting mixture was heated at the reflux temp. with stirring for 18 h and then allowed to cool to room temp. The mixture was then poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo.* The resulting oil was purified by flash chromatography (30% EtOAc/70% hexane) to afford the desired product (2.0 g, 32 %). This material was used in the next step without further purification. 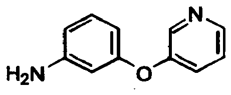 **Step 2. 3-(3-Pyridinyloxy)aniline:** A solution of 3-(3-pyridinyloxy)-1-nitrobenzene (2.0 g, 9.2 mmol) in EtOAc (100 mL) was added to 10% Pd/C (0.200 g) and the resulting mixture was placed under a H₂ atmosphere (balloon) and was allowed to stir for 18 h at room temp. The mixture was then filtered through a pad of Celite^{®} and concentrated *in vacuo* to afford the desired product as a red oil (1.6 g, 94%): EI-MS *m*/*z* 186 (M⁺).

### B3g. General Method for Substituted Aniline Formation via Nitroarene Formation Through Nucleophilic Aromatic Substitution, Followed by Reduction

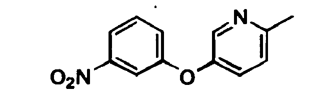

**Step 1. 3-(5-Methyl-3-pyridinyloxy)-1-nitrobenzene:** To a solution of 3-hydroxy-5-methylpyridine (5.0 g, 45.8 mmol), 1-bromo-3-nitrobenzene (12.0 g, 59.6 mmol) and copper(I) iodide (10.0 g, 73.3 mmol) in anh DMF (50 mL) was added K₂CO₃ (13.0 g, 91.6 mmol) in one portion. The mixture was heated at the reflux temp. with stirring for 18 h and then allowed to cool to room temp. The mixture was then poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo.* The resulting oil was purified by flash chromatography (30% EtOAc/70% hexane) to afford the desired product (1.2 g, 13%). 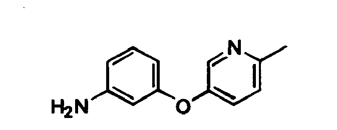 **Step 2. 3-(5-Methyl-3-pyridinyloxy)-1-nitrobenzene:** A solution of 3-(5-methyl-3-pyridinyloxy)-1-nitrobenzene (1.2 g, 5.2 mmol) in EtOAc (50 mL) was added to 10% Pd/C (0.100 g) and the resulting mixture was placed under a H₂ atmosphere (balloon) and was allowed to stir for 18 h at room temp. The mixture was then filtered through a pad of Celite^{®} and concentrated *in vacuo* to afford the desired product as a red oil (0.9 g, 86%): CI-MS *m*/*z* 201 ((M+H)⁺).

### B3h. General Method for Substituted Aniline Formation via Nitroarene Formation Through Nucleophilic Aromatic Substitution, Followed by Reduction

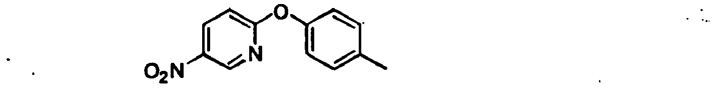

**Step 1. 5-Nitro-2-(4-methylphenoxy)pyridine:** To a solution of 2-chloro-5-nitropyridine (6.34 g, 40 mmol) in DMF (200 mL) were added of 4-methylphenol (5.4 g, 50 mmol, 1.25 equiv) and K₂CO₃ (8.28 g, 60 mmol, 1.5 equiv). The mixture was stirred overnight at room temp. The resulting mixture was treated with water (600 mL) to generate a precipitate. This mixture was stirred for 1 h, and the solids were separated and sequentially washed with a 1 N NaOH solution (25 mL), water (25 mL) and pet ether (25 mL) to give the desired product (7.05 g, 76%): mp 80-82 °C; TLC (30% EtOAc/70% pet ether) R*_{f}* 0.79; ¹H-NMR (DMSO-d₆) δ 2.31 (s, 3H), 7.08 (d, *J*=8.46 Hz, 2H), 7.19 (d, *J*=9.20 Hz, 1H), 7.24 (d, *J*=8.09 Hz, 2H), 8.58 (dd, *J*=2.94, 8.82 Hz, 1H), 8.99 (d, *J*=2.95 Hz, 1H); FAB-MS *m*/*z* (rel abundance) 231 ((M+H)⁺), 100%). 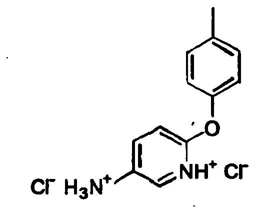 **Step 2. 5-Amino-2-(4-methylphenoxy)pyridine Dihydrochloride:** A solution 5-nitro-2-(4-methylphenoxy)pyridine (6.94 g, 30 mmol, 1 eq) and EtOH (10 mL) in EtOAc (190 mL) was purged with argon then treated with 10% Pd/C (0.60 g). The reaction mixture was then placed under a H₂ atmosphere and was vigorously stirred for 2.5 h. The reaction mixture was filtered through a pad of Celite^{®}. A solution of HCl in Et₂O was added to the filtrate was added dropwise. The resulting precipitate was separated and washed with EtOAc to give the desired product (7.56 g, 92%): mp 208-210 °C (dec); TLC (50% EtOAc/50% pet ether) R_{f} 0.42; ¹H-NMR (DMSO-d₆) δ 2.25 (s, 3H), 6.98 (d, *J*=8.45 Hz, 2H), 7.04 (d, *J*=8.82 Hz, 1H), 7.19 (d, J=8.09 Hz, 2H), 8.46 (dd, *J*=2.57, 8.46 Hz, 1H), 8.63 (d, *J*=2.57 Hz, 1H); EI-MS *m*/*z* (rel abundance) (M⁺, 100%).

### B3i. General Method for Substituted Aniline Formation via Nitroarene Formation Through Nucleophilic Aromatic Substitution, Followed by Reduction

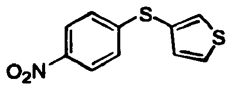

**Step 1. 4-(3-Thienylthiol-1-nitrobenzene:** To a solution of 4-nitrothiophenol (80%pure; 1.2 g, 6.1 mmol), 3-bromothiophene (1.0 g, 6.1 mmol) and copper(II) oxide (0.5 g, 3.7 mmol) in anhydrous DMF (20 mL) was added KOH (0.3 g, 6.1 mmol), and the resulting mixture was heated at 130 °C with stirring for 42 h and then allowed to cool to room temp. The reaction mixture was then poured into a mixture of ice and a 6N HCl solution (200 mL) and the resulting aqueous mixture was extracted with EtOAc (3 x 100 mL). The combined organic layers were sequentially washed with a 1M NaOH solution (2 x 100 mL) and a saturated NaCl solution (2 x 100 mL), dried (MgSO₄), and concentrated *in vacuo.* The residual oil was purified by MPLC (silica gel; gradient from 10% EtOAc/90% hexane to 5% EtOAc/95% hexane) to afford of the desired product (0.5 g, 34%). GC-MS *m*/*z* 237 (M⁺). 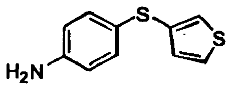 **Step 2. 4-(3-Thienylthio)aniline:** 4-(3-Thienylthio)-1-nitrobenzene was reduced to the aniline in a manner analogous to that described in Method B1.

### B3j. General Method for Substituted Aniline Formation via Nitroarene Formation Through Nucleophilic Aromatic Substitution, Followed by Reduction

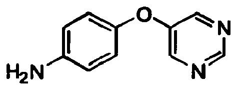

**4-(5-Pyrimininyloxy)aniline:** 4-Aminophenol (1.0 g, 9.2 mmol) was dissolved in DMF (20 mL) then 5-bromopyrimidine (1.46 g, 9.2 mmol) and K₂CO₃ (1.9 g, 13.7 mmol) were added. The mixture was heated to 100°C for 18 h and at 130°C for 48 h at which GC-MS analysis indicated some remaining starting material. The reaction mixture was cooled to room temp. and diluted with water (50 mL). The resulting solution was extracted with EtOAc (100 mL). The organic layer was washed with a saturated NaCl solution (2 x 50 mL), dried (MgSO₄), and concentrated *in vacuo.* The residular solids were purified by MPLC (50% EtOAc/50% hexanes) to give the desired amine (0.650 g, 38%).

### B3k. General Method for Substituted Aniline Formation via Nitroarene Formation Through Nucleophilic Aromatic Substitution, Followed by Reduction

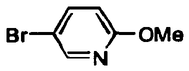

**Step 1. 5-Bromo-2-methoxypyridine:** A mixture of 2,5-dibromopyridine (5.5 g, 23.2 mmol) and NaOMe (3.76g, 69.6 mmol) in MeOH (60 mL) was heated at 70°C in a sealed reaction vessel for 42 h, then allowed to cool to room temp. The reaction mixture was treated with water (50 mL) and extracted with EtOAc (2 x 100 mL). The combined organic layers were dried (Na₂SO₄) and concentrated under reduced pressure to give a pale yellow, volatile oil (4.1g, 95% yield): TLC (10% EtOAc / 90% hexane) R*_{f}* 0.57. 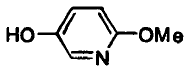 **Step 2. 5-Hydroxy-2-methoxypyridine:** To a stirred solution of 5-bromo-2-methoxypyridine (8.9 g, 47.9 mmol) in THF (175 mL) at -78 °C was added an n-butyllithium solution (2.5 M in hexane; 28.7 mL, 71.8 mmol, dropwise and the resulting mixture was allowed to stir at -78 °C for 45 min. Trimethyl borate (7.06 mL, 62.2 mmol) was added via syringe and the resulting mixture was stirred for an additional 2 h. The bright orange reaction mixture was warmed to 0 °C and was treated with a mixture of a 3 N NaOH solution (25 mL, 71.77 mmol) and a hydrogen peroxide solution (30%; approx. 50 mL). The resulting yellow and slightly turbid reaction mixture was warmed to room temp. for 30 min and then heated to the reflux temp. for 1 h. The reaction mixture was then allowed to cool to room temp. The aqueous layer was neutralized with a 1N HCl solution then extracted with Et₂O (2 x 100 mL). The combined organic layers were dried (Na₂SO₄) and concentrated under reduced pressure to give a viscous yellow oil (3.5g, 60%). 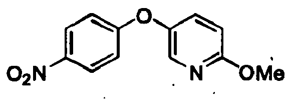 **Step 3. 4-(5-(2-Methoxy)pyridyl)oxy-1-nitrobenzene:** To a stirred slurry of NaH (97%, 1.0 g, 42 mmol) in anh DMF (100 mL) was added a solution of 5-hydroxy-2-methoxypyridine (3.5g, 28 mmol) in DMF (100 mL). The resulting mixture was allowed to stir at room temp. for 1 h, 4-fluoronitrobenzene (3 mL, 28 mmol) was added via syringe. The reaction mnixture was heated to 95 °C overnight, then treated with water (25 mL) and extracted with EtOAc (2 x 75 mL). The organic layer was dried (MgSO₄) and concentrated under reduced pressure. The residual brown oil was crystalized EtOAc/hexane) to afford yellow crystals (5.23 g, 75%). 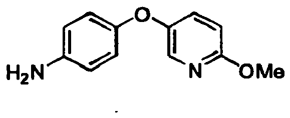 **Step 4. 4-(5-(2-Methoxy)pyridyl)oxyaniline:** 4-(5-(2-Methoxy)pyridyl)oxy-1-nitrobenzene was reduced to the aniline in a manner analogous to that described in Method B3d, Step2.

### B4a. General Method for Substituted Aniline Synthesis via Nucleophilic Aromatic Substitution using a Halopyridine

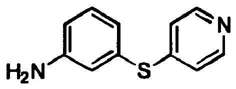

**3-(4-Pyridinylthio)aniline:** To a solution of 3-aminothiophenol (3.8 mL, 34 mmoles) in anh DMF (90mL) was added 4-chloropyridine hydrochloride (5.4 g, 35.6 mmoles) followed by K₂CO₃ (16.7 g, 121 mmoles). The reaction mixture was stirred at room temp. for 1.5 h, then diluted with EtOAc (100 mL) and water (100mL). The aqueous layer was back-extracted with EtOAc (2 x 100 mL). The combined organic layers were washed with a saturated NaCl solution (100 mL), dried (MgSO₄), and concentrated under reduced pressure. The residue was filtered through a pad of silica (gradient from 50% EtOAc/50% hexane to 70% EtOAc/30% hexane) and the resulting material was triturated with a Et₂O/hexane solution to afford the desired product (4.6 g, 66%): TLC (100 % ethyl acetate) R_{f} 0.29; ¹H-NMR (DMSO-d₆) δ 5.41 (s, 2H), 6.64-6.74 (m, 3H), 7.01 (d, J=4.8, 2H), 7.14 (t, J=7.8 Hz, 1H), 8.32 (d, J=4.8, 2H).

### B4b. General Method for Substituted Aniline Synthesis via Nucleophilic Aromatic Substitution using a Halopyridine

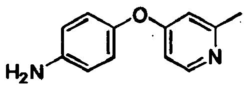

**4-(2-Methyl-4-pyridinylogy)aniline:** To a solution of 4-aminophenol (3.6 g, 32.8 mmol) and 4-chloropicoline (5.0 g, 39.3 mmol) in anh DMPU (50 mL) was added potassium tert-butoxide (7.4 g, 65.6 mmol) in one portion. The reaction mixture was heated at 100°C with stirring for 18 h, then was allowed to cool to room temp. The resulting mixture was poured into water (200 mL) and extracted with EtOAc (3 x 150 mL). The combined extracts were sequentially washed with water (3 x 100 mL) and a saturated NaCl solution (2 x 100 mL), dried (Na₂SO₄), and concentrated in *vacuo.* The resulting oil was purified by flash chromatography (50 % EtOAc/50% hexane) to afford the desired product as a yellow oil (0.7 g, 9%): CI-MS *m*/*z* 201 ((M+FD⁺).

### B4c. General Method for Substituted Aniline Synthesis via Nucleophilic Aromatic Substitution using a Halopyridine (not part of claimed invention)

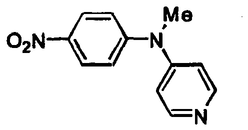

**Step 1. Methyl(4-nitrophenyl)-4-pyridylamine:** To a suspension of *N*-methyl-4-nitroaniline (2.0 g, 13.2 mmol) and K₂CO₃ (7.2 g, 52.2 mmol) in DMPU (30mL) was added 4-chloropyridine hydrochloride (2.36 g, 15.77 mmol). The reaction mixture was heated at 90 °C for 20 h, then cooled to room temperature. The resulting mixture was diluted with water (100 mL) and extracted with EtOAc (100 mL). The organic layer was washed with water (100 mL), dried (Na₂SO₄) and concentrated under reduced pressure. The residue was purified by column chromatography (silica gel, gradient from 80% EtOAc /20% hexanes to 100% EtOAc) to afford methyl(4-nitrophenyl)-4-pyridylamine (0.42 g) 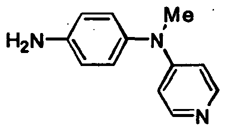 **Step 2. Methyl(4-aminophenyl)-4-pyridylamine:** Methyl(4-nitrophenyl)-4-pyridylamine was reduced in a manner analogous to that described in Method B1.

### B5. General Method of Substituted Aniline Synthesis via Phenol Alkylation Followed by Reduction of a Nitroarene

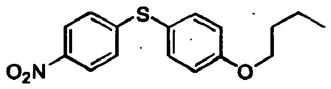

**Step 1. 4-(4-Butoxyphenyl)thio-1-nitrobenzene:** To a solution of 4-(4-nitrophenyl-thio)phenol (1.50 g, 6.07 mmol) in anh DMF (75 ml) at 0 °C was added NaH (60% in mineral oil, 0.267 g, 6.67 mmol). The brown suspension was stirred at 0 °C until gas evolution stopped (15 min), then a solution of iodobutane (1.12 g, .690 ml, 6.07 mmol) in anh DMF (20 mL) was added dropwise over 15 min at 0 °C. The reaction was stirred at room temp. for 18 h at which time TLC indicated the presence of unreacted phenol, and additional iodobutane (56 mg, 0.035 mL, 0.303 mmol, 0.05 equiv) and NaH (13 mg, 0.334 mmol) were added. The reaction was stirred an additional 6 h room temp., then was quenched by the addition of water (400 mL). The resulting mixture was extracted with Et₂O (2 x 500 mL). The combibed organics were washed with water (2 x 400 mL), dried (MgSO₄), and concentrated under reduced pressure to give a clear yellow oil, which was purified by silica gel chromatography (gradient from 20% EtOAc/80% hexane to 50% EtOAc/50% hexane) to give the product as a yellow solid (1.24 g, 67%): TLC (20% EtOAc/80% hexane) R_{f} 0.75; ¹H-NMR (DMSO-d₆) δ 0.92 (t, *J*= 7.5 Hz, 3H), 1.42 (app hex, *J*=7.5 Hz, 2H), 1.70 (m, 2H), 4.01 (t, *J*= 6.6 Hz, 2H), 7.08 (d, *J*=8.7 Hz, 2H), 7.17 (d, *J*=9 Hz, 2H), 7.51 (d, *J*= 8.7 Hz, 2H), 8.09 (d, *J*= 9 Hz, 2H). 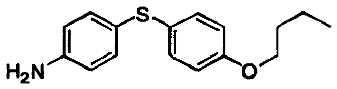 **Step 2. 4-(4-Butoxyphenyl)thioaniline:** 4-(4-Butoxyphenyl)thio-1-nitrobenzene was reduced to the aniline in a manner analagous to that used in the preparation of 3-(trifluoromethyl)-4-(4-pyridinylthio)aniline (Method B3b, Step 2): TLC (33% EtOAc/ 77% hexane) R*_{f}* 0.38.

### B6. General Method for Synthesis of Substituted Anilines by the Acylation of Diaminoarenes

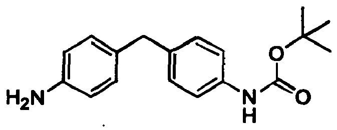

**4-(4-*tert-*Butogycarbamoylbenzyl)aniline:** To a solution of 4,4'-methylenedianiline (3.00 g, 15.1 mmol) in anh THF (50 mL) at room temp was added a solution of di-tert-butyl dicarbonate (3.30 g, 15.1 mmol) in anh THF (10 mL). The reaction mixture was heated at the reflux temp. for 3 h, at which time TLC indicated the presence of unreacted methylenedianiline. Additional di-*tert*-butyl dicarbonate (0.664 g, 3.03 mmol, 0.02 equiv) was added and the reaction stirred at the reflux temp. for 16 h. The resulting mixture was diluted with Et₂O (200 mL), sequentially washed with a saturated NaHCO₃ solution (100 ml), water (100 mL) and a saturated NaCl solution (50 mL), dried (MgSO₄) and concentrated under reduced pressure. The resulting white solid was purified by silica gel chromatography (gradient from 33% EtOAc/67% hexane to 50% EtOAc/50% hexane) to afford the desired product as a white solid (2.09 g, 46%): TLC (50% EtOAc/50% hexane) R*_{f}* 0.45; ¹H-NMR (DMSO-d₆) δ 1.43 (s, 9H), 3.63 (s, 2H), 4.85 (br s, 2H), 6.44 (d, *J*=8.4 Hz, 2H), 6.80 (d, *J*=8.1 Hz, 2H), 7.00 (d, *J*=8.4 Hz, 2H), 7.28 (d, *J*=8.1 Hz, 2H), 9.18 (br s, 1H); FAB-MS *m*/*z* 298 (M⁺).

### B7. General Method for the Synthesis of Aryl Amines via Electrophilic Nitration Followed by Reduction

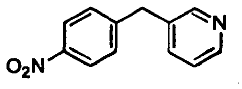

**Step 1. 3-(4-Nitrobenzyl)pyridine:** A solution of 3-benzylpyridine (4.0 g, 23.6 mmol) and 70% nitric acid (30 mL) was heated overnight at 50 °C. The resulting mixture was allowed to cool to room temp. then poured into ice water (350 mL). The aqueous mixture then made basic with a 1N NaOH solution, then extracted with Et₂O (4 x 100 mL). The combined extracts were sequentially washed with water (3 x 100 mL) and a saturated NaCl solution (2 x 100 mL), dried (Na₂SO₄), and concentrated in *vacuo.* The residual oil was purified by MPLC (silica gel; 50 % EtOAc/50% hexane) then recrystallization (EtOAc/hexane) to afford the desired product (1.0 g, 22%): GC-MS *m*/*z* 214 (M⁺). 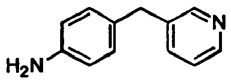 **Step 2. 3-(4-Pyridinyl)methylaniline: 3-(4-Nitrobenzyl)pyridine was reduced to the** aniline in a manner analogous to that described in Method B1.

### B8. General Method for Synthesis of Aryl Amines via Substitution with Nitrobenzyl Halides Followed by Reduction

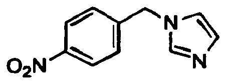

**Step 1. 4-(1-Imidazolylmethyl)-1-nitrobenzene:** To a solution of imidazole (0.5 g, 7.3 mmol) and 4-nitrobenzyl bromide (1.6 g, 7.3 mmol) in anh acetonitrile (30 mL) was added K₂CO₃ (1.0 g, 7.3 mmol). The resulting mixture was stirred at rooom temp. for 18 h and then poured into water (200 mL) and the resulting aqueous solution wasextracted with EtOAc (3 x 50 mL). The combined organic layers were sequentially washed with water (3 x 50 mL) and a saturated NaCl solution (2 x 50 mL), dried (MgSO₄), and concentrated *in vacuo.* The residual oil was purified by MPLC (silica gel; 25% EtOAc/75% hexane) to afford the desired product (1.0 g, 91%): EI-MS *m*/*z* 203 (M⁺). 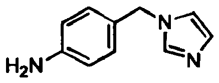 **Step 2. 4-(1-Imidazolylmethyl)aniline:** 4-(1-Imidazolylmethyl)-1-nitrobenzene was reduced to the aniline in a manner analogous to that described in Method B2.

### B9. Formation of Substituted Hydroaymethylanilines by Oxidation of Nitrobenzyl Compounds Followed by Reduction

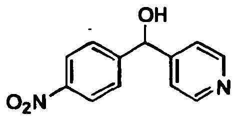

**Step 1. 4-(1-Hydroxy-1-(4-pyridyl)methyl-1-nitrobenzene:** To a stirred solution of 3-(4-nitrobenzyl)pyridine (6.0 g, 28 mmol) in CH₂Cl₂ (90 mL) was added m-CPBA (5.80 g, 33.6 mmol) at 10 °C, and the mixture was stirred at room temp. overnight: The reaction mixture was successively washed with a 10% NaHSO₃ solution (50 mL), a saturated K₂CO₃ solution (50 mL) and a saturated NaCl solution (50 mL), dried (MgSO₄) and concentrated under reduced pressure. The resulting yellow solid (2.68 g) was dissolved in anh acetic anhydride (30 mL) and heated at the reflux temperature overnight. The mixture was concentrated under reduced pressure. The residue was dissolved in MeOH (25 mL) and treated with a 20% aqueous NH₃ solution (30 mL). The mixture was stirred at room temp. for 1 h, then was concentrated under reduced pressure. The residue was poured into a mixture of water (50 mL) and CH₂Cl₂ (50 mL). The organic layer was dried (MgSO₄), concentrated under reduced pressure, and purified by column chromatography (80% EtOAc/ 20% hexane) to afford the desired product as a white solid. (0.53 g, 8%): mp 110-118 °C; TLC (80% EtOAc/20% hexane) R*_{f}* 0.12; FAB-MS *m*/*z* 367 ((M+H)⁺, 100%). 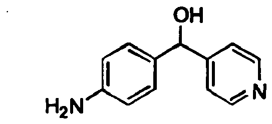 **Step 2. 4-(1-Hydroxy-1-(4-pyridyl)methylaniline:** 4-(1-Hydroxy-1-(4-pyridyl)-methyl-1-nitrobenzene was reduced to the aniline in a manner analogous to that described in Method B3d, Step2.

### B10. Formation of 2-(N-methylcarbamoyl)pyridines via the Menisci reaction (not part of claimed invention)

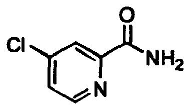

**Step 1. 2-(*N*-methylcarbamoyl)-4-chloropyridine.** (**Caution:** this is a highly hazardous, potentially explosive reaction.) To a solution of 4-chloropyridine (10.0 g) in N-methylformamide (250 mL) under argon at ambient temp was added conc. H₂SO₄ (3.55 mL) (exotherm). To this was added H₂O₂ (17 mL, 30% wt in H2O) followed by FeSO₄·7H2O (0.55 g) to produce an exotherm. The reaction was stirred in the dark at ambient temp for 1h then was heated slowly over 4 h at 45 °C. When bubbling subsided,the reaction was heated at 60 °C for 16 h. The opaque brown solution was diluted with H2O (700 mL) fol.lowed by a 10% NaOH solution (250 mL). The aqueous mixture was extracted with EtOAc (3 x 500 mL) and the organic layers were washed separately with a saturated NaCl solution (3 x 150 mlL. The combined organics were dried (MgSO₄) and filtered through a pad of silica gel eluting with EtOAc. The solvent was removed in vacuo and the brown residue was purified by silica gel chromatography (gradient from 50% EtOAc / 50% hexane to 80% EtOAc / 20% hexane). The resulting yellow oil crystallized at 0 °C over 72 h to give 2-(N rnethylcarbamoyl)-4-chloropyridine in yield (0.61 g, 5.3%): TLC (50% EtOAc/50% hexane) R*_{f}* 0.50; MS; ¹H NMR (CDCl₃): d 8.44 (d, 1 H, J = 5.1 Hz, CHN), 8.21 (s, 1H, CHCCO), 7.96 (b s, 1H, NH), 7.43 (dd, 1H, J = 2.4, 5.4 Hz, ClCHCN), 3.04 (d, 3H, J = 5.1 Hz, methyl); CI-MS *m*/*z* 171 ((M+H)+).

### B11. General method for the Synthesis of ω-Sulfonylphenyl Anilines

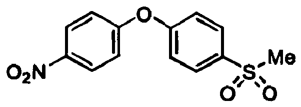

**Step 1. 4-(4-Methylsulfonylphenoxy)-1-nitrobenzene:** To a solution of 4-(4-methylthiophenoxy)-1-ntirobenzene (2 g, 7.66 mmol) in CH₂Cl₂ (75 mL) at 0 °C was slowly added *m*CPBA (57-86%, 4 g), and the reaction mixture was stirred at room temperature for 5 h. The reaction mixture was treated with a 1 N NaOH solution (25 mL). The organic layer was sequentially washed with a 1N NaOH solution (25 mL), water (25 mL) and a saturated NaCl solution (25 mL), dried (MgSO₄) and concentrated under reduced pressure to give 4-(4-methylsulfonylphenoxy)-1-nitrobenzene as a solid (2.1 g).
**Step 2. 4-(4-Methylsulfonylphenoxy)-1-aniline:** 4-(4-Methylsulfonylphenoxy)-1-nitrobenzene was reduced to the aniline in a manner anaologous to that described in Method B3d, step 2.

### B12. General Method for Synthesis of ω-Alkoxy-ω-carboxyphenyl Anilines

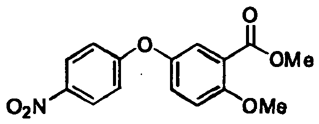

**Step 1. 4-(3-Methoxycarbonyl-4-methoxyphenoxy)-1-nitrobenzene:** To a solution of -(3-carboxy-4-hydroxyphenoxy)-1-nitrobenzene (prepared in a manner analogous to that described in Method B3a, step 1, 12 mmol) in acetone (50 mL) was added K₂CO₃ (5 g) and dimethyl sulfate (3.5 mL). The resulting mixture was heated aaaaaat the reflux tempoerature overnight, then cooled to room temperature and filtered through a pad of Celite^{®}. The resulting solution was concentrrated under reduced pressure, absorbed onto silica gel, and purified by column chromatography (50% EtOAc / 50% hexane) to give 4-(3-methoxycarbonyl-4-methoxyphenoxy)-1-nitrobenzene as a yellow powder (3 g): mp 115 118 °C. 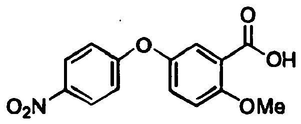 **Step 2. 4-(3-Carboxy-4-methoxyphenoxy)-1-nitrobenzene:** A mixture of 4-(3-methoxycarbonyl-4-methoxyphenoxy)-1-nitrobenzene (1.2 g), KOH (0.33 g),and water (5 mL) in MeOH (45 mL) was stirred at room temperature overnight and then heated at the reflux temperature for 4 h. The resulting mixture was cooled to room temperature and concentrated under reduced pressure. The residue was dissolved in water (50 mL), and the aqueous mixture was made acidic with a 1N HCl solution. The resulting mixture was extracted with EtOAc (50 mL). The organic layer was dried (MgSO₄) and concentrated under reduced pressure to give 4-(3-carboxy-4-methoxyphenoxy)-1-nitrobenzene (1.04 g).

### C. General Methods of Urea Formation

### C1a. Reaction of a Heterocyclic Amine with an Isocyanate (not part of claimed invention)

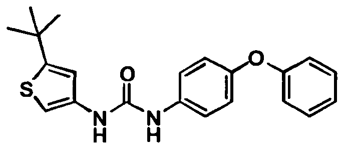

***N*-(5-*tert*-Butyl-3-thienyl)-*N'*-(4-phenoxyphenyl)urea:** To a solution of 5*-tert-*butyl-3-thiophene-ammonium chloride (prepared as described in Method A4b; 7.28 g, 46.9 mmol, 1.0 equiv) in anh DMF (80 mL) was added 4-phenoxyphenyl isocyanate (8.92 g, 42.21 mmol, 0.9 equiv) in one portion. The resulting solution was stirred at 50-60 °C overnight, then diluted with EtOAc (300 mL). The resulting solution was sequentially washed with H₂O (200 mL), a 1 N HCl solution (50 mL) and a saturated NaCl solution (50 mL), dried (Na₂SO₄), and concentrated under reduced pressure. The resulting off-white solid was recrystallized (EtOAc/hexane) to give a white solid (13.7 g, 88%), which was contaminated with approximately 5% of bis(4-phenoxyphenyl)urea. A portion of this material (4.67 g) was purified by flash chromatography (9% EtOAc/27% CH₂Cl₂/64% cyclohexane) to afforded the desired product as a white solid (3.17 g).

### C1b. Reaction of a Heterocyclic Amine with an Isocyanate (not part of claimed invention)

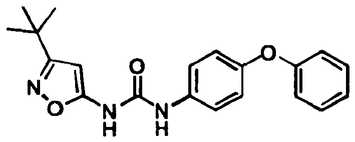

***N*-(3-tert Butyl-5-isoxazolyl)-*N'*-(4-phenoxyphenyl)urea:** To a solution of 5-amino-3-*tert*-butylisoxazole (8.93 g, 63.7 mmol, 1 eq.) in CH₂Cl₂ (60 mL) was added 4-phenyloxyphenyl isocyanate (15.47 g, 73.3 mmol, 1.15 eq.) dropwise. The mixture was heated at the reflux temp. for 2 days, eventually adding additional CH₂Cl₂ (80 mL). The resulting mixture was poured into water (500 mL) and extracted with Et₂O (3 x 200 mL). The organic layer was dried (MgSO₄) then concentrated under reduced pressure. The residue was recrystallized (EtOAc) to give the desired product (15.7 g, 70%): mp 182-184 °C; TLC (5% acetone/95% acetone) R*_{f}* 0.27; ¹H-NMR (DMSO-d₆) δ 1.23 (s, 9H), 6.02 (s, 1H), 6.97 (dd, *J*=0.2, 8.8 Hz, 2H), 6.93 (d, *J*=8.8 Hz, 2H), 7.08 (t, *J*=7.4 Hz, 1H), 7.34 (m, 2H), 7.45 (dd, *J*=2.2, 6.6 Hz, 2H) , 8.80 (s, 1H), 10.04 (s, 1H); FAB-MS *m*/*z* (rel abundance) 352 ((M+H)⁺,70%).

### C1c. Reaction of a Heterocyclic Amine with an Isocyanate

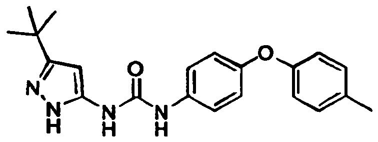

**N-(3-*tert*-Butyl-5-pyrazolyl)-N'-(4-(4-methylphenyl)oxyphenyl)urea:** A solution of 5-amino-3-*tert*-butylpyrazole (0.139 g, 1.0 mmol, 1.0 equiv) and 4-(4-methylphenoxy)phenyl isocyanate (0.225 g, 1.0 mmol 1.0 equiv) in toluene (10 mL) was heated at the reflux temp. overnight The resulting mixture was cooled to room temp and quenched with MeOH (a few mL). After stirring for 30 min, the mixture was concentrated under reduced pressure. The residue was purified by prep. HPLC (silica, 50% EtOAc/50% hexane) to give the desired product (0.121 g, 33%): mp 204 °C; TLC (5% acetone/95% CH₂Cl₂) R_{f} 0.92; ¹H-NMR (DMSO-d₆) δ 1.22 (s, 9H), 2.24 (s, 3H), 5.92 (s, 1H), 6.83 (d, *J*=8.4 Hz, 2H), 6.90 (d, *J*=8.8 Hz, 2H), 7.13 (d, *J*=8.4 Hz, 2H), 7.40 (d, J=8.8 Hz, 2H), 8.85 (s, 1H), 9.20 (br s, 1H), 11.94 (br s, 1H); EI-MS m/z 364 (M⁺).

### C1d. Reaction of a Heterocyclic Amine with an Isocyanate (not part of claimed invention)

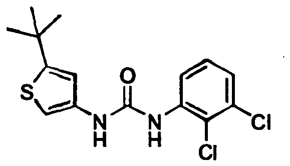

***N*-(5-tert-Butyl-3-thienyl)-*N*'-(2,3-dichlorophenyl)urea:** Pyridine (0.163 mL, 2.02 mmol) was added to a slurry of 5-*tert*-butylthiopheneammonium chloride (Method A4c; 0.30 g, 1.56 mmol) and 2,3-dichlorophenyl isocyanate (0.32 mL, 2.02 mmol) in CH₂Cl₂ (10 mL) to clarify the mixture and the resulting solution was stirred at room temp. overnight. The reaction mixture was then concentrated under reduced pressure and the residue was separated between EtOAc (15 mL) and water (15 mL). The organic layer was sequentially washed with a saturated NaHCO₃ solution (15 mL), a 1N HCl solution (15 mL) and a saturated NaCl solution (15 mL), dried (Na₂SO₄), and concentrated under reduced pressure. A portion of the residue was by preparative HPLC (C-18 column; 60% acetonitrile/40% water/0.05% TFA) to give the desired urea (0.180 g, 34%): mp 169-170 °C; TLC (20% EtOAc/80% hexane) R_{f} 0.57; ¹H-NMR (DMSO-d₆) δ 1.31 (s, 9H), 6.79 (s, 1H), 7.03 (s, 1H), 7.24-7.33 (m, 2H), 8.16 (dd, *J*=1.84, 7.72 Hz, 1H), 8.35 (s, 1H), 9.60 (s, 1H); ¹³C-NMR (DMSO-d₆) δ 31.9 (3C), 34.0, 103.4, 116.1, 119.3, 120.0, 123.4, 128.1, 131.6, 135.6, 138.1, 151.7, 155.2; FAB-MS m/z (rel abundance) 343 ((M+H)⁺, 83%), 345 ((M+H+2)⁺, 56%), 347 ((M+H+4)⁺, 12%).

### C1e. Reaction of a Heterocyclic Amine with an Isocyanate

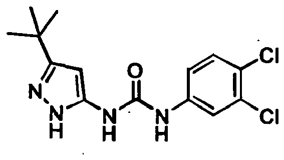

**N-(3-*tert*-Butyl-5-pyrazolyl)-N'-(3,4-dichlorophenyl)urea:** A solution of 5-amino-3-*tert*-butyl-*N¹*-(*tert*-butoxycarbonyl)pyrazole (Method A5; 0.150 g, 0.63 mmol) and 3,4-dichlorophenyl isocyanate (0.118 g, 0.63 mmol) were in toluene (3.1 mL) was stirred at 55 °C for 2 d. The toluene was removed *in vacuo* and the solid was redissolved in a mixture of CH₂Cl₂ (3 mL) and TFA (1.5 mL). After 30 min, the solvent was removed *in vacuo* and the residue was taken up in EtOAc (10 mL). The resulting mixture was sequentially washed with a saturated NaHCO₃ solution (10 mL) and a NaCl solution (5 mL), dried (Na₂SO₄), and concentrated *in vacuo.* The residue was purified by flash chromatography (gradient from 40% EtOAc/ 60% hexane to 55%EtOAc/ 5% hexane) to give the desired product (0.102 g, 48%): mp 182-184 °C; TLC (40% EtOAc/60% hexane) R*_{f}* 0.05, FAB-MS *m*/*z* 327 ((M+H)⁺).

### C2a. Reaction of a Heterocyclic Amine with Phosgene to Form an Isocyanate, then Reaction with Substituted Aniline (not part of claimed invention)

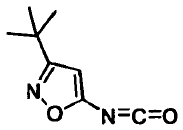

**Step 1. 3-*tert*-Butyl-5-isoxazolyl Isocyanate:** To a solution of phosgene (20% in toluene, 1.13 mL, 2.18 mmol) in CH₂Cl₂ (20 mL) at 0 °C was added anh. pyridine (0.176 mL, 2.18 mmol), followed by 5-amino-3-tert-butylisoxazole (0.305 g, 2.18 mmol). The resulting solution was allowed to warm to room temp. over 1 h, and then was concentrated under reduced pressure. The solid residue dried *in vacuo* for 0.5 h. 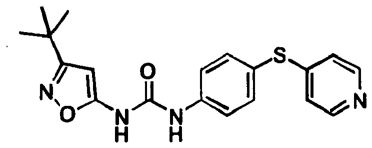 **Step 2**. *N*-(3-*tert*-Butyl-5-isoxazolyl)-*N*'-(4-(4-pyridinylthio)phenyl)urea: The crude 3-*tert*-butyl-5-isoxazolyl isocyanate was suspended in anh toluene (10 mL) and 4-(4-pyridinylthio)aniline (0.200 g, 0.989 mmol) was rapidly added. The suspension was stirred at 80 °C for 2 h then cooled to room temp. and diluted with an EtOAc/CH₂Cl₂ solution (4:1, 125 mL). The organic layer was washed with water (100 mL) and a saturated NaCl solution (50 mL), dried (MgSO₄), and concentrated under reduced pressure. The resulting yellow oil was purified by column chromatography (silica gel, gradient from 2% MeOH/98% CH₂Cl₂ to 4% MeOH/6% CH₂Cl₂) to afford a foam, which was triturated (Et₂O/hexane) in combination with sonication to give the product as a white powder (0.18 g, 49%): TLC (5% MeOH/95% CH₂Cl₂) R*_{f}* 0.21; ¹H-NMR (DMSO-d₆) δ 1.23 (s, 9H), 6.06 (s, 1H), 6.95 (d, *J*=5 Hz, 2H), 7.51 (d, *J*=8 Hz, 2H), 7.62 (d, *J*=8 Hz, 2H), 8.32 (d, *J*=5 Hz, 2H), 9.13 (s, 1H), 10.19 (s, 1H); FAB-MS m/z 369 ((M+H)⁺).

### C2b. Reaction of a Heterocyclic Amine with Phosgene to Form an Isocyanate Followed by Reaction with Substituted Aniline (not part of claimed invention)

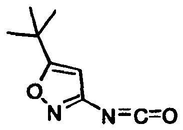

**Step 1. 5-*tert* Butyl-3-isoxazolyl Isocyanate:** To a solution of phosgene (148 mL, 1.93 M in toluene, 285 mmol) in anhydrous CH₂Cl₂ (1 L) was added 3-amino-5-*tert-*butylisoxazole (10.0 g, 71 mmol) followed by pyridine (46 mL, 569 mmol). The mixture was allowed to warm to room temp and stirred overnight (ca. 16 h), then mixture was concentrated *in vacuo.* The residue was dissolved in anh. THF (350 mL) and stirred for 10 min. The orange precipitate (pyridinium hydrochloride) was removed and the isocyanate-containing filtrate (approximately 0.2 M in THF) was used as a stock solution: GC-MS (aliquot obtained prior to concentration) m/z 166 (M ⁺). 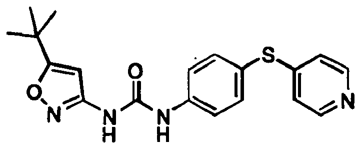 **Step 2. *N*-5-*tert*-Butyl-3-isoxazolyl)-*N*'-(4-(4-pyridinylthio)phenyl)urea: To a** solution of 5-*tert*-butyl-3-isoxazolyl isocyanate (247 mL, 0.2 M in THF, 49.4 mmol) was added 4-(4-pyridinylthio)aniline (5 g, 24.72 mmol), followed by THF (50 mL) then pyridine (4.0 mL, 49 mmol) to neutralize any residual acid. The mixture was stirred overnight (ca. 18 h) at room temp. Then diluted with EtOAc (300 mL). The organic layer was washed successively with a saturated NaCl solution (100 mL), a saturated NaHCO3 solution (100 mL), and a saturated NaCl solution (100 mL), dried (MgSO₄), and concentrated *in vacuo.* The resulting material was purified by MPLC (2 x 300 g silica gel, 30 % EtOAc/70% hexane) to afford the desired product as a white solid (8.24 g, 90 %): mp 178-179 °C; ¹H-NMR (DMSO-d₆) δ 1.28 (s, 9H), 6.51 (s, 1H), 6.96 (d, *J*=6.25 Hz, 2H), 7.52 (d, *J*=8.82 Hz, 2H), 7.62 (d, *J*=8.83 Hz, 2H), 8.33 (d, *J*=6.25 Hz, 2H), 9.10 (s, 1H), 9.61 (s, 1H); EI-MS *m*/*z* 368 (M⁺).

### C2c. Reaction of a Heterocyclic Amine with Phosgene to Form an Isocyanate Followed by Reaction with Substituted Aniline

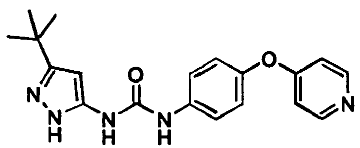

*N*-(3-*tert*-Butyl-5-pyrazolyl)-*N'*-(4-(4-pyridinylozy)phenyl)urea: To a solution of phosgene (1.9M in toluene, 6.8 mL) in anhydrous CH₂Cl₂ (13 mL) at 0 °C was slowly added pyridine (0.105 mL) was added slowly over a 5 min, then 4-(4-pyridinyloxy)aniline (0.250 g, 1.3 mmol) was added in one aliquot causing a transient yellow color to appear. The solution was stirred at 0 °C for 1 h, then was allowed to warm to room temp. over 1 h. The resulting solution was concentrated *in vacuo* then the white solid was suspended in toluene (7 mL). To this slurry, 5-amino-3-*tert-*butyl-*N^{t}*-(*tert*-butoxycarbonyl)pyrazole (0.160 g, 0.67 mmol) was added in one aliquot and the reaction mixture was heated at 70 °C for 12 h forming a white precipitate. The solids were dissolved in a 1N HCl solution and allowed to stir at room temp. for 1 h to form a new precipitate. The white solid was washed (50% Et₂O/50% pet. ether) to afford the desired urea (0.139 g, 59%): mp >228 °C dec; TLC (10% MeOH/ 90% CHCl₃) R*_{f}* 0.239; ¹H-NMR (DMSO-d₆) δ 1.24 (s, 9H), 5.97 (s, 1H), 6.88 (d, *J*=6.25 Hz, 2H), 7.10 (d, *J*=8.82 Hz, 2H), 7.53 (d, J=9.2 Hz, 2H), 8.43 (d, *J*=6.25 Hz, 2H), 8.92 (br s, 1H), 9.25 (br s, 1H), 12.00 (br s, 1H); EI-MS m/z rel abundance 351 (M⁺, 24%).

### C3a. Reaction of a Heterocyclic Amine with N,N'-Carbonyldiimidazole Followed by Reaction with a Substituted Aniline

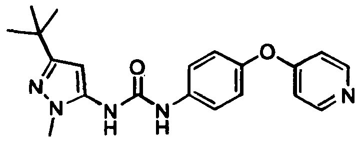

***N*-(3-tert-Butyl-1-methyl-5-pyrazolyl)-*N*'-(4-(4-pyridinyloxy)phenyl)urea:** To a solution of 5-amino-3-*tert*-butyl-1-methylpyrazole (189 g, 1.24 mol) in anh. CH₂Cl₂ (2.3 L) was added *N,N'*carbonyldiimidazole (214 g, 1.32 mol) in one portion. The mixture was allowed to stir at ambient temperature for 5 h before adding 4-(4-pyridinyloxy)aniline. The reaction mixture was heated to 36 °C for 16 h. The resulting mixture was cooled to room temp, diluted with EtOAc (2 L) and washed with H₂O (8 L) and a saturated NaCl solution (4 L). The organic layer was dried (Na₂SO₄) and concentrated *in vacuo.* The residue was purified by crystallization (44.4% EtOAc/44.4% Et₂O/11.2% hexane, 2.5 L) to afford the desired urea as a white solid (230 g, 51%): mp 149-152 °C; ¹H-NNM (DMSO-d₆) δ 1.18 (s, 9H), 3.57 (s, 3H), 6.02 (s, 1H), 6.85 (d, *J*=6.0 Hz, 2H), 7.08 (d, *J*=9.0 Hz, 2H), 7.52 (d, *J*=9.0 Hz, 2H), 8.40 (d, *J*=6.0 Hz, 2H), 8.46 (s, 1H), 8.97 (s, 1H); FAB-LSIMS m/z 366 ((M+H) ⁺).

### C3b. Reaction of a Heterocyclic Amine with N,N'-Carbonyldiimidazole Followed by Reaction with a Substituted Aniline

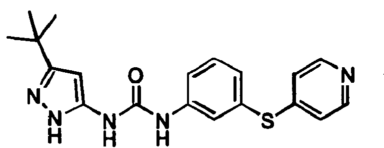

***N*-(3-*tert*-Butyl-5-pyrazolyl)-N'-(3-(4-pyridinylthio)phenyl)urea:** To a solution of 5-amino-3-*tert*-butyl-*N'*-(*tert*-butoxycarbonyl)pyrazole (0.282 g, 1.18 mmol) in CH₂Cl₂ (1.2 mL) was added *N*,*N*'-carbonyldiimidazole (0.200 g, 1.24 mmol) and the mixture was allowed to stir at room temp. for 1 day. 3-(4-Pyridinylthio)aniline (0.239 g, 1.18 mmol) was added to the reaction solution in one aliquot and the resulting mixture was allowed to stir at room temp. for 1 day. Then resulting solution was treated with a 10% citric acid solution (2 mL) and was allowed to stir for 4 h. The organic layer was extracted with EtOAc (3 x 15 mL), dried (MgSO₄), and concentrated *in vacuo.* The residue was diluted with CH₂Cl₂ (5 mL) and trifluoroacetic acid (2 mL) and the resulting solution was allowed to stir for 4 h. The trifluoroacetic reaction mixture was made basic with a saturated NaHCO₃ solution, then extracted with CH₂Cl₂ (3 x 15 mL). The combined organic layers were dried (MgSO₄) and concentrated *in vacuo.* The residue was purified by flash chromatography (5% MeOH/95% CH₂Cl₂). The resulting brown solid was triturated with sonication (50% Et₂O/50% pet. ether) to give the desired urea (0.122 g, 28%): mp >224 °C dec; TLC (5% MeOH/ 95% CHCl₃) R_{f} 0.067; ¹H-NMR (DMSO-d₆) δ 1.23 (s, 9H), 5.98 (s, 1H), 7.04 (dm, *J*=13.24 Hz, 2H), 7.15-7.19 (m, 1H), 7.40-7.47 (m, 2H), 7.80-7.82 (m, 1H), 8.36 (dm, *J*=15.44 Hz, 2H), 8.96 (br s, 1H), 9.32 (br s, 1H), 11.97 (br s, 1H); FAB-MS m/z (rel abundance) 368 (M⁺, 100%).

### C4a. Reaction of Substituted Aniline with N,N'-Carbonyldiimidazole Followed by Reaction with a Heterocyclic Amine

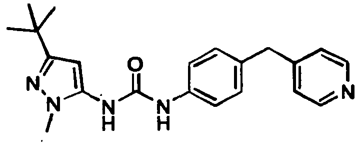

***N*-(3-*tert*-Butyl-1-methyl-5-pyrazolyl)-N'-(4-(4-pyridinylmethyl)phenyl)urea:** To a solution of 4-(4-pyridinylmethyl)aniline (0.200 g, 1.08 mmol) in CH₂Cl₂ (10 mL) was added N,N'-carbonyldiimidazole (0.200 g, 1.23 mmol). The resulting mixture was stirred at room tempe for 1 h after which TLC analysis indicated no starting aniline. The reaction mixture was then treated with 5-amino-3-*tert*-butyl-1-methylpyrazole (0.165 g, 1.08 mmol) and stirred at 40-45 °C overnight. The reaction mixture was cooled to room temp and purified by column chromatography (gradient from 20% acetone/80% CH₂Cl₂ to 60% acetone/40% CH₂Cl₂) and the resulting solids were crystallized (Et2O) to afford the desired urea (0.227 g, 58%): TLC (4% MeOH/ 96% CH₂Cl₂) R*_{f}* 0.15; ¹H-NMR (DMSO-d₆) δ 1.19 (s, 9H), 3.57 (s, 3H), 3.89 (s, 2H), 6.02 (s, 1H), 7.14 (d, *J*=8.4 Hz, 2H), 7.21 (d, *J*=6 Hz, 2H), 7.37 (d, *J*=8.4 Hz, 2H), 8.45-8.42 (m, 3H), 8.81 (s, 1H); FAB-MS *m*/*z* 364 (M+H)⁺).

### C4b. Reaction of Substituted Aniline with N,N'-Carbonyldiimidazole Followed by Reaction with a Heterocyclic Amine

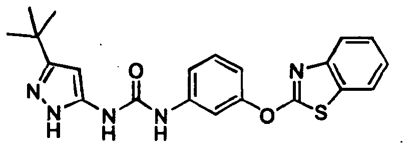

***N*-(3-*tert*-Butyl-5-pyrazolyl)-*N*'-(3-(2-benzothiazolyloxy)phenyl)urea:** A solution of 3-(2-benzothiazolyloxy)aniline (0.24 g, 1.0 mmol, 1.0 equiv) and *N.N'-*carbonyldiimidazole (0.162 g, 1.0 mmol, 1.0 equiv) in toluene (10 mL) was stirred at room temp for 1 h. 5-Amino-3-tert-butylpyrazole (0.139 g, 1.0 mmol) was added and the resulting mixture was heated at the reflux temp. overnight. The resulting mixture was poured into water and extracted with CH₂Cl₂ (3 x 50 mL). The combined organic layers were concentrated under reduced pressure and dissolved in a minimal amount of CH₂Cl₂. Petroleum ether was added and resulting white precipitate was resubmitted to the crystallization protocol to afford the desired product (0.015 g, 4%): mp 110-111°C; TLC (5% acetone/95% CH₂Cl₂) R*_{f}* 0.05; ¹H-NMR (DMSO-d₆) δ 1.24 (s, 9H), 5.97 (s, 1H), 7.00-7.04 (m, 1H), 7.21-7.44 (m, 4H), 7.68 (d, *J*=5.5 Hz, 1H), 7.92 (d, *J*=7.7 Hz, 1H), 7.70 (s, 1H), 8.95 (s, 1H), 9.34 (br s, 1H), 11.98 (br s, 1H); EI-MS *m*/*z* 408 (M⁺).

### C4c. Reaction of a Heterocyclic Amine with Phosgene to Form an Isocyanate Followed by Reaction with Substituted Aniline (not part of claimed invention)

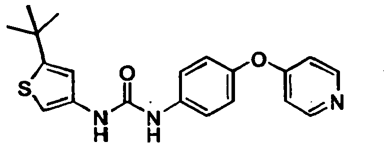

**N-(5-*tert*-Butyl-3-thienyl)-N'-(4-(4-pyridinylogy)phenyl)urea:** To an ice cold solution phosgene (1.93M in toluene; 0.92 mL, 1.77 mmol) in CH₂Cl₂ (5 mL) was added a solution of 4-(4-pyridinyloxy)aniline (0.30 g, 1.61 mmol) and pyridine (0.255 g, 3.22 mmol) in CH₂Cl₂ (5 mL). The resulting mixture was allowed to warm to room temp. and was stirred for 1 h, then was concentrated under reduced pressure. The residue was dissolved in CH₂Cl₂ (5 mL), then treated with 5*-tert-*butylthiopheneammonium chloride (Method A4c; 0.206 g, 1.07 mmol), followed by pyridine (0.5 mL). The resulting mixture was stirred at room temp for 1 h, then treated with 2-(dimethylamino)ethylamine (1 mL), followed by stirring at room temp an additional 30 min. The reaction mixture was then diluted with EtOAc (50 mL), sequentially washed with a saturated NaHCO₃ solution (50 mL) and a saturated NaCl solution (50 mL), dried (Na₂SO₄), and concentrated under reduced pressure. The residue was purified by column chromatography (gradient from 30% EtOAc/70% hexane to 100% EtOAc) to give the desired product (0.38 g , 97%): TLC (50% EtOAc/50% hexane) R*_{f}* 0.13; ¹H-NMR (CDCl₃) δ 1.26 (s, 9H), 6.65 (d, *J*=1.48 Hz, 1H), 6.76 (dd, *J*=1.47, 4.24 Hz, 2H), 6.86 (d, *J*=1.47 Hz, 1H), 6.91 (d, *J*=8.82 Hz, 2H), 7.31 (d, *J*=8.83 Hz, 2H), 8.39 (br s, 2H), 8.41 (d, *J*=1.47 Hz, 2H); ¹³C-NMR (CDCl₃) δ 32.1 (3C), 34.4, 106.2, 112.0 (2C), 116.6, 121.3 (2C), 121.5 (2C), 134.9, 136.1, 149.0, 151.0 (2C), 154.0, 156.9, 165.2; FAB-MS *m*/*z* (rel abundance) 368 ((M +H)⁺, 100%).

### C5. General Method for the Reaction of a Substituted Aniline with Triphosgene Followed by Reaction with a Second Substituted Amine

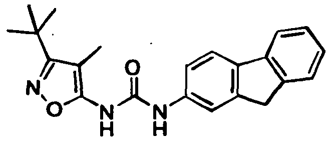

***N*-(3-*tert*-Butyl-4-methyl-5-isoxazolyl)-N'-(2-fluorenyl)urea:** To a solution of triphosgene (55 mg, 0.185 mmol, 0.37eq) in 1,2-dichloroethane (1.0mL) was added a solution of 5-amino-4-methyl-3-*tert*-butylisoxazole (77.1 mg, 0.50 mmol, 1.0 eq) and diisopropylethylamine (0.104 mL, 0.60 mmol, 1.2 eq) in 1,2-dichloroethane (1.0 mL). The reaction mixture was stirred at 70 °C for 2 h, cooled to room temp., and treated with a solution of 2-aminofluorene (30.6 mg, 0.50 mmol, 1.0 eq) and diisopropylethylamine (0.087 mL, 1.0 eq) in 1,2-dichloroethane (1.0 mL). The reaction mixture was stirred at 40 °C for 3 h and then at RT for 17 h to produce a precipitate. The solids were washed with Et₂O and hexanes to give the desired urea as a beige solid (25 mg, 14%): mp 179-181 °C; ¹H-NMR (DMSO-d₆) δ 1.28 (s, 9H), 2.47 (s, 3H), 3.86 (s, 2H), 7.22 (t, *J*=7.3 Hz, 1H), 7.34 (m, 2H), 7.51 (d, *J*=7.3 Hz, 1H), 7.76 (m, 3H), 8.89 (s, 1H), 9.03 (s, 1H); HPLC ES-MS *m*/*z* 362 ((M+H)⁺).

### C6. General Method for Urea Formation by Curtius Rearrangement and Carbamate Trapping

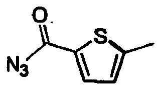

**Step 1. 5-Methyl-2-(azidocarbonyl)thiophene:** To a solution of 5-Methyl-2-thiophenecarboxylic acid (1.06 g, 7.5 mmol) and Et₃N (1.25 mL, 9.0 mmol) in acetone (50 mL) at -10 °C was slowly added ethyl chloroformate (1.07 mL, 11.2 mmol) to keep the internal temperature below 5 °C. A solution of sodium azide (0.83 g, 12.7 mmol) in water (6 mL) was added and the reaction mixture was stirred for 2 h at 0 °C. The resulting mixture was diluted with CH₂Cl₂ (10 mL) and washed with a saturated NaCl solution (10 mL). The aqueous layer was back-extracted with CH₂Cl₂ (10 mL), and the combined organic layers were dried (MgSO₄) and concentrated *in vacuo.* The residue was purified by column chromatography (10% EtOAc/ 90% hexanes) to give the azidoester (0.94 g, 75%). Azidoester (100 mg, 0.6 mmol) in anhydrous toluene (10 mL) was heated to reflux for 1 h then cooled to rt. This solution was used as a stock solution for subsequent reactions. 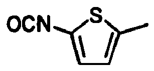 Step 2. 5-Methyl-2-thiophene Isocyanate: 5-Methyl-2-(azidocarbonyl)thiophene (0.100 g, 0.598 mmol) in anh toluene (10 mL) was heated at the reflux temp. for 1 h then cooled to room temp. This solution was used as a stock solution for subsequent reactions. 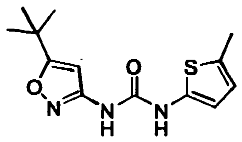 **Step 3. *N*-(5-*tert*-Butyl-3-isoxazolyl)-*N'*-(5-methyl-2-thienyl)urea: To a solution** of 5-methyl-2-thiophene isocyanate (0.598 mmol) in toluene (10 mL) at room temp. was added 3-amino-5-tert-butylisoxazole (0.092 g, 0.658 mmol) and the resulting mixture was stirred overnight. The reaction mixture was diluted with EtOAc (50 mL) and sequentially washed with a 1 N HCl solution (2 x 25 mL) and a saturated NaCl solution (25 mL), dried (MgSO₄), and concentrated under reduced pressure. The residue was purified by MPLC (20% EtOAc/80% hexane) to give the desired urea (0.156 g, 93%): mp 200-201 °C; TLC (20% EtOAc/80% hexane) R*_{f}* 0.20; EI-MS *m*/*z* 368 (M⁺).

### C7. General Methods for Urea Formation by Curtius Rearrangement and Isocyanate Trapping

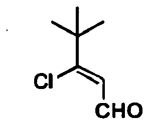

**Step 1. 3-Chloro-4,4-dimethylpent-2-enal:** POCl₃ (67.2 mL, 0.72 mol) was added to cooled (0 °C) DMF (60.6 mL, 0.78 mol) at rate to keep the internal temperature below 20 °C. The viscous slurry was heated until solids melted (approximately 40 °C), then pinacolone (37.5 mL, 0.30 mol) was added in one portion. The reaction mixture was then to 55 °C for 2h and to 75 °C for an additional 2 h. The resulting mixture was allowed to cool to room temp., then was treated with THF (200 mL) and water (200 mL), stirred vigorously for 3 h, and extracted with EtOAc (500 mL). The organic layer was washed with a saturated NaCl solution (200 mL), dried (Na₂SO₄) and concentrated under reduced pressure. The residue was filtered through a pad of silica (CH₂Cl₂) to give the desired aldehyde as an orange oil (15.5 g, 35%): TLC (5% EtOAc/95% hexane) R*_{f}* 0.54; ¹H NMR (CDCl₃) d 1.26 (s, 9H), 6.15 (d, *J*=7.0 Hz, 1H), 10.05 (d, *J*=6.6 Hz, 1H). 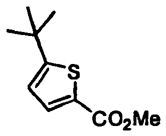 **Step 2. Methyl 5-tert-butyl-2-thiophenecarbozylate:** To a solution of 3-chloro-4,4-dimethylpent-2-enal (1.93 g, 13.2 mmol) in anh. DMF (60 mL) was added a solution of Na₂S (1.23 g, 15.8 mmol) in water (10 mL). The resulting mixture was stirred at room temp. for 15 min to generate a white precipitate, then the slurry was treated with methyl bromoacetate (2.42 g, 15.8 mmol) to slowly dissolve the solids. The reaction mixture was stirred at room temp. for 1.5 h, then treated with a 1 N HCl solution (200 mL) and stirred for 1 h. The resulting solution was extracted with EtOAc. (300 mL). The organic phase was sequentially washed with a 1 N HCl solution (200 mL), water (2 x 200 mL) and a saturated NaCl solution (200 mL), dried (Na₂SO₄) and concentrated under reduced pressure. The residue was purified using column chromatography (5% EtOAc/95% hexane) to afford the desired product (0.95 g, 36%): TLC (20% EtOAc/80% hexane) R*_{f}* 0.79; ¹H NMR (CDCl₃) δ 1.39 (s, 9H), 3.85 (s, 3H), 6.84 (d, *J*=3.7 Hz, 1H), 7.62 (d, *J*=4.1 Hz, 1H); GC-MS *m*/*z* (rel abundance) 198 (M⁺, 25%). 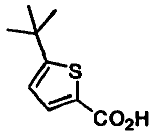 **Step 3. 5*-tert-*Butyl-2-thiophenecarbozylic acid:** Methyl 5-*tert*-butyl-2-thiophenecarboxylate (0.10 g, 0.51 mmol) was added to a KOH solution (0.33 M in 90% MeOH/10% water, 2.4 mL, 0.80 mmol) and the resulting mixture was heated at the reflux temperature for 3 h. EtOAc (5 mL) was added to the reaction mixture, then the pH was adjusted to approximately 3 using a 1 N HCl solution. The resulting organic phase was washed with water (5 mL), dried (Na₂SO₄), and concentrated under reduced pressure (0.4 mmHg) to give the desired carboxylic acid as a yellow solid (0.067 g, 73%): TLC (20% EtOAc/79.5% hexane/0.5% AcOH) R*_{f}* 0.29; ¹H NMR (CDCl₃) δ 1.41 (s, 9H), 6.89 (d, *J*=3.7 Hz, 1H), 7.73 (d, *J*=3.7 Hz, 1H), 12.30 (br s, 1H); ¹³CNMR (CDCl₃) δ 32.1 (3C), 35.2, 122.9, 129.2, 135.1, 167.5, 168.2. 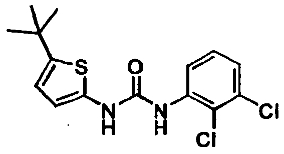 **Step 4. *N*-(5-*tert*-Butyl-2-thienyl)-*N*'-(2,3-dicblorophenyl)urea:** A mixture of 5-*tert*-butyl-2-thiophenecarboxylic acid (0.066 g, 0.036 mmol), DPPA (0.109 g, 0.39 mmol) and Et₃N (0.040 g, 0.39 mmol) in toluene (4 mL) was heated to 80 °C for 2 h, 2,3-dichloroaniline (0.116 g, 0.72 mmol) was added, and the reaction mixture was heated to 80°C for an additional 2 h. The resulting mixture was allowed to cool to room temp. and treated with EtOAc (50 mL). The organic layer was washed with a 1 N HCl solution (3 x 50 mL), a saturated NaHCO₃ solution (50 mL), and a saturated NaCl solution (50 mL), dried (Na₂SO₄), and concentrated under reduced pressure. The residue was purified by column chromatography (5% EtOAc/95% hexane) to afford the desired urea as a purple solid (0.030 g, 24%): TLC (10% EtOAc/90% hexane) R*_{f}* 0.28; ¹H NMR (CDCl₃) δ 1.34 (s, 9H), 6.59 (br s, 2H), 7.10-7.13 (m, 2H), 7.66 (br s, 1H), 8.13 (dd, *J*=2.9, 7.8 Hz, 1H); ¹³C NMR (CDCl₃) δ 32.2 (3C), 34.6, 117.4, 119.0⁷, 119.1⁵, 119.2, 121.5, 124.4, 127.6, 132.6, 135.2, 136.6, 153.4; HPLC ES-MS *m*/*z* (rel abundance) 343 ((M+H)⁺, 100%), 345 ((M+H+2)⁺, 67%), 347 ((M+H +4)⁺, 14%).

### C8. Combinatorial Method for the Synthesis of Diphenyl Ureas Using Triphosgene

One of the anilines to be coupled was dissolved in dichloroethane (0.10 M). This solution was added to a 8 mL vial (0.5 mL) containing dichloroethane (1 mL). To this was added a triphosgene solution (0.12 M in dichloroethane, 0.2 mL, 0.4 equiv.), followed by diisopropylethylamine (0.35 M in dichloroethane, 0.2 mL, 1.2 equiv.). The vial was capped and heat at 80 °C for 5 h, then allowed to cool to room temp for approximately 10 h. The second aniline was added (0.10 M in dichloroethane, 0.5 mL, 1.0 equiv.), followed by diisopropylethylamine (0.35 M in dichloroethane, 0.2 mL, 1.2 equiv.). The resulting mixture was heated at 80°C for 4 h, cooled to room temperature and treated with MeOH (0.5 mL). The resulting mixture was concentrated under reduced pressure and the products were purified by reverse phase HPLC.

### D. Misc. Methods of Urea Synthesis

### D1. Electrophylic Halogenation

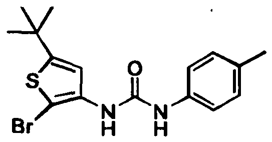

***N*-(2-Bromo-5-*tert*-butyl-3-thienyl)-*N'*-(4-methylphenyl)urea:** To a slurry of *N-*(5-*tert*-butyl-3-thienyl)-*N'*-(4-methylphenyl)urea (0.50 g, 1.7 mmol) in CHCl₃ (20 mL) at room temp was slowly added a solution of Br₂ (0.09 mL, 1.7 mmol) in CHCl₃ (10 mL) via addition funnel causing the reaction mixture to become homogeneous. Stirring was continued 20 min after which TLC analysis indicated complete reaction. The reaction was concentrated under reduced pressure, and the residue triturated (2 x Et₂O/hexane) to give the brominated product as a tan powder (0.43 g, 76%): mp 161-163 °C; TLC (20% EtOAc/ 80% hexane) R*_{f}* 0.71;¹H NMR (DMSO-d₆) δ 1.29 (s, 9H), 2.22 (s, 3H), 7.07 (d, *J*=8.46 Hz, 2H), 7.31 (d, *J*=8.46 Hz, 2H), 7.38 (s, 1H), 8.19 (s, 1H), 9.02 (s, 1H); ¹³C NMR (DMSO-d₆) δ 20.3,31.6 (3C), 34.7,89.6,117.5, 118.1 (2C), 129.2 (2C), 130.8, 136.0, 136.9, 151.8, 155.2; FAB-MS m/z (rel abundance) 367 ((M+H)⁺, 98%), 369 (M+2+H)⁺, 100%).

### D2. Synthesis of ω-Alkoxy Ureas

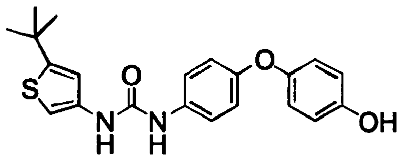

**Step 1. *N*-(5-*tert*-Butyl-3-thienyl)-*N*'-(4-(4-hydroxyphenyl)oxyphenyl)urea:** A solution of *N*-(5-*tert*-butyl-3-thienyl)-*N'*-(4-(4-methoxyphenyl)oxyphenyl)urea (1.2 g, 3 mmol) in CH₂Cl₂ (50 mL) was cooled to -78 °C and treated with BBr₃ (1.0 M in CH₂Cl₂, 4.5 mL, 4.5 mmol, 1.5 equiv) dropwise via syringe. The resulting bright yellow mixture was warmed slowly to room temp and stirred overnight The resulting mixture was concentrated under reduced pressure. The residue was dissolved in EtOAc (50 mL), then washed with a saturated NaHCO₃ solution (50 mL) and a saturated NaCl solution (50 mL), dried (Na₂SO₄), and concentrated under reduced pressure. The residue was purified via flash chromatography (gradient from 10% EtOAc/90% hexane to 25% EtOAc/75% hexane) to give the desired phenol as a tan foam (1.1 g, 92%): TLC (20% EtOAc/80% hexane) R*_{f}* 0.23; ¹H NMR (DMSO-d₆) δ 1.30 (s, 9H), 6.72-6.84 (m, 7H), 6.97 (d, *J*=1.47 Hz, 1H), 7.37 (dm, *J*=9.19 Hz, 2H), 8.49 (s, 1H), 8.69 (s, 1H), 9.25 (s, 1H); FAB-MS *m*/*z* (rel abundance) 383 ((M+H)⁺, 33%). 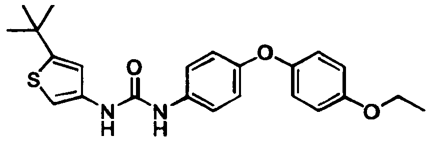 **Step 2. *N*-(5-*tert-*Butyl-3-thienyl)-*N'*-(4-(4-ethoxyphenyl)oxyphenyl)urea:** To a mixture of *N*-(5-*tert*-butyl-3-thienyl)-*N'*-(4-(4-hydroxyphenyl)oxyphenyl)urea (0.20 g, 0.5 mmol) and Cs₂CO₃ (0.18 g, 0.55 mmol, 1.1 equiv) in reagent grade acetone (10 mL) was added ethyl iodide (0.08 mL, 1.0 mmol, 2 equiv) via syringe, and the resulting slurry was heated at the reflux temp. for 17 h. The reaction was cooled, filtered, and the solids were washed with EtOAc. The combined organics were concentrated under reduced pressure, and the residue was purified via preparative HPLC (60% CH₃CN/40% H₂O/0.05% TFA) to give the desired urea as a colorless powder (0.16 g, 73%): mp 155-156 °C; TLC (20% EtOAC/ 80% hexane) R*_{f}* 0.40; ¹H-NMR (DMSO-d₆) δ 1.30 (s, 9H), 1.30 (t, *J*=6.99 Hz, 3H), 3.97 (q, *J*=6.99 Hz, 2H), 6.80 (d, *J*=1.47 Hz, 1H), 6.86 (dm, *J*=8.82 Hz, 2H), 6.90 (s, 4H), 6.98 (d, *J*=1.47, 1H), 7.40 (dm, *J*=8.83 Hz, 2H), 8.54 (s, 1H), 8.73 (s, 1H); ¹³C-NMR (DMSO-d₆) δ 14.7, 32.0 (3C), 33.9, 63.3, 102.5, 115.5 (2C), 116.3, 118.4 (2C), 119.7 (2C), 119.8 (2C), 135.0, 136.3, 150.4, 152.1, 152.4, 154.4, 154.7; FAB-MS *m*/*z* (rel abundance) 411 ((M+H)⁺, 15%).

### D3. Synthesis of ω-Carbamoyl Ureas

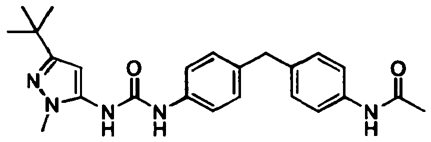

***N*-(3-*tert*-Butyl-1-methyl-5-pyrazolyl)-*N'*-(4-(4-acetaminophenyl)methylphenyl)urea:** To a solution of *N*-(3-*tert*-butyl-1-methyl-5-pyrazolyl)-*N'*-(4-(4-aminophenyl)methylphenyl)urea (0.300 g, 0.795 mmol) in CH₂Cl₂ (15 mL) at 0 °C was added acetyl chloride (0.057 mL, 0.795 mmol), followed by anhydrous Et₃N (0.111 mL, 0.795 mmol). The solution was allowed to warm to room temp over 4 h, then was diluted with EtOAc (200 mL). The organic layer was sequentially washed with a 1M HCl solution (125 mL) then water (100 mL), dried (MgSO₄ and concentrated under reduced pressure. The resulting residue was purified by filtration through a pad of silica (EtOAc) to give the desired product as a white solid (0.160 g, 48%): TLC (EtOAc) R*_{f}* 0.33; ¹H-NMR (DMSO-d₆) δ 1.17 (s, 9H), 1.98 (s, 3H), 3.55 (s, 3H), 3.78 (s, 2H), 6.00 (s, 1H), 7.07 (d, *J*=8.5 Hz, 2H), 7.09 (d, *J*=8.5 Hz, 2H), 7.32 (d, *J*=8.5 Hz, 2H), 7.44 (d, *J*=*8.5* Hz, 2H), 8.38 (s, 1H), 8.75 (s, 1H), 9.82 (s, 1H); FAB-MS *m*/*z* 420 ((M+H)⁺).

### D4. General Method for the Conversion of Ester-Containing Ureas into Alcohol-Containing Ureas

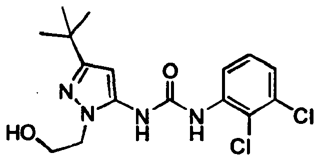

*N*-(*N¹*-(2-Hydroxyethyl)-3-*tert*-butyl-5-pyrazolyl)-*N*'-(2,3-dichlorophenyl)urea: A solution of *N*-(*N¹*-(2-(2,3-dichlorophenylamino)carbonyloxyethyl)-3-*tert*-butyl-5-pyrazolyl)-*N'*-(2,3-dichlorophenyl)urea. (prepared as described in Method A3; 0.4 g, 0.72 mmoles) and NaOH ( 0.8 mL, 5N in water, 4.0 mmoles) in EtOH (7 mL) was heated at -65 °C for 3 h at which time TLC indicated complete reaction. The reaction mixture was diluted with EtOAc (25 mL) and acidified with a 2N HCl solution (3 mL). The resulting organic phase was washed with a saturated NaCl solution (25 mL), dried (MgSO₄) and concentrated under reduced pressure. The residue was crystallized (Et₂O) to afford the desired product as a white solid (0.17 g, 64 %): TLC (60% EtOAc/40% hexane) R*_{f}* 0.16; ¹H-NMR (DMSO-d₆) δ 1.23 (s, 9H), 3.70 (t, *J*=5.7 Hz, 2H), 4.10 (t, *J*=5.7 Hz, 2H), 6.23 (s, 1H), 7.29-7.32 (m, 2H), 8.06-8.09 (m, 1H), 9.00 (br s, 1H), 9.70 (br s, 1H); FAB-MS *m*/*z* (rel abundance) 371 ((M+H)⁺, 100%).

### D5a. General Method for the Conversion of Ester-Containing Ureas into Amide-Containing Ureas

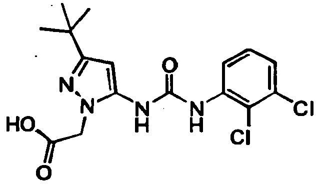

**Step 1. *N*-(*N¹*-(Carboxymethyl)-3-*tert*-butyl-5-pyrazolyl)-*N'*-(2,3-dichlorophenyl)urea:** A solution of *N*-(*N¹*-(ethoxycarbonylmethyl)-3-*tert*-butyl-5-pyrazolyl)-*N'*-(2,3-dichlorophenyl)urea (prepared as described in Method A3, 0.46 g, 1.11 mmoles) and NaOH (1.2 mL, 5N in water, 6.0 mmoles) in EtOH (7 mL) was stirred at room temp. for 2 h at which time TLC indicated complete reaction. The reaction mixture was diluted with EtOAc (25 mL) and acidified with a 2N HCl solution (4 mL). The resulting organic phase was washed with a saturated NaCl solution (25 mL), dried (MgSO₄) and concentrated under reduced pressure. The residue was crystallized (Et₂O/hexane) to afford the desired product as a white solid (0.38 g, 89%): TLC (10% MeOH/90% CH₂Cl₂) R*_{f}* 0.04;¹H-NMR (DMSO-d₆) δ 1.21 (s, 9H), 4.81 (s, 2H), 6.19 (s, 1H), 7.28-7.35 (m, 2H), 8.09-8.12 (m, 1H), 8.76 (br s, 1H), 9.52 (br s, 1H); FAB-MS *m*/*z* (rel abundance) 385 ((M+H)⁺, 100%). 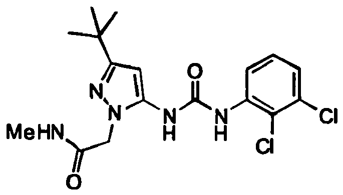 **Step 2. *N*-(*N¹*-((Methylcarbamoyl)methyl)-3-*tert*-butyl-5-pyrazolyl)-*N'*-(2,3-dichlorophenyl)urea:** A solution of *N*-(*N¹*-(carboxymethyl)-3-*tert*-butyl-5-pyrazolyl-)-*N'*-(2,3-dichlorophenyl)urea (100 mg, 0.26 mmole) and *N,N'-*carbonyldiimidazole (45 mg, 0.28 mmole) in CH₂Cl₂ (10 mL) was stirred at room temp. 4 h at which time TLC indicated formation of the corresponding anhydride (TLC (50% acetone/50% CH₂Cl₂) R*_{f}* 0.81). Dry methylamine hydrochloride (28 mg, 0.41 mmole) was then added followed by of diisopropylethylamine (0.07 mL, 0.40 mmole). The reaction mixture was stirred at room temp. overnight, then diluted with CH₂Cl₂, washed with water (30 mL), a saturated NaCl solution (30 mL), dried (MgSO₄) and concentrated under reduced pressure. The residue was purified by column chromatography (gradient from 10% acetone/90% CH₂Cl₂ to 40% acetone/60% CH₂Cl₂ and the residue was crystallized (Et₂O/hexane) to afford the desired product (47 mg, 46%): TLC (60% acetone/40% CH₂Cl₂) R*_{f}* 0.59; ¹H-NMR (DMSO-d₆) δ 1.20 (s, 9H), 2.63 (d, *J*=4.5 Hz, 3H), 4.59 (s, 2H), 6.15 (s, 1H), 7.28-7.34 (m, 2H), 8.02-8.12 (m, 2H), 8.79 (br s, 1H), 9.20 (br s, 1H); FAB-MS *m*/*z* (rel abundance) 398 ((M+H)⁺, 30%).

### D5b. General Method for the Conversion of Ester-Containing Ureas into Amide-Containing Ureas

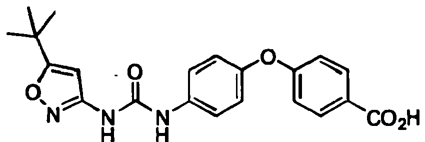

**Step 1. *N*-(5-*tert*-Butyl-3-isoxazolyl)-*N'*-(4-(4-carboxyphenyl)oxyphenyl)urea:** To a solution of *N*-(5-*tert*-butyl-3-isoxazolyl)-*N'*-(4-(4-ethoxyoxycarbonylphenyl)-oxyphenyl)urea (0.524 g, 1.24 mmol) in a mixture of EtOH (4 mL) and THF (4 mL) was added a 1M NaOH solution (2 mL) and the resulting solution was allowed to stir overnight at room temp. The resulting mixture was diluted with water (20 mL) and treated with a 3M HCl solution (20 mL) to form a white precipitate. The solids were washed with water (50 mL) and hexane (50 mL), and then dried (approximately 0.4 mmHg) to afford the desired product (0.368 g, 75 %). This material was carried to the next step without further purification. 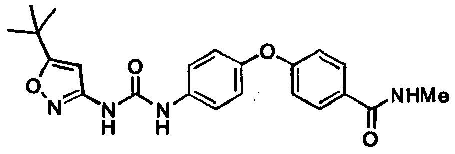 **Step 2. *N*-(5-*tert*-Butyl-3-isoxazolyl)-*N'*-(4-(4-(*N*-methylcarbamoyl)-phenyl)oxyphenyl)urea:** A solution of *N*-(5-*tert*-butyl-3-isoxazolyl)-*N'*-(4-(4-carboxyphenyl)oxyphenyl)urea (0.100 g, 0.25 mmol), methylamine (2.0 M in THF; 0.140 mL, 0.278 mmol), 1-ethyl-3-(3-dimethylaminopropyl)carbodiimide hydrochloride (76 mg, 0.39 mmol), and *N*-methylmorpholine (0.030 mL, 0.27 mmol) in a mixture of THF (3 mL) and DMF (3mL) was allowed to stir overnight at room temp. then was poured into a 1M citric acid solution (20 mL) and extracted with EtOAc (3 x 15 mL). The combined extracts were sequentially washed with water (3 x 10 mL) and a saturated NaCl solution (2 x 10 mL), dried (Na₂SO₄), filtered, and concentrated *in vacuo*. The resulting crude oil was purified by flash chromatography (60 % EtOAc/40% hexane) to afford the desired product as a white solid (42 mg, 40%): EI-MS *m*/*z* 409 ((M+H)⁺).

### D6. General Method for the Conversion of ω-Amine-Containing Ureas into Amide-Containing Ureas

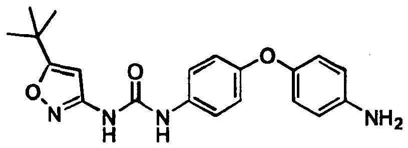

***N*-(5-*tert*-Butyl-3-isoxazolyl)-*N'*-(4-(4-aminophenyl)oxyphenyl)urea:** To a solution of *N*-(5-*tert*-butyl-3-isoxazolyl)-*N'*-(4-(4-tert-butoxycarbonylaminophenyl)oxy-phenyl)-urea (prepared in a manner analogous to Methods B6 then C2b; 0.050 g, 0.11 mmol) in anh 1,4-dioxane (3 mL) was added a conc HCl solution (1 mL) in one portion and the mixture was allowed to stir overnight at room temp. The mixture was then poured into water (10 mL) and EtOAc(10 mL) and made basic using a 1M NaOH solution (5 mL). The aqueous layer was extracted with EtOAc (3 x 10 mL). The combined organic layers were sequentially washed with water (3 x 100 mL) and a saturated NaCl solution (2 x 100 mL), dried (Na₂SO₄), and concentrated *in vacuo* to afford the desired product as a white solid (26 mg, 66%). EI-MS *mlz* 367 ((M+ H)⁺).

### D7. General Method for the Oxidation of Pyridine-Containing Ureas

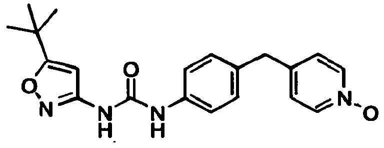

***N*-(5-*tert*-Butyl-3-isoxazolyl)-*N*'-(4-(*N*-oxo-4-pyridinyl)methylphenyl)urea:** To a solution of *N*-(5-*tert*-butyl-3-isoxazolyl)-*N'*-(4-(4-pyridinyl)methylphenyl)urea (0.100 g, 0.29 mmol) in CHCl₃ (10 mL) was added *m*-CPBA (70% pure, 0.155 g, 0.63 mmol) and the resulting solution was stirred at room temp for 16 h. The reaction mixture was then treated with a saturated K₂CO₃ solution (10 mL). After 5 min, the solution was diluted with CHCl₃ (50 mL). The organic layer was washed successively with a saturated aqueous NaHSO₃ solution (25 mL), a saturated NaHCO3 solution (25 mL) and a saturated NaCl solution (25 mL), dried (MgSO₄), and concentrated *in vacuo.* The residual solid was purified by MPLC (15% MeOH/85% EtOAc) to give the *N*-oxide (0.082 g, 79%).

### D8. General Method for the Acylation of a Hydroxy-Containing Urea

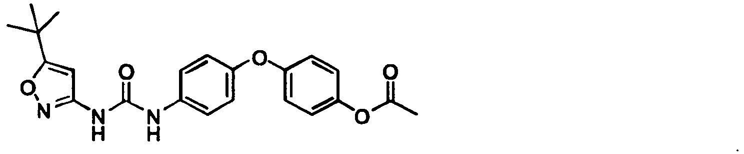

***N*-(5-*tert*-Butyl-3-isoxazolyl)-*N*'-(4-(4-acetoxyphenyloxy)phenyl)urea:** To a solution of *N*-(5-*tert*-butyl-3-isoxazolyl)-*N'*-(4-(4-hydroxyphenyloxy)phenyl)urea (0.100 g, 0.272 mmol), *N,N*-dimethylaminopyridine (0.003 g, 0.027 mmol) and Et₃N (0.075 mL, 0.544 mmol) in anh THF (5 mL) was added acetic anhydride (0.028 mL, 0.299 mmol), and the resulting mixture was stirred at room temp. for 5 h. The resulting mixture was concentrated under reduced pressure and the residue was dissolved in EtOAc (10 mL). The resulting solution was sequentially washed with a 5% citric acid solution (10 mL), a saturated NaHCO₃ solution (10 mL) and a saturated NaCl solution (10 mL), dried (Na₂SO₄), and concentrated under reduced pressure to give an oil which slowly solidified to a glass (0.104 g, 93%) on standing under reduced pressure (approximately 0.4 mmHg): TLC (40% EtOAc/60% hexane) R*_{f}* 0.55; FAB-MS *mlz* 410 ((M+H)⁺).

### D9. Synthesis of ω-Alkoxypyridines

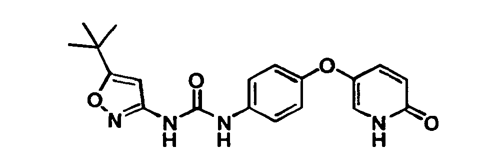

**Step 1. *N*-(5-*tert*-Butyl-3-isoxazolyl)-*N'*-(4-(2(1*H*)-pyridinon-5-yl)oxyphenyl)-urea:** A solution of *N*-(5-*tert*-butyl-3-isoxazolyl)-*N'*(4-(5-(2-methoxy)pyridyl)-oxyaniline (prepared in a manner analogous to that described in Methods B3k and C3b; 1.2 g, 3.14 mmol) and trimethylsilyl iodide (0.89 mL, 6.28 mmol) in CH₂Cl₂ (30 mL) was allowed to stir overnight at room temp., then was to 40°C for 2 h. The resulting mixture was concentrated under reduced pressure and the residue was purified by column chromatography (gradient from 80% EtOAc/20% hexans to 15% MeOH/85% EtOAc) to give the desired product (0.87 g, 75%): mp 175-180 °C; TLC (80% EtOAc/20% hexane) R*_{f}* 0.05; FAB-MS *m*/*z* 369 ((M+H)⁺, 100%). 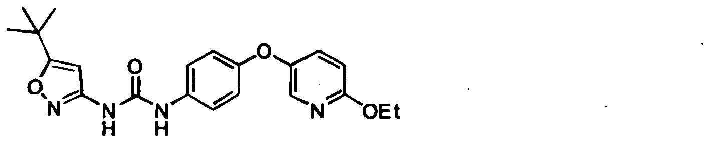 **Step 2. *N*-(5-*tert*-Butyl-3-isoxazolyl)-*N'*-(4-(5-(2-Ethoxy)pyridyl)oxyphenyl)urea:** A slurry of *N*-(5-*tert*-butyl-3-isoxazolyl)-*N'*-(4-(2(1*H*)-pyridinon-5-yl)oxyphenyl)urea (0.1 g, 0.27 mmol) and Ag₂CO₃ (0.05 g, 0.18 mmol) in benzene (3 mL) was stirred at room temp. for 10 min. lodoethane (0.023 mL, 0.285 mmol) was added and the resulting mixture was heated at the reflux temp. in dark overnight. The reaction mixture was allowed to cool to room temp., and was filtered through a plug of Celite^{®} then concentrated under reduced pressure. The residue was purified by column chromatography (gradient from 25% EtOAc/75% hexane to 40% EtOAc/60% hexane) to afford the desired product (0.041 g, 38%): mp 146 °C; TLC (40% EtOAc/60% hexane) R*_{f}* 0.49; FAB-MS *m*/*z* 397 ((M+H)⁺, 100%).

### D10. Reduction of an Aldehyde- or Ketone-Containing Urea to a Hydroxide-Containing Urea

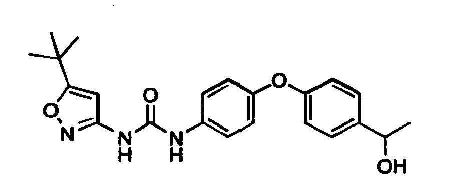

***N*-(5-*tert*-Butyl-3-isoxazolyl)-*N'*-(4-(4-(1-hydroxyethyl)phenyl)oxyphenyl)urea:** To a solution of *N*-(5-*tert*-butyl-3-isoxazolyl)-*N'*-(4-(4-(1-acetylphenyl)oxyphenyl)urea (prepared in a manner analogous to that described in Methods B1 and C2b; 0.060 g, 0.15 mmol) in MeOH (10 mL) was added NaBH₄ (0.008 g, 0.21 mmol) in one portion. The mixture was allowed to stir for 2 h at room temp., then was concentrated *in vacuo.* Water (20 mL) and a 3M HCl solution (2 mL) were added and the resulting mixture was extracted with EtOAc (3 x 20 mL). The combined organic layers were washed with water (3 x 10 mL) and a saturated NaCl solution (2 x 10 mL), dried (MgSO₄), and concentrated *in vacuo*. The resulting white solid was purified by trituration (Et₂O/hexane) to afford the desired product (0.021 g, 32 %): mp 80-85 °C; ¹H NMR (DMSO-d₆) δ 1.26 (s, 9H), 2.50 (s, 3H), 4.67 (m, 1H), 5.10 (br s, 1H), 6.45 (s, 1H), 6.90 (m, 4H), 7.29 (d, *J*=9.0 Hz, 2H), 7.42 (d, *J*=9.0 Hz, 2H), 8.76 (s, 1H), 9.44 (s, 1H); HPLC ES-MS *m*/*z* 396 ((M+H)⁺).

### D11. Synthesis of Nitrogen-Substituted Ureas by Curtius Rearrangement of Carboxy-Substituted Ureas

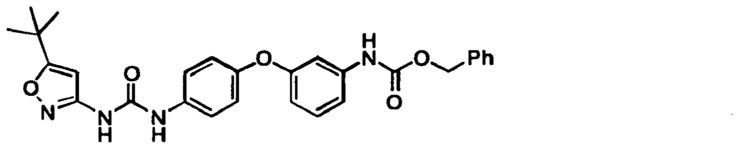

***N*-(5-*tert*-Butyl-3-isoxazolyl)-*N'*-(4-(3-(benzyloxycarbonylamino)phenyl)-oxyphenyl)urea:** To a solution of the *N*-(5-*tert*-butyl-3-isoxazolyl)-*N'*-(4-(3-carboxyphenyl)oxyphenyl)urea (prepared in a manner analogous to that described in Methods B3a, Step 2 and C2b; 1.0 g, 2.5 mmol) in anh toluene (20 mL) was added Et₃N, (0.395 mL, 2.8 mmol) and DPPA (0.610 mL, 2.8 mmol). The mixture was heated at 80°C with stirring for 1.5 h then allowed to cool to room temp. Benzyl alcohol (0.370 mL, 3.5 mmol) was added and the mixture was heated at 80°C with stirring for 3 h then allowed to cool to room temp. The resulting mixture was poured into a 10% HCl solution (50 mL) and teh resulting solution extracted with EtOAc (3 x 50 mL). The combined organic layers were washed with water (3 x 50 mL) and a saturated NaCl (2 x 50 mL), dried (Na₂SO₄), and concentrated *in vacuo.* The crude oil was purified by column chromatography (30% EtOAc/70% hexane) to afford the desired product as a white solid (0.7 g, 60 %): mp 73-75 °C; ¹H NMR (DMSO-d₆) δ 1.26 (s, 9H), 5.10 (s, 2H), 6.46 (s, 1H), 6.55 (d, *J*=7.0 Hz, 1H), 6.94 (d, *J*=7.0 Hz, 2H), 7.70 (m, 7H), 8.78 (s, 1H), 9.46 (s, 1H), 9.81 (s, 1H); HPLC ES-MS *mlz* 501 ((M +H)⁺).

The following compounds have been synthesized according to the General Methods listed above:

**Table 3. N¹-Substituted-3-tert-butyl-5-pyrazolyl Ureas**

| | | | | | | | | |
|---|---|---|---|---|---|---|---|---|
| 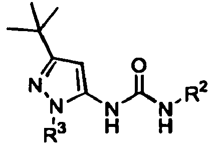 | | | | | | | | |

| Ex. | R¹ | R² | mp (°C) | TLC R*_{f}* | Solvent System | Mass Spec. | Source | Synth. Method |
|---|---|---|---|---|---|---|---|---|
| 212 | H | 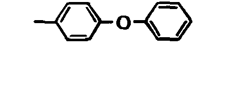 | | 0.27 | 50% EtOAc/ 50% hexane | 351 (M+H)+ | FAB | C1c |
| 213 | H | 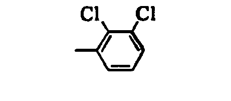 | | 0.59 | 50% EtOAc/ 50% hexane | 327 (M+H)+ | FAB | C1c |
| 214 | H | 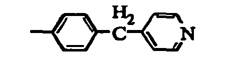 | | 0.30 | 60% acetone / 40% CH2Cl2 | 350 (M+H)+ | FAB | C4a |
| 215 | H | 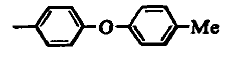 | 204 | 0.06 | 5% acetone / 95% CH2Cl2 | 364 (M+) | EI | C3b |
| 216 | H | 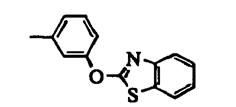 | 110-111 | 0.05 | 5% acetone /95% CH2Cl2 | 408 (M+H+) | FAB | C3b |
| 217 | H | 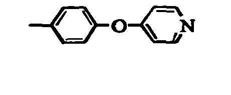 | 228-232 dec | 0.24 | 10% MeOH/ 90% CHCl3 | 351 (M+) | EI | C3a |
| 218 | H | 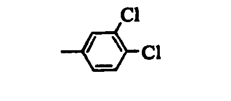 | 182-184 | 0.05 | 40% EtOAc/ 60% hexane | 327 (M+H)+ | FAB | A5, C1e |
| 219 | H | 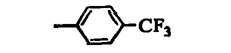 | 110-112 | | | 326 (M+) | EI | A5, C1e |
| 220 | H | 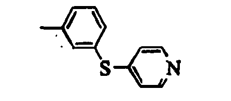 | | 0.07 | 5% MeOH/ 95% CHCl3 | 368 (M+H)+ | FAB | B4a, C4a |
| 221 | H | 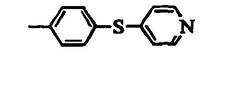 | | 0.18 | 5% MeOH/ 95% CHCl3 | 364 (M+) | EI | B4a, C4a |
| 222 | H | 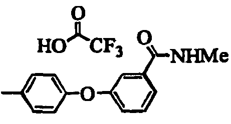 | 160-161 | | | 408 (M+H)+ | FAB | A5, B6, C3b isolated at TFA salt |
| 223 | H | 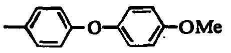 | 181-183 | | | 381 (M+H)+ | FAB | C2b |
| 224 | Me | 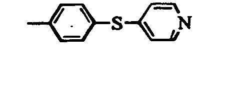 | | 0.35 | 70% acetone /30% CH2Cl2 | 382 (M+H)+ | FAB | B4a, C4a |
| 225 | Me | 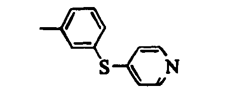 | | 0.46 | 70% acetone / 30% CH2Cl2 | 382 (M+H)+ | FAB | C4a, B4a |
| 226 | Me | 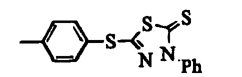 | | 0.47 | 100% EtOAc | 497 (M+H)+ | FAB | B3c, C4a |
| 227 | Me | 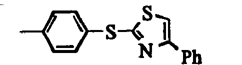 | | 0.46 | 100% EtOAc | 464 (M+H)+ | FAB | B3c, C4a |
| 228 | Me | 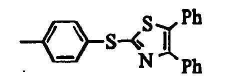 | | 0.50 | 100% EtOAc | 540 (M+H)+ | FAB | B3c, C4a |
| 229 | Me | 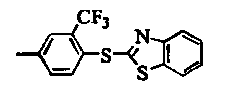 | | 0.52 | 100% EtOAc | 506 (M+H)+ | FAB | B3c, C4a |
| 230 | Me | 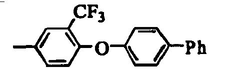 | | 0.51 | 100% EtOAc | 509 (M+H)+ | FAB | B3c, C4a |
| 231 | Me | 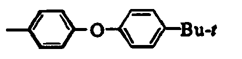 | | 0.75 | 100% EtOAc | 421 (M+H)+ | FAB | B3c, C4a |
| 232 | Me | 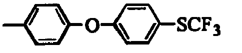 | | 0.50 | 100% EtOAc | 465 (M+H)+ | FAB | B3c, C4a |
| 233 | Me | 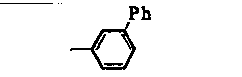 | | 0.50 | 100% EtOAc | 349 (M+H)+ | FAB | C4a |
| 234 | Me | 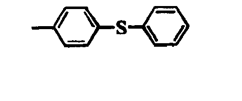 | | 0.09 | 50% EtOAc/ 50% hexane | 381 (M+H)+ | FAB | C4a |
| 235 | Me | 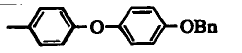 | | 0.60 | 100% EtOAc | 471 (M+H)+ | FAB | B2, C4a |
| 236 | Me | 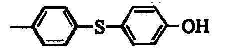 | | 0.61 | 100% EtOAc | 397 (M+H)+ | FAB | B3c, C4a |
| 237 | Me | 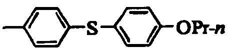 | | 0.42 | 100% EtOAc | 439 (M+H)+ | FAB | B5, C4a |
| 238 | Me | 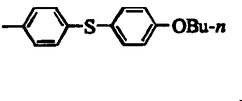 | | 0.25 | 50% EtOAc/ 50% hexane | 453 (M+H)+ | FAB | B5, C4a |
| 239 | Me | 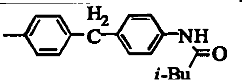 | | 0.65 | 100% EtOAc | 462 (M+H)+ | FAB | B6, C4a |
| 240 | Me | 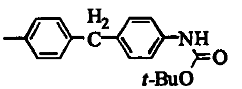 | | 0.67 | 100% EtOAc | 478 (M+H)+ | FAB | B6, C4a |
| 241 | Me | 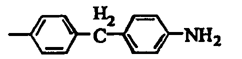 | | 0.50 | 100% EtOAc | 378 (M+H)+ | FAB | C4a |
| 242 | Me | 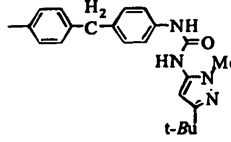 | | 0.30 | 100% EtOAc | 557 (M+H)+ | FAB | C4a |
| 243 | Me | 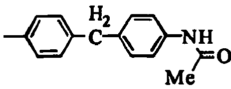 | | 0.33 | 100% EtOAc | 420 (M+H)+ | FAB | C4a, D3 |
| 244 | Me | 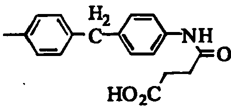 | | 0.60 | 10% water/ 90% CH3CN | 478 (M+H)+ | FAB | C4a, D3 |
| 245 | Me | 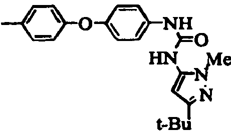 | | 0.28 | 100% EtOAc | 559 (M+H)+ | FAB | C4a |
| 246 | Me | 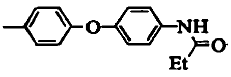 | | 0.40 | 100% EtOAc | 436 (M+H)+ | FAB | C4a, D3 |
| 247 | Me | 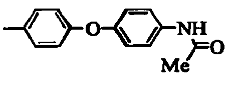 | | 0.46 | 50% acetone / 50% CH2Cl2 | 422 (M+H)+ | FAB | C4a, D3 |
| 248 | Me | 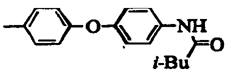 | | 0.50 | 100% EtOAc | 464 (M+H)+ | FAB | C4a, D3 |
| 249 | Me | 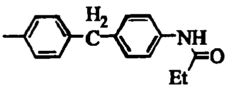 | | 0.55 | 100% EtOAc | 434 (M+H)+ | FAB | C4a, D3 |
| 250 | Me | 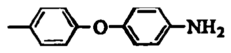 | | 0.52 | 100% EtOAc | 380 (M+H)+ | FAB | C4a |
| 251 | Me | 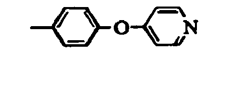 | | 0.25 | 60% acetone /40% CH2Cl2 | 366 (M+H)+ | FAB | C4a |
| 252 | Me | 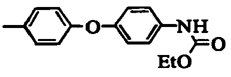 | | 0.52 | 100% EtOAc | 452 (M+H)+ | FAB | C4a, D3 |
| 253 | Me | 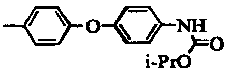 | | 0.52 | 100% EtOAc | 466 (M+H)+ | FAB | C4a, D3 |
| 256 | Me | 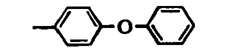 | 147-149 | | | 365 (M+H)+ | FAB | C1c |
| 257 | Me | 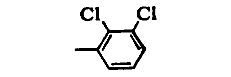 | 173-175 | | | 341 (M+H)+ | FAB | C1c |
| 258 | Me | 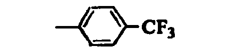 | 185-187 | | | 341 (M+H)+ | HPLC/ ES-MS | C1c |
| 259 | Me | 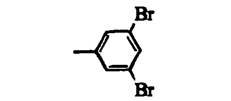 | 195-197 | | | 429 (M+H)+ | FAB | C1c |
| 260 | Me | 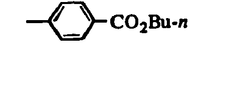 | | 0.25 | 50% EtOAc/ 50% hexane | 373 (M+H)+ | FAB | C1c |
| 261 | Me | 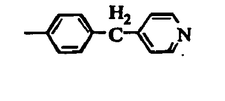 | 161-162 | 0.15 | 4% MeOH/ 96% CH2Cl2 | 364 (M+H)+ | FAB | C2b |
| 262 | Me | 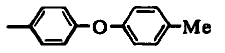 | 228 dec | | | 379 (M+H)+ | FAB | C2b |
| 263 | Me | 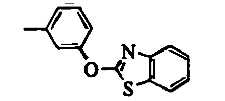 | | 0.30 | 5% MeOH/ 95% CH2Cl2 | 422 (M+H)+ | FAB | C2b |
| 264 | Me | 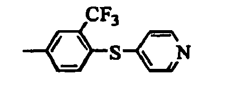 | | 0.32 | 70% acetone / 30% CH2Cl2 | 450 (M+H)+ | FAB | B3b, C4a |
| 265 | Me | 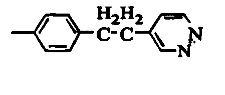 | | 0.15 | 40% acetone /60% CH2Cl2 | 379 (M+H)+ | FAB | B1, B2, C3a |
| 268 | Me | 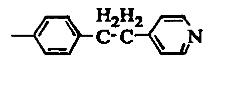 | | 0.48 | 30% acetone / 70% CH2Cl2 | 378 (M+H)+ | FAB | B1, C3a |
| 269 | -CH₂CF₃ | 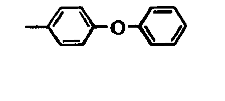 | | 0.22 | 30% EtOAc/ 70% hexane | 433 (M+H)+ | FAB | A3, C1b |
| 270 | -CH₂CF₃ | 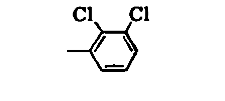 | | 0.38 | 30% EtOAc. / 70% hexane | 409 (M+H)+ | FAB | A3, C1b |
| 271 | -(CH₂)₂CN | 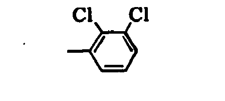 | | 0.53 | 70% EtOAc/ 30% hexane | 380 (M+) | HPLC/ ES-MS | A3, C1b |
| 272 | -(CH₂)₂CN | 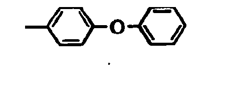 | | 0.37 | 70% EtOAc/ 30% hexane | 404 (M+H)+ | HPLC/ ES-MS | A3, C1b |
| 273 | -(CH₂)₂OH | 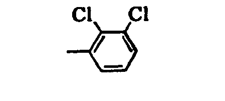 | | 0.15 | 60% EtOAc/ 40% hexane | 371 (M+H)+ | FAB | A3, C1b, D4 |
| 274 | 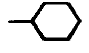 | 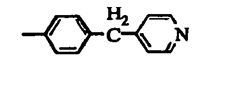 | | 0.49 | 40% acetone /60% CH2Cl2 | 432 (M+H)+ | FAB | A3, C4a |
| 275 | -CH₂CO₂Et | 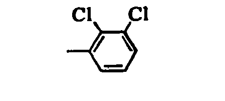 | | 0.44 | 50% EtOAc/ 50% hexane | 413 (M+H)+ | FAB | A3, C1b |
| 276 | NHMe | 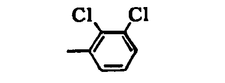 | | 0.59 | 60% acetone / 40% CH2Cl2 | 398 (M+H)+ | FAB | A3, C1b, D5a |
| 277 | 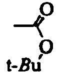 | 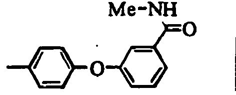 | 159-161 | | | 508 (M+H)+ | FAB | A5, B6, C2b |

### BIOLOGICAL EXAMPLES

### P38 Kinase Assay:

The *in vitro* inhibitory properties of compounds were determined using a p38 kinase inhibition assay. P38 activity was detected using an *in vitro* kinase assay run in 96-well microtiter plates. Recombinant human p38 (0.5 µg/mL) was mixed with substrate (myelin basic protein, 5 µg/mL) in kinase buffer (25 mM Hepes, 20 mM MgCl₂ and 150 mM NaCl) and compound. One µCi/well of ³³P-labeled ATP (10 µM) was added to a final volume of 100 µL. The reaction was run at 32°C for 30 min. and stopped with a 1M HCl solution. The amount of radioactivity incorporated into the substrate was determined by trapping the labeled substrate onto negatively charged glass fiber filter paper using a 1% phosphoric acid solution and read with a scintillation counter. Negative controls include substrate plus ATP alone.

All compounds exemplified displayed p38 IC₅₀s of between 1 nM and 10 µM.

### LPS Induced TNF Production in Mice:

The *in vivo* inhibitory properties of selected compounds were determined using a murine LPS induced TNFa production *in vivo* model. BALB/c mice (Charles River Breeding Laboratories; Kingston, NY) in groups of ten were treated with either vehicle or compound by the route noted. After one hour, endotoxin (E. coli lipopolysaccharide (LPS) 100 µg was administered intraperitoneally (i.p.). After 90 min, animals were euthanized by carbon dioxide asphyxiation and plasma was obtained from individual animals by cardiac puncture ionto heparinized tubes. The samples were clarified by centrifugation at 12,500 x g for 5 min at 4 °C. The supernatants were decanted to new tubes, which were stored as needed at -20 °C. TNFα levels in sera were measured using a commercial murine TNF ELISA kit (Genzyme).

The preceeding examples can be repeated with similar success by substituting the generically of specifically described reactants and/or operating conditions of this invention for those used in the preceeding examples

## Claims

1. Use of a compound of formula I 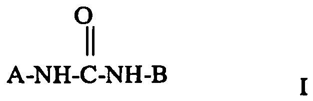 wherein B is selected from the group consisting of 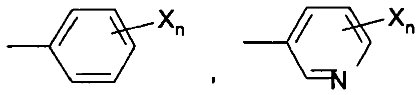 which is unsubstituted or substituted by halogen, up to per-halosubstitution, and
wherein n = 0-3 and each X is independently selected from the group consisting of -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀ alkyl, C₂-₁₀-alkenyl, C₁₋₁₀-alkoxy, C₃-C₁₀ cycloalkyl, C₆-C₁₄ aryl, C₇-C₂₄ alkaryl, C₃-C₁₃ heteroaryl, C₄-C₂₃ alkheteroaryl, and substituted C₁-C₁₀ alkyl, substituted C₂₋₁₀-alkenyl, substituted C₁₋₁₀-alkoxy, substituted C₃-C₁₀ cycloalkyl, substituted C₄-C₂₃ alkheteroaryl and -Y-Ar;
wherein if X is a substituted group, it is substituted by one or more substituents independently selected from the group consisting of -CN, -CO₂R⁵, -C(O)R⁵, -C(O)NR⁵R^{5'}, -OR⁵, -SR⁵, -NR⁵R^{5'}, NO₂, -NR⁵C(O)R^{5'}, -NR⁵C(O)OR^{5'} and halogen up to per-halosubstitution;
wherein R⁵ and R^{5'} are independently selected from H, C₁-C₁₀ alkyl, C₂-₁₀-alkenyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstituted C₂₋₁₀-alkenyl, up to per-halosubstituted C₃-C₁₀ cycloalkyl, up to per-halosubstituted C₆-C₁₄ aryl and up to per-halosubstituted C₃-C₁₃ heteroaryl,
wherein Y is - O-, or -S-,
and
Ar is a 5-10 member aromatic structure containing 0-4 members of the group consisting of nitrogen, oxygen and sulfur which is unsubstituted or substituted by halogen up to per-halo and optionally substituted by Zₙ₁, wherein nl is 0 to 3 and each Z is independently selected from the group consisting of -CN, -CO₂R⁵, -C(O)R⁵, =O, -SO₂R⁵, -SO₂NR⁵R^{5'}, C(O)NR⁵R^{5'}, -C(O)R⁵, -NO_{2,} -OR⁵, -SR⁵, -NR⁵R^{5'}, -NR⁵C(O)OR^{5'}, -NR⁵C(O)R^{5'}, C₁-C₁₀ alkyl, C₁-C₁₀ alkoxy, C₃-C₁₀ cycloalkyl, C₆-C₁₄ aryl, C₃-C₁₃ heteroaryl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl; wherein if Z is a substituted group, it is substituted by one or more substituents independently selected from the group consisting of -CN, -CO₂R⁵, -C(O)NR⁵R⁵', =O, -OR⁵, -SR⁵, -NO₂, -NR⁵R^{5'}, -NR⁵C(O)R^{5'}, -NR⁵C(O)OR^{5'}, C₁-C₁₀ alkyl, C₁-C₁₀ alkoxy, C₁-C₁₀ cycloalkyl, C₃-C₁₀ heteroaryl, C₆-C₁₄ aryl, C₄-C₂₄ alkheteroaryl and C₇₋C₂₄ alkaryl, and
A is 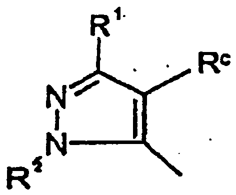 wherein
R¹ is selected from the group consisting of halogen, C₃-C₁₀ alkyl, C₃-C₁₀ cycloalkyl, C₁-C₁₃ heteroaryl, C₆-C₁₄ aryl, C₇-C₂₄ alkaryl, up to per-halosubstituted C₁-C₁₀ alkyl, up to per-halosubstituted C₃-C₁₀ cycloalkyl, up to per-halosubstituted C₁-C₁₃ heteroaryl, up to per-halosubstituted C₆-₁₄ aryl, and up to per-halosubstituted C₇-₂₄ alkaryl;
R² is selected from the group consisting of H, -C(O)R⁴, -CO₂R⁴, -C(O)NR³R^{3'}, C₁-C₁₀ alkyl, C₃-C₁₀ cycloalkyl, C₇-C₂₄ alkaryl, C₄-C₂₃, alkheteroaryl, substituted C₁-C₁₀ alkyl, substituted C₃-C₁₀ cycloalkyl, substituted C₇-C₂₄ alkaryl and substituted C₄-C₂₃ alkheteroaryl,
where R² is a substituted group, it is substituted by one or more substituents independently selected from the group consisting of -CN, - CO₂R⁴, -C(O)-NR³R^{3'}, -NO₂, -OR⁴, -SR⁴, and halogen up to per-halosubstitution,
wherein R³ and R^{3'} are independently selected from the group consisting of H, -OR⁴, -SR⁴, -NR⁴R^{4'}, -C(O)R⁴, -CO₂R⁴, -C(O)NR⁴R^{4'}, C₁-C₁₀ alkyl, C₃-C₁₀ cycloalkyl, C₆-C₁₄ aryl, C₃-C₁₃ heteroaryl, C₇-C₂₄ alkaryl, C₄-C₂₃ alkheteroaryl, up to per-halosubstituted C₁-C₁₀ alkyl, up to per-halosubstitutedC₃-C₁₀ cycloalkyl, up to per-halosubstituted C₆-C₁₄ aryl and up to per-halosubstituted C₃-C₁₃ heteroaryl; and
wherein R⁴ and R^{4'} are independently selected from the group consisting of H, C₁-C₁₀ alkyl, C₃-C₁₀ cycloalkyl, C₆-C₁₄ aryl, C₃-C₁₃ heteroaryl; C₇-C₂₄ alkaryl, C₄-C₂₃ alkheteroaryl, up to per-halosubstituted C₁-C₁₀ alkyl, up to per-halosubstituted C₃-C₁₀ cycloalkyl, up to per-halosubstituted C₆-C₁₄ aryl and up to per-halosubstituted C₃-C₁₃ heteroaryl,
and
R^{c} is hydrogen, halogen, C₁-C₁₀ alkyl, up to per-halosubstituted C₁-C₁₀ alkyl or combines with R¹ and the ring carbon atoms to which R¹ and R^{c} are bound to form a 5- or 6-membered cycloalkyl, aryl or heteroaryl ring with 0-2 members selected from O, N and S, and pharmaceutically acceptable salts thereof for the preparation of a medicament for the treatment of a disease mediated by p38 other than cancer, wherein the disease is an inflammatory or immunomodulatory disease.

2. The use of claim 1, wherein B is 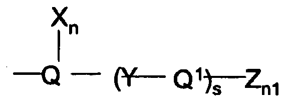 wherein Y is selected from the group consisting of -O-, or -S-,
Q is phenyl or 3-pyridinyl, substituted or unsubstituted by halogen, up to perhalosubstitution;
Q¹ is a mono- or bicyclic aromatic structure of 5 to 10 carbon atoms and 0-4 members of the group consisting of N, O and S, unsubstituted or unsubstituted by halogen up to per-halosubstitution, and
X, Z, n and n1 are as defined in claim 1 and s is 0 or 1.

3. The use of claim 2, wherein
Q is phenyl or 3-pyridinyl, substituted or unsubstituted by halogen, up to perhalosubstitution,
Q¹ is selected from the group consisting of phenyl, pyridinyl, naphthyl, pyrimidinyl, quinoline, isoquinoline, imidazole and benzothiazolyl, substituted or unsubstituted by halogen, up to per-halo substitution, or -Y-Q¹ is phthalimidinyl substituted or unsubstituted by halogen up to per-halo substitution, and
Z and X are independently selected from the group consisting of -R⁶, -OR⁶ and -NHR⁷, wherein R⁶ is hydrogen, C₁-C₁₀-alkyl or C₃-C₁₀-cycloalkyl and R⁷ is selected from the group consisting of hydrogen, C₃-C₁₀-akyl, C₃-C₆-cycloalkyl and C₆-C₁₀-aryl, wherein R⁶ and R⁷ can be substituted by halogen or up to perhalosubstitution.

4. The use of claim 1, wherein the compound of formula I is a compound of the formula 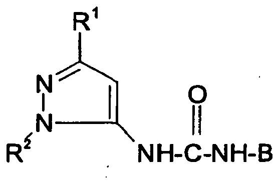 wherein R¹ and R² and B are as defined in claim 1.

5. The use of claim 4, wherein B is 2,3-dichlorophenyl or of the formula 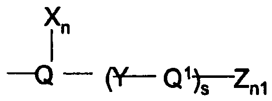 wherein Q is phenyl, Q¹ is phenyl or pyridinyl, Y is -0-, or -S-, X is CF₃, and Z is -OH, -Cl or NHC(O)-CₚH₂ₚ₊₁, where p = 2-4, s = 0 or 1, n = 0 and n1 = 0 or 1.

6. The use of claim 1, wherein the compound of formula I is a compound selected from the group consisting of:
*N*-(3-*tert*-Butyl-5-pyrazolyl)-*N*'-(4-(2,3-dichlorophenyl)urea;
*N*-(3-*tert*-Butyl-5-pyrazolyl)-*N*'-(3-(4-pyridinyl)thiophenyl)urea;
*N*-(3-*tert*-Butyl-5-pyrazolyl)-*N*'-(4-(4-pyridinyl)methylphenyl)urea;
*N*-(3-*tert*-Butyl-5-pyrazolyl)-*N*'-(4-(4-pyridinyl)oxyphenyl)urea;
*N*-(3-*tert*-Butyl-5-pyrazolyl)-*N*'-(4-(4-pyridinyl)thiophenyl)urea;
*N*-(3-*tert*-Butyl-5-pyrazolyl)-*N*'-(4-(4-pyridinyl)methylphenyl)urea;
*N*-(1-Methyl-3-*tert*-butyl-5-pyrazolyl)-*N*'-(2,3-dichlorophenyl)urea;
*N*-(1-Methyl-3-*tert*-butyl-5-pyrazolyl)-*N*'-(4-(4-hydroxyphenyl)thiophenyl)urea;
*N*-(1-Methyl-3-*tert*-butyl-5-pyrazolyl)-*N*'-(4-(4-ethylaminocarbonylphenyl)oxyphenyl)urea;
*N*-(1-Methyl-3-*tert*-butyl-5-pyrazolyl)-*N*'-(4-(4-isobutylaminocarbonylphenyl)thiophenyl)urea;
*N*-(1-Methyl-3-*tert*-butyl-5-pyrazolyl)-*N*'-(4-(4-pyridinyl)thiophenyl)urea;
*N*-(1-Methyl-3-*tert*-butyl-5-pyrazolyl)-*N*'-(3-(4-pyridinyl)thiophenyl)urea;
*N*-(1-Methyl-3-*tert*-butyl-5-pyrazolyl)-*N*'-(4-(4-pyridinyl)thio-3-(trifluoromethyl)phenyl)urea;
*N*-(1-Methyl-3-*tert*-butyl-5-pyrazolyl)-*N*'-(4-(4-pyridinyl)oxyphenyl)urea;
*N*-(1-Methyl-3-*tert*-butyl-5-pyrazolyl)-*N*'-(4-((4-pyridinyl)methylthio)-phenyl)urea;
*N*-(1-(2,2,2-Trifluoroethyl)-3-*tert*-butyl-5-pyrazolyl)-*N*'-(2,3-dichlorophenyl)urea;
*N*-(1-(2-Hydroxyethyl)-3-*tert*-butyl-5-pyrazolyl)-*N'*-(2,3-dichlorophenyl)urea;
*N*-(1-Ethoxycarbonylmethyl-3-*tert*-butyl-5-pyrazolyl)-*N*'-(2,3-dichlorophenyl)urea;
*N*-(1-(2-Cyanoethyl)-3-*tert*-butyl-5-pyrazolyl)-*N*'-(2,3-dichlorophenyl)urea;
*N*-(1-(3-Hydroxyphenyl)methyl-3-*tert*-butyl-5-pyrazolyl)-*N*'(2,3-dichlorophenyl)urea;
*N*-(1-Cyclohexyl-3-*tert*-butyl-5-pyrazolyl)-*N*'-(4-(4-pyridinyl)methyl-phenyl)urea;
*N*-(1-methyl-3-phenyl-5-pyrazolyl)-*N*'-(3-(4-(2-methylcarbamoyl)-pyridyl)thiophenyl) urea;
*N*-(1-methyl-3-*tert*-butyl-5-pyrazolyl)-*N*'-(4-(4-pyridyl)thiophenyl) urea;
*N*-(1-methyl-3-*tert*-butyl-5-pyrazolyl)-*N*'-(3-(4-pyridyl)thiophenyl) urea;
*N*-(1-methyl-3-*tert*-butyl-5-pyrazolyl)-*N*'-(3-trifluoromethyl-4-(4-pyridylthio)phenyl) urea;
*N*-(3-*tert*-butyl-5-pyrazolyl)-*N*'-(3-(4-pyridyl)oxyphenyl) urea;
*N*-(3-*tert*-butyl-5-pyrazolyl)-*N*'-(4-(4-pyridyl)oxyphenyl) urea;
and pharmaceutically acceptable salts thereof.

7. The use of claim 4, wherein R¹ is t-butyl.

8. The use of claim 1, wherein the compound for formula I displays p38 activity (IC₅₀) better than 10 µm as determined by an in-vitro kinase assay.

9. The use of claim 1, wherein the disease is mediated by a cytokine or protease regulated by p38.

10. The use of claim 1, wherein the amount of the compound of formula I is effective to inhibit p38.

11. The use of claim 1, wherein the amount of the compound of formula I is effective to inhibit production of a disease-mediating cytokine or protease.

12. The use of claim 1, wherein the disease is mediated by TNFα , MMP-1, MMP-3, IL-1, IL-6 or IL-8.

13. The use of claim 1, wherein the disease is an inflammatory disease.

14. The use of claim 1, wherein the disease is an immunomodulatory disease.

15. The use of claim 1, wherein the disease is rheumatoid arthritis, osteoporosis, osteoarthritis, asthma, septic shock, inflammatory bowel disease, or the result of host-versus-graft reactions.

16. A compound of one of the formulae
d) 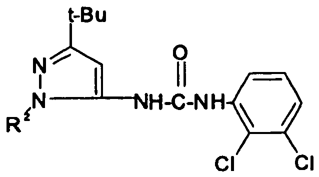 wherein R² is -CH₂CF₃, -C₂H₄ -OH, -CH₂-(3-HOC₆H₄), -CH₂C(O)NHCH₃, -CH₂C(O)OC₂H₅, -C₂H₄CN, or 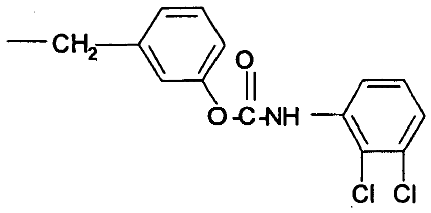 or
e) 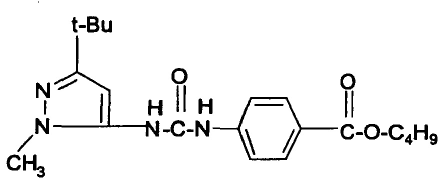 and pharmaceutically acceptable salts thereof.

17. A pharmaceutical composition comprising a compound according to claim 16 or a pharmaceutically acceptable salt thereof and a physiologically acceptable carrier.

## Patentansprüche

1. Verwendung einer Verbindung der Formel I 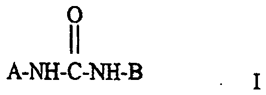 wobei B ausgewählt wird aus der Gruppe bestehend aus 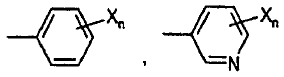 welches unsubstituiert oder unsubstituiert ist mit Halogen, bis zur Perhalosubstitution, und wobei n = 0-3 und jedes X unabhängig ausgewählt ist aus der Gruppe bestehend aus -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀-Alkyl, C₂-₁₀-Alkenyl, C₁-₁₀-Alkoxy, C₃-C₁₀-Cycloalkyl, C₆-C₁₄-Aryl, C₇-C₂₄-Alkaryl, C₃-C₁₃-Heteroaryl, C₄-C₂₃-Alkheteroaryl und substituiertem C₁-C₁₀-Alkyl, substituiertem C₂-₁₀-Alkenyl, substituiertem C₁-10-Alkoxy, substituiertem C₃-C₁₀-Cycloalkyl, substituiertem C₄-C₂₃-Alkheteroaryl und -Y-Ar;
wobei falls X eine substituierte Gruppe ist, es mit einem oder mehreren Substituenten substituiert ist, unabhängig ausgewählt aus der Gruppe bestehend aus -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} und Halogen bis zur Perhalosubstitution;
wobei R⁵ und R^{5'} unabhängig ausgewählt sind aus H, C₁-C₁₀₋Alkyl, C₂-₁₀-Alkenyl, C₃-C₁₀-Cycloalkyl, C₆-C₁₄-Aryl, C₃-C₁₃-Heteroaryl, C₇-C₂₄-Alkaryl, C₄-C₂₃-Alkheteroaryl, bis zu perhalosubstituiertem C₁-C₁₀-Alkyl, bis zu perhalosubstituiertem C₂-₁₀-Alkenyl, bis zu perhalosubstituiertem C₃-C₁₀-Cycloalkyl, bis zu perhalosubstituiertem C₆-C₁₄-Aryl und bis zu perhalosubstituiertem C₃-C₁₃-Heteroaryl,
wobei Y -O-, oder -S- ist, und
Ar eine 5-10-gliedrige aromatische Struktur ist, die 0-4 Mitglieder aus der Gruppe bestehend aus Stickstoff, Sauerstoff und Schwefel enthält, welche unsubstituiert oder substituiert mit Halogen bis zu Perhalogen ist und optional substituiert mit Zₙ₁, wobei n1 0 bis 3 ist und jedes Z unabhängig ausgewählt ist aus der Gruppe bestehend aus -CN, -CO₂R⁵, -C(O)R⁵, =O, -SO₂R⁵, -SO₂NR⁵R^{5'}, -C(O)NR⁵R^{5'}, -C(O)R⁵, -NO₂ -OR⁵, -SR⁵, -NR⁵R^{5'}, -NR⁵C(O)OR^{5'}, -NR⁵C(O)R^{5'}, C₁-C₁₀-Alkyl, C₁-C₁₀-Alkoxy, C₃-C₁₀-Cycloalkyl, C₆-C₁₄-Aryl, C₃-C₁₃-Heteroaryl, C₇-C₂₄-Alkaryl, C₄-C₂₃-Alkheteroaryl, substituiertem C₁-C₁₀-Alkyl, substituiertem C₃-C₁₀-Cycloalkyl, substituiertem C₇-C₂₄-Alkaryl und substituiertem C₄-C₂₃-Alkheteroaryl; wobei, falls Z eine substituierte Gruppe ist, es mit einem oder mehreren Substituenten substituiert ist, unabhängig ausgewählt aus der Gruppe bestehend aus -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵C(O)OR^{5'}, C₁-C₁₀-Alkyl, C₁-C₁₀-Alkoxy, C₃-C₁₀-Cycloalkyl, C₃-C₁₀-Heteroaryl, C₆-C₁₄-Aryl, C₄-C₂₄-Alkheteroaryl und C₇-C₂₄-Alkaryl und
A 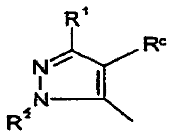 ist, wobei
R¹ ausgewählt ist von der Gruppe bestehend aus Halogen, C₃-C₁₀-Alkyl, C₃-C₁₀-Cycloalkyl, C₁-C₁₃-Heteroaryl, C₆-₁₄-Aryl, C₇₋₂₄-Alkaryl, bis zu perhalosubstituiertem C₁-C₁₀-Alkyl, bis zu perhalosubstituiertem C₃-C₁₀-Cycloalkyl, bis zu perhalosubstituiertem C₁-C₁₃-Heteroaryl, bis zu perhalosubstituiertem C₆-C₁₄-Aryl und bis zu perhalosubstituiertem C₇-₂₄-Alkaryl;
R² ausgewählt ist aus einer Gruppe bestehend aus H, -C(O)R⁴, -CO₂R⁴, -C(O)NR³R^{3'}, C₁-C₁₀-Alkyl, C₃-C₁₀-Cycloalkyl, C₇-C₂₄-Alkaryl, C₄-C₂₃-Alkheteroaryl, substituiertem C₁-C₁₀-Alkyl, substituiertem C₃-C₁₀-Cycloalkyl, substituiertem C₇-C₂₄-Alkaryl und substituiertem C₄-C₂₃-Alkheteroaryl,
wo R² eine substituierte Gruppe ist, ist es mit einem oder mehreren Substituenten substituiert, unabhängig ausgewählt aus der Gruppe bestehend aus -CN, -CO₂R⁴, -C(O)-NR³R^{3'}, -NO₂, -OR⁴, -SR⁴, und Halogen bis zur Perhalosubstitution,
wobei R³ und R^{3'} unabhängig ausgewählt sind aus der Gruppe bestehend aus H, -OR⁴, -SR⁴, -NR⁴R^{4'}, -C(O)R⁴, -CO₂R⁴, -C(O)NR⁴R^{4'}, C₁-C₁₀-Alkyl, C₃-C₁₀-Cycloalkyl, C₆-C₁₄-Aryl, C₃-C₁₃-Heteroaryl, C₇-C₂₄-Alkaryl, C₄-C₂₃-Alkheteroaryl, bis zu perhalosubstituiertem C₁-C₁₀-Alkyl, bis zu perhalosubstituiertem C₃-C₁₀-Cycloalkyl, bis zu perhalosubstituiertem C₅-C₁₄-Aryl und bis zu perhalosubstituiertem C₃-C₁₃-Heteroaryl; und
wobei R⁴ und R^{4'} unabhängig ausgewählt sind aus der Gruppe bestehend aus H, C₁-C₁₀-Alkyl, C₃-C₁₀-Cycloalkyl, C₆-C₁₄-Aryl, C₃-C₁₃-Heteroaryl, C₇-C₂₄-Alkaryl, C₄-C₂₃-Alkheteroaryl, bis zu perhalosubstituiertem C₁-C₁₀-Alkyl, bis zu perhalosubstituiertem C₃-C₁₀-Cycloalkyl, bis zu perhalosubstituiertem C₆-C₁₄-Aryl und bis zu perhalosubstituiertem C₃-C₁₃-Heteroaryl
und
R^{c} Wasserstoff, Halogen, C₁-C₁₀-Alkyl, bis zu perhalosubstituiertem C₁-C₁₀-Alkyl ist oder mit R¹ und den Ringkohlenstoffatomen, an welchen R¹ und R^{c} gebunden sind kombiniert, um einen 5- oder gliedrigen Cycloalkyl-, Aryl- oder Heteroarylring mit 0-2 Gliedern ausgewählt aus O, N und S zu bilden, und pharmazeutisch akzeptable Salze davon zur Herstellung eines Medikamentes für die Behandlung einer durch p38-vermittelten Krankheit außer Krebs, wobei die Krankheit eine entzündliche oder immunmodulatorische Krankheit ist.

2. Verwendung von Anspruch 1, wobei B 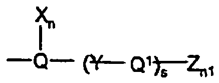 ist,
wobei Y ausgewählt ist aus der Gruppe bestehend aus -O-, oder -S-, Q Phenyl oder 3-Pyridinyl, substituiert oder unsubstituiert mit Halogen, bis zur Perhalosubstitution ist;
Q¹ eine mono- oder bizyklische aromatische Struktur von 5-10 Kohlenstoffatomen und 0-4 Gliedern aus einer Gruppe bestehend aus N, O und S, unsubstituiert oder substituiert durch Halogen bis zur Perhalosubstitution ist, und
X, Z, n und n1 wie in Anspruch 1 definiert sind und s 0 oder 1 ist.

3. Verwendung von Anspruch 2, wobei
Q Phenyl oder 3-Pyridinyl ist, substituiert oder unsubstituiert mit Halogen bis zur Perhalosubstitution,
Q¹ ausgewählt ist aus der Gruppe bestehend aus Phenyl, Pyridinyl, Naphthyl, Pyrimidinyl, Quinolin, Isoquinolin, Imidazol und Benzothiazolyl, substituiert oder unsubstituiert mit Halogen, bis zur Perhalosubstitution, oder -Y-Q¹ Phthalimidinyl substituiert oder unsubstituiert mit Halogen bis zur Perhalosubstitution ist, und
Z und X unabhängig gewählt werden aus der Gruppe bestehend aus -R⁶, -OR⁶ und -NHR⁷, wobei R⁶ Wasserstoff, C₁-C₁₀-Alkyl oder C₃-C₁₀-Cycloalkyl ist und R⁷ ausgewählt aus der Gruppe bestehend aus Wasserstoff, C₃-C₁₀-Alkyl, C₃-C₆-Cycloalkyl und C₆-C₁₀-Aryl ist, wobei R⁶ und R⁷ mit Halogen bis zur Perhalosubstitution substituiert sein können.

4. Verwendung von Anspruch 1, wobei die Verbindung von Formel 1 die Verbindung der Formel 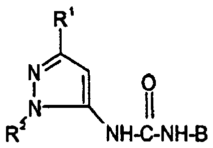 ist,
wobei R¹ und R² und B wie in Anspruch 1 definiert sind.

5. Die Verwendung von Anspruch 4, wobei B 2,3-Dichlorphenyl ist oder von der Formel 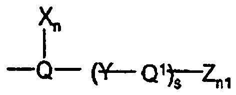 wobei Q Phenyl ist, Q¹ Phenyl oder Piridinyl ist, Y -O-, oder -S- ist, X CF₃ ist, und Z -OH, -Cl oder NHC(O)-CₚH₂ₚ₊₁ ist, wo p = 2-4, s = 0 oder 1, n = 0 und n1 = 0 oder 1 ist.

6. Verwendung von Anspruch 1, wobei die Verbindung der Formel 1 eine Verbindung ist ausgewählt von der Gruppe bestehend aus:
*N*-(3-*tert*-Butyl-5-pyrazolyl)-*N*'-(4-(2,3-dichlorphenyl)harnstoff;
*N*-(3-*tert-*Butyl-5-pyrazolyl)-*N*'-(3-(4-pyridinyl)thiophenyl)harnstoff;
*N-(3-tert-*Butyl-5-pyrazolyl)-*N*'-(4-(4-pyridinyl)methylphenyl)harnstoff;
*N*-(3-*tert*-Butyl-5-pyrazolyl)-*N*'-(4-(4-pyridinyl)oxyphenyl)harnstoff;
*N*-(3-*tert*-Butyl-5-pyrazolyl)-*N*'-(4-(4-pyridinyl)thiophenyl)harnstoff;
*N*-(3-*tert-*Butyl-5-pyrazolyl)-*N*'-(4-(4-pyridinyl)methylphenyl)harnstoff;
N-(1-Methyl-3-*tert*-butyl-5-pyrazolyl)-*N*'-(2,3-dichlorphenyl)harnstoff;
*N*-(1-Methyl-3-*tert*-butyl-5-pyrazolyl)-*N*'-(4-(4-hydroxyphenyl)thiophenyl)harnstoff;
*N*-(1-Methyl-3-*tert*-butyl-5-pyrazolyl)-*N*'-(4-(4-ethylaminocarbonyl-phenyl)oxyphenyl)harnstoff;
*N*-(1-Methyl-3-*tert*-butyl-5-pyrazolyl)-*N*'-(4-(4-isobutylaminocarbonyl-phenyl)thiophenyl)harnstoff;
*N*-(1-Methyl-3-*tert*-butyl-5-pyrazolyl)-*N*'-(4-(4-pyridinyl)thiophenyl)harnstoff;
*N*-(1-Methyl-3-*tert*-butyl-5-pyrazolyl)-*N*'-(3-(4-pyridinyl)thiophenyl)harnstoff;
*N*-(1-Methyl-3-*tert*-butyl-5-pyrazolyl)-*N*'-(4-(4-pyridinyl)thio-3-(trifluormethyl)phenyl)harnstoff;
*N*-(1-Methyl-3-*tert*-butyl-5-pyrazolyl)-*N*'-(4-(4-pyridinyl)oxyphenyl)harnstoff;
*N*-(1-Methyl-3-*tert*-butyl-5-pyrazolyl)-*N*'-(4-((4-pyridinyl)methylthio)-phenyl)harnstoff;
*N*-(1-(2,2,2-Trifluorethyl)-3-*tert*-butyl-5-pyrazolyl)-*N*'-(2,3-dichlorphenyl)harnstoff;
*N*-(1-(2-Hydroxyethyl)-3-*tert*-butyl-5-pyrazolyl)-*N*'-(2,3-dichlorphenyl)harnstoff;
*N*-(1-Ethoxycarbonylmethyl-3-*tert*-butyl-5-pyrazolyl)-*N*'-(2,3-dichlorphenyl)harnstoff;
*N*-(1-(2-Cyanoethyl)-3-*tert*-butyl-5-pyrazolyl)-*N*'-(2,3-dichlorphenyl)harnstoff;
*N*-(1-(3-Hydroxyphenyl)methyl-3-*tert*-butyl-5-pyrazolyl)-*N*'-(2,3-dichlorphenyl)harnstoff;
*N*-(1-Cyclohexyl-3-*tert*-butyl-5-pyrazolyl)-*N*'-(4-(4-pyridinyl)methyl-phenyl)harnstoff;
*N*-(1-Methyl-3-phenyl-5-pyrazolyl)-*N*'-(3-(4-(2-methylcarbamoyl)-pyridyl)thiophenyl)harnstoff;
*N*-(1-Methyl-3-*tert*-butyl-5-pyrazolyl)-*N*'(4-(4-pyridyl)thiophenyl)harnstoff;
*N*-(1-Methyl-3-*tert* butyl-5-pyrazolyl)-*N*'-(3-(4-pyridyl)thiophenyl)harnstoff;
*N*-(-Methyl-3-*tert*-butyl-5-pyrazolyl)-*N*'-(3-trifluormethyl-4-(4-pyridylthio)phenyl)harnstoff;
*N*-(3-*tert*-Butyl-5-pyrazolyl)-*N*'-(3-(4-pyridyl)oxyphenyl)harnstoff;
*N*-(3-*tert*-Butyl-5-pyrazolyl)-*N*'-(4-(4-pyridyl)oxyphenyl)harnstoff;
und pharmazeutisch akzeptablen Salzen davon ist.

7. Verwendung von Anspruch 4, wobei R¹ t-Butyl ist.

8. Verwendung von Anspruch 1, wobei die Verbindung der Formel I p38 Aktivität (IC₅₀) besser als 10 µm wie bestimmt durch einen in-vitro-Kinase-Assay anzeigt.

9. Verwendung von Anspruch 1, wobei die Krankheit durch ein Cytokin oder p38 regulierte Protease vermittelt ist.

10. Verwendung von Anspruch 1, wobei die Menge der Verbindung von Formel 1 effektiv ist zur Inhibierung von p38.

11. Verwendung von Anspruch 1, wobei die Menge der Verbindung von Formel I effektiv ist die Produktion eines krankheitsvermittelnden Cytokines oder Protease zu inhibieren.

12. Verwendung von Anspruch 1, wobei die Krankheit durch TNFα, MMP-1, MMP-3, IL-1, IL-6 oder IL-8 vermittelt ist.

13. Verwendung von Anspruch 1, wobei die Krankheit eine entzündliche Krankheit ist.

14. Verwendung von Anspruch 1, wobei die Krankheit eine immunmodulatorische Krankheit ist.

15. Verwendung von Anspruch 1, wobei die Krankheit rheumatoide Arthritis, Osteoporose, Osteoarthritis, Asthma, septischer Schock, entzündliche Darmerkrankung , oder ein Ergebnis von Host-versus-Graft-Reaktionen ist.

16. Verbindung einer der Formeln
d) 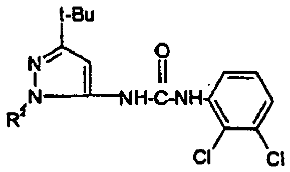 wobei R2 -CH₂CF₃, -C₂H₄, -OH, -CH₂-(3-HOC₆H₄), -CH₂C(O)NHCH₃, -CH₂C(O)OC₂H₅, -C₂H₄CN ist oder 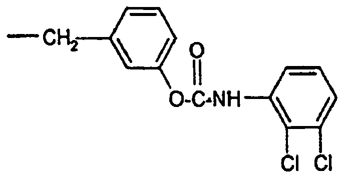 oder
e) 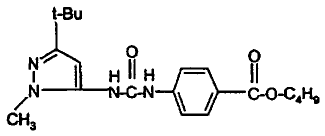 und pharmazeutisch akzeptable Salze davon sind.

17. Pharmazeutische Zusammensetzung umfassend eine Verbindung gemäß Anspruch 16 oder ein pharmazeutisch akzeptables Salz davon und einem physiologisch akzeptablen Träger.

## Revendications

1. Utilisation d'un composé de formule I 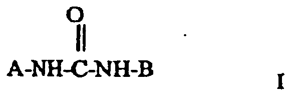 où B est choisi dans le groupe consistant en 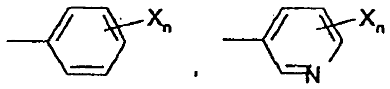 qui est non substitué ou substitué par halogène, jusqu'à la per-halosubstitution, et
où n = 0-3 et chaque X est choisi indépendamment dans le groupe consistant en -CN, -CO₂R⁵, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀ alkyle, C₂-C₁₀ alcényle, C₁-C₁₀ alcoxy, C₃-C₁₀ cycloalkyle, C₆-C₁₄ aryle, C₇-C₂₄ alkylaryle, C₃-C₁₃ hétéroaryle, C₄-C₂₃ alkylhétéroaryle, et C₁-C₁₀ alkyle substitué, C₂-C₁₀ alcényle substitué, C₁-C₁₀ alcoxy substitué, C₃-C₁₀ cycloalkyle substitué, C₄-C₂₃ alkylhétéroaryle substitué et -Y-Ar ;
où si X est un groupe substitué, il est substitué par un ou plusieurs substituants choisis indépendamment dans le groupe consistant en -CN, -CO₂R⁵, -C(O)R⁵, -C(O)NR⁵R^{5'}, -OR⁵, -SR⁵, -NR⁵R^{5'}, -NO₂, -NR⁵C(O)R^{5'}, -NR⁵C(O)OR^{5'} et halogène jusqu'à la per-halosubstitution ;
où R⁵ et R^{5'} sont choisis indépendamment parmi H, C₁-C₁₀ alkyle, C₂-C₁₀ alcényle, C₃-C₁₀ cycloalkyle, C₆-C₁₄ aryle, C₃-C₁₃ hétéroaryle, C₇-C₂₄ alkylaryle, C₄-C₂₃ alkylhétéroaryle, C₁-C₁₀ alkyle jusqu'à per-halosubstitué, C₂-C₁₀ alcényle jusqu'à per-halosubstitué, C₃-C₁₀ cycloalkyle jusqu'à per-halosubstitué, C₆-C₁₄ aryle jusqu'à per-halosubstitué et C₃-C₁₃ hétéroaryle jusqu'à per-halosubstitué,
où Y est -O- ou -S-,
et
Ar est une structure aromatique à 5-10 chaînons contenant 0-4 chaînons du groupe consistant en l'azote, l'oxygène et le soufre qui est non substituée ou substituée par halogène jusqu'à per-halo et éventuellement substituée par Zₙ₁, où n1 est 0 à 3 et chaque Z est choisi indépendamment dans le groupe consistant en -CN, -CO₂R⁵, -C(O)R⁵, =O, -SO₂R⁵, -SO₂NR⁵R^{5'}, -C(O)NR⁵R^{5'}, -C(O)R⁵, -NO₂, -OR⁵, -SR⁵, -NR⁵R^{5'}, -NR⁵C(O)OR^{5'}, -NR⁵C(O)R^{5'}, C₁-C₁₀ alkyle, C₁-C₁₀ alcoxy, C₃-C₁₀ cycloalkyle, C₆-C₁₄ aryle, C₃-C₁₃ hétéroaryle, C₇-C₂₄ alkylaryle, C₄-C₂₃ alkylhétéroaryle, C₁-C₁₀ alkyle substitué, C₃-C₁₀ cycloalkyle substitué, C₇-C₂₄ alkylaryle substitué et C₄-C₂₃ alkylhétéroaryle substitué ; où si Z est un groupe substitué, il est substitué par un ou plusieurs substituants choisis indépendamment dans le groupe consistant en -CN, -CO₂R⁵, -C(O)NR⁵R^{5'}, =O, -OR⁵, -SR⁵, -NO₂, -NR⁵R^{5'}, -NR⁵C(O)R^{5'}, -NR⁵SC(O)OR^{5'}, C₁-C₁₀ alkyle, C₁-C₁₀ alcoxy, C₃-C₁₀ cycloalkyle, C₃-C₁₀ hétéroaryle, C₆-C₁₄ aryle, C₄-C₂₄ alkylhétéroaryle et C₇-C₂₄ alkylaryle, et
A est 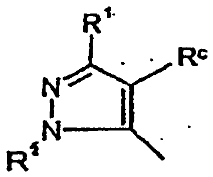 où
R¹ est choisi dans le groupe consistant en halogène, C₃-C₁₀ alkyle, C₃-C₁₀ cycloalkyle, C₁-C₁₃ hétéroaryle, C₆-C₁₄ aryle, C₇-C₂₄ alkylaryle, C₁-C₁₀ alkyle jusqu'à per-halosubstitué, C₃-C₁₀ cycloalkyle jusqu'à per-halosubstitué, C₁-C₁₃ hétéroaryle jusqu'à per-halosubstitué, C₆-C₁₄ aryle jusqu'à per-halosubstitué et C₇-C₂₄ alkylaryle jusqu'à per-halosubstitué,
R² est choisi dans le groupe consistant en H, -C(O)R⁴, -CO₂R⁴, -C(O)NR³R^{3'}, C₁-C₁₀ alkyle, C₃-C₁₀ cycloalkyle, C₇-C₂₄ alkylaryle, C₄-C₂₃ alkylhétéroaryle, C₁-C₁₀ alkyle substitué, C₃-C₁₀ cycloalkyle substitué, C₇-C₂₄ alkylaryle substitué et C₄-C₂₃ alkylhétéroaryle substitué,
quand R² est un groupe substitué, il est substitué par un ou plusieurs substituants choisis indépendamment dans le groupe consistant en -CN, -CO₂R⁴, -C(O)-NR³R^{3'}, -NO₂, -OR⁴, -SR⁴, et halogène jusqu'à la per-halosubstitution,
où R³ et R^{3'} sont choisis indépendamment dans le groupe consistant en H, -OR⁴, -SR⁴, -NR⁴R^{4'}, -C(O)R⁴, -CO₂R⁴, -C(O)NR⁴R^{4'}, C₁-C₁₀ alkyle, C₃-C₁₀ cycloalkyle, C₆-C₁₄ aryle, C₃-C₁₃ hétéroaryle, C₇-C₂₄ alkylaryle, C₄-C₂₃ alkylhétéroaryle, C₁-C₁₀ alkyle jusqu'à per-halosubstitué, C₃-C₁₀ cycloalkyle jusqu'à per-halosubstitué, C₆-C₁₄ aryle jusqu'à per-halosubstitué et C₃-C₁₃ hétéroaryle jusqu'à per-halosubstitué ; et
où R⁴ et R^{4'} sont choisis indépendamment dans le groupe consistant en H, C₁-C₁₀ alkyle, C₃-C₁₀ cycloalkyle, C₆-C₁₄ aryle, C₃-C₁₃ hétéroaryle, C₇-C₂₄ alkylaryle, C₄-C₂₃ alkylhétéroaryle, C₁-C₁₀ alkyle jusqu'à per-halosubstitué, C₃-C₁₀ cycloalkyle jusqu'à per-halosubstitué, C₆-C₁₄ aryle jusqu'à per-halosubstitué et C₃-C₁₃ hétéroaryle jusqu'à per-halosubstitué ;
et
R^{c} est l'hydrogène, halogène, C₁-C₁₀ alkyle, C₁-C₁₀ alkyle jusqu'à per-halosubstitué ou se combine avec R¹ et les atomes de carbone cycliques auxquels R¹ et R^{c} sont liés pour former un cycle cycloalkyle, aryle ou hétéroaryle à 5 ou 6 chaînons avec 0-2 chaînons choisis parmi O, N et S, et de ses sels pharmaceutiquement acceptables pour la préparation d'un médicament pour le traitement d'une maladie à médiation par p38 différente du cancer, où la maladie est une maladie inflammatoire ou immunomodulatrice.

2. Utilisation selon la revendication 1 où B est 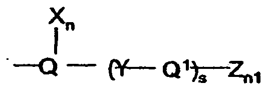 où Y est choisi dans le groupe consistant en -O- et -S-,
Q est phényle ou 3-pyridinyle, substitué ou non substitué par halogène, jusqu'à la per-halosubstitution ;
Q¹ est une structure aromatique mono- ou bicyclique de 5 à 10 atomes de carbone et 0-4 chaînons du groupe consistant en N, O et S, non substituée ou substituée par halogène jusqu'à la per-halosubstitution, et
X, Z, n et n1 sont définis comme dans la revendication 1 et s est 0 ou 1.

3. Utilisation selon la revendication 2 où
Q est phényle ou 3-pyridinyle, substitué ou non substitué par halogène, jusqu'à la per-halosubstitution,
Q¹ est choisi dans le groupe consistant en phényle, pyridinyle, naphtyle, pyrimidinyle, quinoléine, isoquinoléine, imidazole et benzothiazolyle, substitué ou non substitué par halogène, jusqu'à la per-halosubstitution, ou -Y-Q¹ est phtalimidinyle substitué ou non substitué par halogène jusqu'à la perhalosubstitution, et
Z et X sont choisis indépendamment dans le groupe consistant en -R⁶, -OR⁶ et NHR⁷, où R⁶ est l'hydrogène, C₁-C₁₀ alkyle ou C₃-C₁₀ cycloalkyle et R⁷ est choisi dans le groupe consistant en l'hydrogène, C₃-C₁₀ alkyle, C₃-C₆ cycloalkyle et C₆-C₁₀ aryle, où R⁶ et R⁷ peuvent être substitués par halogène jusqu'à la per-halosubstitution.

4. Utilisation selon la revendication 1 où le composé de formule I est un composé de formule 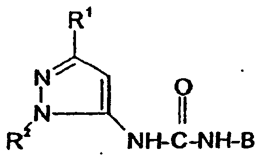 où R¹ et R² et B sont définis comme dans la revendication 1.

5. Utilisation selon la revendication 4 où B est 2,3-dichlorophényle ou de formule 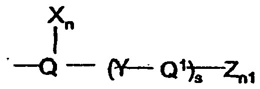 où Q est phényle, Q¹ est phényle ou pyridinyle, Y est -O- ou -S-, X est CF₃ et Z est -OH, -Cl ou NHC(O)-CₚH₂ₚ₊₁, où p = 2-4, s = 0 ou 1, n = 0 et n1 = 0 ou 1.

6. Utilisation selon la revendication 1 où le composé de formule I est un composé choisi dans le groupe consistant en :
la *N*-(3-*tert*-butyl-5-pyrazolyl)-*N*'-(4-(2,3-dichlorophényl)urée ;
la *N*-(3-*tert*-butyl-5-pyrazolyl)-*N*'-(3-(4-pyridinyl)thiophényl)urée ;
la *N*-(3-*tert*-butyl-5-pyrazolyl)-*N*'-(4-(4-pyridinyl)méthylphényl)urée ;
la *N*-(3-*tert*-butyl-5-pyrazolyl)-*N*'-(4-(4-pyridinyl)oxyphényl)urée ;
la *N*-(3-*tert*-butyl-5-pyrazolyl)-*N*'-(4-(4-pyridinyl)thiophényl)urée ;
la *N*-(3-*tert*-butyl-5-pyrazolyl)-*N*'-(4-(4-pyridinyl)méthylphényl)urée ;
la *N*-(1-méthyl-3-*tert*-butyl-5-pyrazolyl)-*N*'-(2,3-dichlorophényl)urée ;
la *N*-(1-méthyl-3-*tert*-butyl-5-pyrazolyl)-*N*'-(4-(4-hydroxyphényl)thiophényl)-urée ;
la *N*-(1-méthyl-3-*tert*-butyl-5-pyrazolyl)-*N*'-(4-(4-éthylaminocarbonylphényl)-oxyphényl)urée ;
la *N*-(1-méthyl-3-*tert*-butyl-5-pyrazolyl)-*N*'-(4-(4-isobutylaminocarbonylphényl)-thiophényl)urée ;
la *N*-(1-méthyl-3-*tert*-butyl-5-pyrazolyl)-*N*'-(4-(4-pyridinyl)thiophényl)urée ;
la *N*-(1-méthyl-3-*tert*-butyl-5-pyrazolyl)-*N*'-(3-(4-pyridinyl)thiophényl)urée ;
la *N*-(1-méthyl-3-*tert*-butyl-5-pyrazolyl)-*N*'-(4-(4-pyridinyl)thio-3-(trifluorométhylphényl)urée ;
la *N*-(1-méthyl-3-*tert*-butyl-5-pyrazolyl)-*N*'-(4-(4-pyridinyl)oxyphényl)urée ;
la *N*-(1-méthyl-3-*tert*-butyl-5-pyrazolyl)-*N*'-(4-((4-pyridinyl)méthylthio)phényl)-urée ;
la *N*-(1-(2,2,2-trifluoroéthyl)-3-*tert*-butyl-5-pyrazolyl)-*N*'-(2,3-dichlorophényl)-urée ;
la *N*-(1-(2-hydroxyéthyl)-3-*tert*-butyl-5-pyrazolyl)-*N*'-(2,3-dichlorophényl)urée ;
la *N*-(1-éthoxycarbonylméthyl-3-*tert*-butyl-5-pyrazolyl)-*N*'-(2,3-dichlorophényl)-urée ;
la *N*-(1-(2-cyanoéthyl)-3-*tert*-butyl-5-pyrazolyl)-*N*'-(2,3-dichlorophényl)urée ;
la *N*-(1-(3-hydroxyphényl)méthyl-3-*tert*-butyl-5-pyrazolyl)-*N*'-(2,3-dichlorophényl)urée ;
la *N*-(1-cyclohexyl-3-*tert*-butyl-5-pyrazolyl)-*N*'-(4-(4-pyridinyl)méthylphényl)-urée ;
la *N*-(1-méthyl-3-phényl-5-pyrazolyl)-*N*'-(3-(4-(2-méthylcarbamoyl)pyridyl)-thiophényl)urée ;
la *N*-(1-méthyl-3-*tert*-butyl-5-pyrazolyl)-*N*'-(4-(4-pyridyl)thiophényl)urée ;
la *N*-(1-méthyl-3-*tert*-butyl-5-pyrazolyl)-*N*'-(3-(4-pyridyl)thiophényl)urée ;
la *N*-(1-méthyl-3-*tert*-butyl-5-pyrazolyl)-*N*'-(3-trifluorométhyl-4-(4-pyridylthio)-phényl)urée ;
la *N*-(3-*tert*-butyl-5-pyrazolyl)-*N*'-(3-(4-pyridyl)oxyphényl)urée ;
la *N*-(3-*tert*-butyl-5-pyrazolyl)-*N*'-(4-(4-pyridyl)oxyphényl)urée ;
et leurs sels pharmaceutiquement acceptables.

7. Utilisation selon la revendication 4 où R¹ est t-butyle.

8. Utilisation selon la revendication 1 où le composé de formule I présente une activité sur p38 (CIso) meilleure que 10 µm déterminée par un test de kinase in vitro.

9. Utilisation selon la revendication 1 où la maladie est à médiation par une cytokine ou une protéase régulée par p38.

10. Utilisation selon la revendication 1 où la quantité de composé de formule I est efficace pour inhiber p38.

11. Utilisation selon la revendication 1 où la quantité de composé de formule I est efficace pour inhiber la production d'une cytokine ou protéase assurant la médiation de maladies.

12. Utilisation selon la revendication 1 où la maladie est à médiation par TNFα, MMP-1, MMP-3, IL-1, IL-6 ou IL-8.

13. Utilisation selon la revendication 1 où la maladie est une maladie inflammatoire.

14. Utilisation selon la revendication 1 où la maladie est une maladie immunomodulatrice.

15. Utilisation selon la revendication 1 où la maladie est la polyarthrite rhumatoïde, l'ostéoporose, l'arthrose, l'asthme, le choc septique, une maladie inflammatoire chronique de l'intestin ou le résultat de réactions du greffon contre l'hôte.

16. Composé de l'une des formules
d) 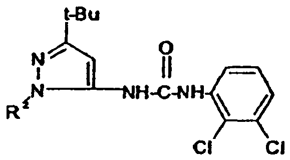 où R² est -CH₂CF₃, -C₂H₄-OH, -CH₂-(3-HOC₆H₄), -CH₂C(O)NHCH₃, -CH₂C(O)OC₂H₅, -C₂H₄CN, ou 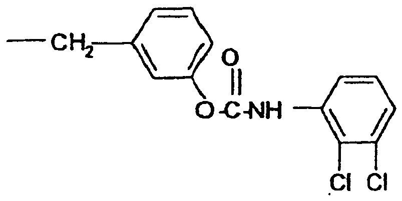 ou
e) 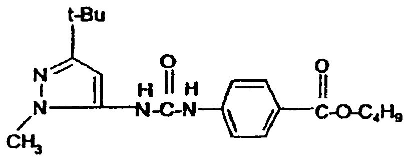 et ses sels pharmaceutiquement acceptables.

17. Composition pharmaceutique comprenant un composé selon la revendication 16 ou un sel pharmaceutiquement acceptable de celui-ci et un vecteur physiologiquement acceptable.
